(19) Europäisches Patentamt
European Patent Office
Office européen des brevets

(11) EP 4 733 326 A1

(12) **EUROPEAN PATENT APPLICATION**
published in accordance with Art. 153(4) EPC

(43) Date of publication:
**29.04.2026 Bulletin 2026/18**

(21) Application number: **24830765.4**

(22) Date of filing: **25.06.2024**

(51) International Patent Classification (IPC):
***C08B 37/00*** *(2006.01)* ***C12P 19/04*** *(2006.01)*
***A61P 1/16*** *(2006.01)* ***A61P 3/10*** *(2006.01)*
***A61P 17/00*** *(2006.01)* ***A61P 37/04*** *(2006.01)*
***A61P 39/06*** *(2006.01)* ***A61P 31/10*** *(2006.01)*

(86) International application number:
**PCT/CN2024/101362**

(87) International publication number:
**WO 2025/002125 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.06.2023 CN 202310751669**

(71) Applicant: **Chengdu Sydix Biotech Co., Ltd**
**Chengdu, Sichuan 610095 (CN)**

(72) Inventor: **ZHANG, Ran**
**Chengdu, Sichuan 610095 (CN)**

(74) Representative: **JD&P Patent Attorneys**
**Joanna Dargiewicz & Partners**
**ul. Mysliborska 93A/50**
**03-185 Warszawa (PL)**

(54) **L-beta-GALACTOGLUCAN PRODUCED FROM AGROBACTERIUM, PREPARATION METHOD FOR L-beta-GALACTOGLUCAN, AND USE OF L-beta-GALACTOGLUCAN**

(57) An L-β-galactoglucan, preparation method and use thereof are disclosed. The polysaccharide is levorotatory, and composed of β-D-glucose and β-D-galactose, with a molecular weight of at least 5000 Da. The polysaccharide is prepared as follows: Inoculating Agrobacterium sp. FN01 into a culture broth for fermentation to obtain a fermentation broth; mixing the fermentation broth and alcohol, precipitating, and oven-drying to obtain a crude polysaccharide; purifying to obtain a pure polysaccharide; or enzymatically hydrolyzing the fermentation broth with glucanase, then filtering, concentrating by ultrafiltration, and spray drying to obtain the pure polysaccharide. The polysaccharide has the functions of treating and/or preventing liver injury, anti-aging, hypoglycemic effect, and repairing tissues, etc., and is a multi-functional polysaccharide.

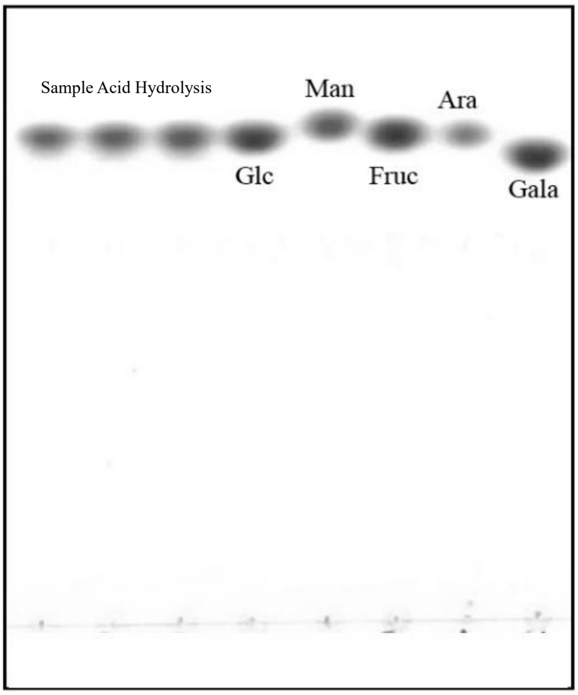


Figure 1

## Description

### Cross-reference

The application claims the priority for Chinese patent application CN2023107516693, with a filing date of June 25, 2023. The above incorporates by reference of the full text of Chinese patent application.

### Technical field

The present disclosure involves the field of polysaccharide technology in microbiology, particularly involves an L-β-galactoglucan produced by Agrobacterium sp. FN01 and the preparation method and use thereof.

### Background

Agrobacterium is a Gram-negative bacterium, a rod-shaped bacterium that is aerobic and does not produce spores. It belongs to the family Rhizobiaceae, but does not fix nitrogen. Some strains are peritrichous, while others are monotrichous. Widely distributed in soil, the vast majority of Agrobacterium strains are capable of inducing tumors in plants.

Polysaccharides are high-molecular-weight carbohydrate polymers composed of more than 10 monosaccharide units linked by glycosidic bonds. They are widely distributed in cell membranes of animals, and cell walls of plants and microorganisms. They not only exhibit multiple biological activities in various organisms, but also have multiple functions in the interactions among organisms. They are efficient biological response modifiers.

Generally, polysaccharides exhibit a variety of activities, such as immunomodulation, anti-tumor effects, hypoglycemic effects, lipid-lowering effects. They have attracted high attention from the medical community at home and abroad and have become a hot topic in research and application during recent years.

However, the structure of polysaccharide compounds is extremely complex, which leads to the complexity of polysaccharide compounds physicochemical properties and physiological activities. It is mainly reflected in the following aspects:

(1) Monosaccharide composition and arrangement: Polysaccharides are composed of multiple monosaccharide units, which can be identical (named as homopolysaccharides) or different (named as heteropolysaccharides). The types of monosaccharides (such as glucose, fructose, and galactose) and their arrangement order can affect the structure and properties of polysaccharides.

(2) The type of glycosidic bond: Monosaccharide units in polysaccharides are connected through glycosidic bonds. The type of glycosidic bond (such as α-1,4 and β-1,4) and the mode of linkage (such as linear, and branched) have a significant impact on the structure and properties of polysaccharides.

(3) Substituents and modifications: Polysaccharide molecules may carry various substituents (such as sulfate, and acetyl) and modifications (such as phosphorylation, and sulfation), which further increase the complexity of polysaccharide structures.

(4) The three-dimensional structures of polysaccharides include linear structure, branch structure, and helicoidal structure. The physicochemical properties and biological activities of polysaccharides with different three-dimensional structures are different. It is because the difference in three-dimensional structures has impact on the spatial conformation and intermolecular interactions of polysaccharides, thereby has impact on solubility, stability, biological activity, etc.

As the properties of polysaccharides depend to a great extent on their structure, even if only one different monosaccharide unit may lead to significant changes in the physicochemical properties of polysaccharides. Such changes may be reflected in solubility, viscosity, gel properties, stability, etc., and may also have impact on the biological activity and function of polysaccharides.

For example, starch and cellulose are both polysaccharides composed of glucose units, but their structures and properties differ greatly. Starch has a helical structure. It is mainly composed of glucose units linked by α-1,4-glycosidic bonds, and is soluble, while cellulose has a linear structure, is composed of glucose units linked by β-1,4-glycosidic bonds, and exists as an insoluble fibrous substance. The structural difference results in their completely different functions and roles in organisms.

In addition, the three-dimensional structure of polysaccharides also affects their interactions with other molecules. For example, certain polysaccharides with specific three-dimensional structures can bind to specific proteins or cell surface receptors, thereby exerting specific biological activities. Such interactions have important application value in fields such as biomedicine and drug development.

Therefore, the structural complexity of polysaccharides is not only reflected in the diversity of their composition and arrangement, but also in the diversity of their substituents and modifications. Such complexity endows polysaccharides with various physicochemical properties and biological activities, and provides broad application prospects in fields such as biomedicine and food science.

The preparation of polysaccharides using Agrobacterium and the determination of the structure and biological activity have great significance for the treatment of related diseases.

## Summary

During the study of Agrobacterium sp. FN01, it was accidentally discovered that the strain can produce an exopolysaccharide with the following activities through fermentation:

(1) Treating and/or preventing diseases related to glucose metabolic disorders;
(2) At least one of the functions of promoting skin repair, tissue regeneration, wound healing, and apoptotic cell clearance;
(3) Enhancing non-specific immune function;
(4) Treating and/or preventing diseases related to liver;
(5) Anti-fatigue;
(6) Regulating the intestinal flora;
(7) Anti-aging;
(8) At least one of the functions of hydrating and moisturizing, increasing skin elasticity, and whitening and freckle removal;
(9) Improving the health status of domestic animals;
(10) A product for inhibiting Malassezia spp.

Based on the above studies, the present disclosure provides an L-β-galactoglucan with levorotatory activity, which is mainly composed of β-D-glucose and β-D-galactose, with a molecular weight of not less than 5000 Da.

Further, in multiple specific embodiments of the present disclosure, the molecular weight distribution is above 1 × 106 Da.

Further, in multiple specific embodiments of the present disclosure, the weight-average molecular weight is 4.5 million to 5.5 million Da, such as 4.98 million to 5 million Da.

Further, the contents of β-D-glucose and β-D-galactose are 94%-98% and 2%-6%, respectively.

Further, in the infrared spectrum of the polysaccharide, there are at least characteristic peaks at 3436.8 cm-1, 2887.3cm-1, 1616.0 cm-1, 1000 cm-1-1200 cm-1, 1043.5 cm-1, 1073.0 cm-1, 1161.0 cm-1, and 893.0 cm-1.

Further, the polysaccharide may contain one or more than two glycosidic bonds at positions of (1→), (1→6), (1→2), (1→2,6), (1→4), (1→4,6), (1→3), (1→3,6), (1→2,3), (1→2,4), (1→3,4), (1→2,3,4).

Further, the polysaccharide may contain one or more of the following glycosidic bond linkages: ① Glcp (1→, ②-3) Galp (1→, ③→>6)Galp (1→, ④→4)Galp (1→, ⑤→6)Glcp (1→, ⑥→3,6)Galp (1→, ⑦→4,6)Glcp (1→, ⑧→2,3,6)Glcp (1→ and ⑨→2,3,4,6)Glcp (1→.

Based on the test results, it can be inferred that the repeating unit structural formula of the polysaccharide is as follows:

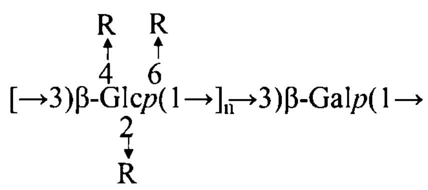

R= →2,3,4,6) β-Glcp (1→

Wherein, Glep is glucopyranose, Galp is galactopyranose, and n is an integer not less than 30.

The present disclosure provides a preparation method for the L-β-galactoglucan. The procedure is as follows:

(1) Inoculating Agrobacterium sp. FN01 into a culture broth for fermentation to obtain a fermentation broth;
(2) Precipitating the fermentation broth with alcohol and oven-drying to obtain a crude polysaccharide, dissolving the crude polysaccharide, adding a base and a filter aid, and then filtering, precipitating the filtrate with alcohol and drying to obtain a pure polysaccharide; or enzymatically hydrolyzing the fermentation broth with glucanase, then filtering, concentrating by ultrafiltration, and spray drying to obtain the pure polysaccharide.

The conventional process and flow of Agrobacterium fermentation mainly include:
Preparation of medium: Firstly, it is required to prepare a medium suitable for the growth of Agrobacterium. Generally, the medium includes basic nutrients (such as carbon source, nitrogen source, inorganic salt), as well as growth factors, etc.

Bacterial activation and amplification culture: Select a small amount of thalli from the preserved Agrobacterium strains, inoculate them onto the plates with appropriate culture medium for activation and culture. After the growth of bacterial colonies, select a single bacterial colony and inoculate it into the seed medium, then conduct shake-flask culture to increase the amount of thalli.

Fermentation culture: Inoculate the Agrobacterium after amplification culture into the fermentation culture medium for fermentation and culture. Fermentation conditions (such as temperature, pH, stirring speed, and aeration rate, etc.) should be optimized based on the characteristics of bacterial strains and product requirements. During the fermentation, it is required to test various indicators of the fermentation broth (such as bacterial density, metabolite concentration, etc.) regularly to monitor the fermentation process.

During the cultivation of Agrobacterium, the important substances in the culture medium or culture broth mainly include the following categories:

> Nitrogen sources: Peptone and yeast extract powder which are raw materials for synthesizing bacterial proteins, nucleic acids, and other nitrogen compounds.
> Carbon sources: Carbohydrates of glucose, sucrose, maltose, and lactose, etc., are the main energy sources for fungi and also participate in the synthesis of cellular substances.
> Inorganic salts: Dipotassium hydrogen phosphate and magnesium sulfate, etc. which participate in the metabolic processes of fungi, and maintains osmotic pressure and acid-base balance.

In addition, the corresponding growth factors can also be added according to the characteristics and requirements of specific fungi. For example, vitamins and amino acids, etc.

The main functions of these nutrients are to provide the energy required for bacterial growth and proliferation and the raw materials for synthesizing thalli, activating the activity of bacterial enzymes and regulating osmotic pressure, etc.

In addition to the essential nutrients mentioned above, water must also be added to the medium as a solvent, and solidifying agents such as agar may need to be added as excipients.

In a specific embodiment of the present disclosure, the culture broth may include the following components: 1.5-6.5% of carbon source, 0.1-0.5% of nitrogen source, 0.1-0.3% of potassium dihydrogen phosphate, 0.01-0.06% of magnesium sulfate, 0.001-0.003% of ferrous sulfate, 0.0006-0.0012% of manganese sulfate, 0.005-0.01% of calcium chloride, and 0.05-0.2% of defoaming agent.

Further, the carbon source may be selected from glucose, mannose, maltose, and lactose; and the nitrogen source can be selected from potassium nitrate, sodium nitrate, and peptone.

Wherein, the inoculum size of Agrobacterium sp. FN01 is 1-10% of the culture broth.

Wherein, in the step (1), the pH is adjusted to 6.5-7.5 using an acid or a base, and the fermentation temperature is 28-32°C.

During the purification of the polysaccharide, the addition of a base can remove impurities such as proteins. The commonly used bases are mainly inorganic bases, such as sodium hydroxide (NaOH), and potassium hydroxide.

Filter aids are generally used to improve filtration efficiency, and help to remove suspended particles and impurities from the solutions, so as to accelerate the purification of polysaccharide. The commonly used filter aids include diatomite, perlite, and cellulose, etc. They have a large specific surface area and good adsorption performance, and they can be sued to effectively remove impurities from solutions.

In a specific embodiment of the present disclosure, the base in the step (3) includes but is not limited to sodium hydroxide, potassium hydroxide, and calcium hydroxide; and the filter aid is selected from diatomite and perlite powder.

The "alcohol precipitation" in the present disclosure is a method of separating the target substance from water and impurities through the use of different solubility characteristics of the target substances in ethanol and water, and separating the precipitate and solution after adding ethanol. The purpose of alcohol precipitation is to remove water and impurities and retain the active ingredients of polysaccharides. In the process of extraction and purification of polysaccharides, polysaccharides are mainly dissolved in water. After adding a certain amount of ethanol, the solubility of polysaccharides is decreased for precipitation, and the polysaccharides are separated from water and other components.

The commonly used alcohol precipitation solvents include but are not limited to methanol, ethanol, and isopropanol. However, with the consideration of safety, cost, etc., ethanol is a commonly used solvent for alcohol precipitation.

In the present disclosure, the fermentation is aerobic fermentation, involving continuous aeration and stirring during the process. The aeration rate is 0.2-1.0 vvm, and the stirring speed is 60-150 rpm. In a specific embodiment of the present disclosure, from 0 to 24 hours, the aeration rate is 0.2-0.4 vvm and the stirring speed is 60 rpm; from 24 to 48 hours, the aeration rate is 0.5-0.7 vvm, and the stirring speed is 100 rpm; from 48 hours to the end of fermentation, the aeration rate

is 0.9-1.0 vvm, and the stirring speed is 150 rpm.

The present disclosure provides a use of the L-β-galactoglucan, the Agrobacterium fermentation broth containing the L-β-galactoglucan, or the crude L-β-galactoglucan polysaccharide in the manufacture of products for the treatment and/or prevention of diseases related to glucose metabolic disorders.

The "glucose metabolic disorders" in the present disclosure refer to the abnormal structure, function, and concentration of hormones or enzymes that regulate the metabolism of glucose, fructose, and galactose, etc., pathophysiologic changes in tissues and organs, or blood glucose increase. The clinically significant glucose metabolic disorders are mainly too high and too low blood glucose concentrations.

The common diseases related to glucose metabolic disorders include diabetes, hypoglycemia, fructose metabolism disorder, glycogenosis, galactose metabolism disorder and pyruvate metabolism disorder.

Diabetes is metabolic disorders such as blood glucose increase which are caused by absolute deficit of insulin and/or the reduction of insulin biological effect, which can also lead to chronic complications of blood vessels and nerves, etc.

Wherein, the product is a product that protects pancreas islet cells and/or lowers blood glucose.

Further, the pancreas islet cells are pancreatic β cells.

Pancreatic β cell (i.e., pancreatic B cells) is a type of pancreas islet cell, accounting for about 60% to 80% of pancreas islet cells. It secretes insulin and works together with glucagon secreted by pancreatic A cells to regulate blood glucose.

The protection of pancreatic islet cells includes avoiding pancreatic islet cell damage, promoting pancreatic islet cell repair or proliferation of new pancreatic islet cells after pancreatic islet cell damage.

Hypoglycemic effect refers to reducing the abnormal hyperglycemia for patient. The hyperglycemia is often associated with diabetes.

The product for treating and/or preventing glucose metabolic disorders can decrease blood glucose through inhibiting hepatic gluconeogenesis and hepatic glucose output, increasing peripheral tissue sensitivity to insulin, promoting glucose uptake and utilization, stimulating pancreatic β cells to secrete insulin, enhancing the binding of insulin to receptors, and increasing target cell sensitivity to insulin, etc.

The present disclosure provides a use of the L-β-galactoglucan, the Agrobacterium fermentation broth containing the L-β-galactoglucan, or the crude L-β-galactoglucan polysaccharide in the manufacture of products for promoting skin repair.

Skin repair usually refers to the process of restoring the normal structure and function of the skin through a series of physiological processes after injury of skin. It includes the regeneration of the epidermal layer, repair of the dermis layer, and reconstruction of skin appendages, etc. Skin repair is an important part of maintaining skin health and it can effectively resist the invasion of the external environment.

The important procedure of skin repair includes but is not limited to at least one of the functions of tissue regeneration, wound healing, and removal of apoptotic cells.

Tissue regeneration refers to the continuous renewal of biological tissues, old cells dying and generation of new cells; or the repair after the injury of tissue.

Wound healing refers to the process of healing and repairing after the tissues such as skin are broken or damaged by external force on the body. Wound healing is a complex biological process that involves multiple stages such as inflammatory reaction, cell proliferation, and tissue remodeling, etc. The quality of wound healing directly affects the recovery rate and prognosis of patients.

For the apoptotic cell clearance, the apoptosis is a form of programmed cell death, which refers to the process of self-apoptosis of cells by initiating internal program after being stimulated by certain signals. The apoptotic cell clearance is an important mechanism for maintaining tissue homeostasis and preventing the spread of inflammatory reaction in the body. The apoptotic cell clearance is usually completed by immune cells such as macrophages, etc., which engulf and digest apoptotic cells to prevent the release of harmful substances that can cause damage to surrounding tissues.

When the skin or tissue is damaged, the body initiates repair mechanisms to restore the normal structure and function of the tissue by promoting cell proliferation, differentiation, and synthesis of extracellular matrix. In the process, the apoptotic cell clearance is also an essential step, which helps to prevent the spread of inflammatory reaction and alleviate tissue damage.

The present disclosure provides a use of the L-β-galactoglucan, the Agrobacterium fermentation broth containing the L-β-galactoglucan, or the crude L-β-galactoglucan polysaccharide in the manufacture of products for inhibiting Malassezia spp.

In the specific embodiment of the present disclosure, the L-β-galactoglucan can effectively inhibit the expression of inflammatory cytokine IL-6 gene induced by Malassezia spp.

Malassezia spp., also known as Pityrosporum, is classified as a hyphomycete in mycology and belongs to the Basidiomycota-Taphrinales - Taphrinaceae. Malassezia spp. is a fungus that colonizes the normal skin surface of humans and animals. It is mainly distributed in areas rich in sebum, such as the scalp, face, chest and back, etc., accounting for

about 50% to 80% of the total amount of fungi colonized on healthy human skin. Under normal circumstances, it does not cause diseases, but under the promotion of certain factors, it can reproduce in large quantities, decompose lipids, lead to morphological changes and apoptosis of keratinocytes, and ultimately cause corresponding diseases. Malassezia spp. can cause diseases such as pityriasis versicolor, Malassezia folliculitis, and seborrheic dermatitis, etc. Patients may experience manifestations such as inflammation, rosacea, and fever, etc.

Further, in the present disclosure, the product for inhibiting Malassezia spp. is a product for treating skin infections caused by Malassezia spp.

Malassezia spp. is a common colonized microbiota on the scalp. As an opportunistic pathogen, it can cause various superficial and deep fungal infections under the impact of predisposing factors. The excessive proliferation of Malassezia spp. on the scalp usually causes tinea capitis and may lead to symptoms such as increased dandruff, oily hair, and scalp pruritus and discomfort.

Abnormal proliferation of Malassezia spp. on the scalp leads to:

(1) Increased Dandruff: Abnormal proliferation of Malassezia spp. may disrupt the normal ecological balance of the scalp, leading to the production of a large amount of dandruff.
(2) Scalp Pruritus: Excessive proliferation of Malassezia spp. may stimulate the scalp, causing pruritus and discomfort.
(3) Oil Hair: Proliferation of Malassezia spp. may promote the secretion of scalp grease, making the hair oily.
(4) Hair Loss: Excessive proliferation of Malassezia spp. may disrupt the normal structure of hair follicles, thereby affecting the normal growth of hair. If the degree of damage to hair follicles is severe, permanent hair loss may occur.

Based on the above situations, the product for inhibiting Malassezia spp. according to the present disclosure is also a product for treating a scalp infection induced by Malassezia spp. Such products can soothe the scalp, restore the ecological balance of the scalp, and reduce irritation, pruritus, and hair loss, etc.

The present disclosure provides a use of the L-β-galactoglucan, the Agrobacterium fermentation broth containing the L-β-galactoglucan, or the crude L-β-galactoglucan polysaccharide in the manufacture of products for enhancing immunity.

The enhancing immunity in the present disclosure includes but is not limited to enhancing specific immunity and non-specific immunity, especially the improvement of non-specific immunity.

Non-specific immunity, also known as innate immunity includes the following items in the body: tissue barrier immunity (the skin and mucosal systems, the blood-brain barrier, and the placental barrier, etc.); innate immune cell-mediated immunity (phagocytes, killer cells, and dendritic cells, etc.), and innate immune molecular immunity (complement, cytokines, and enzymes, etc.).

In a specific embodiment of the present disclosure, the enhancing non-specific immunity mainly includes but is not limited to increasing the number and/or regulating the function of innate immune cells.

Immune cells are those involved in or related to immune responses. They include lymphocytes, dendritic cells, monocytes/macrophages, granulocytes, and mast cells, etc.

Wherein, the product may be used to increase the number of phagocytes and/or to promote their phagocytic activity.

Further, the phagocytes are macrophages and/or neutrophils.

Phagocytes are a group of cells in the body that possess phagocytic function, comprising mainly the mononuclear phagocyte system and neutrophils. The mononuclear phagocyte system includes monocytes that are free in the blood and macrophages that develop after entering various tissues. Macrophages have strong phagocytic ability and are a major type of antigen-presenting cells, which play a crucial role in inducing and regulating specific immune responses. Neutrophils are a type of small phagocytes that have non-specific immune defense effect and participate in the body's immune response, and inflammatory injury, etc.

Wherein, may be used to increase the number of lymphocytes.

Further, the lymphocytes are T cells.

Lymphocytes are a type of cell line with immune recognition function. They can be classified into T lymphocytes (T cells), B lymphocytes (B cells), and natural killer (NK) cells based on the differences in their migration, surface molecules, and functions. T cells and B cells are antigen-specific lymphocytes; and lymphocytes are important cells for combating external infections and monitoring cellular mutations in the body.

The present disclosure provides a use of the L-β-galactoglucan, the Agrobacterium fermentation broth containing the L-β-galactoglucan, or the crude L-β-galactoglucan polysaccharide in the product for treating and/or preventing liver injury.

Liver injury refers to damage to the liver caused by one or more etiologies, resulting in inflammation, bleeding, and infection, etc. In clinical practice, liver injuries can be classified into narrow-sense and broad-sense types based on etiology. Liver injury in the narrow sense generally refers to the rupture, and bleeding resulting from trauma. Liver injury in

the broad sense also includes various inflammatory diseases of the liver in the internal medicine system, such as drug-induced hepatitis, alcoholic liver disease, non-alcoholic fatty liver, various viral hepatitides, and autoimmune liver diseases, etc.

Alcoholic liver injury: Based on the severity of liver damage caused by alcohol, it can be divided into three stages: alcoholic fatty liver, alcoholic hepatitis, and alcoholic cirrhosis. Long-term heavy alcohol consumption can lead to hepatic steatosis, consequently, the fat cannot be metabolized in hepatocytes and gradually accumulates, resulting in alcoholic fatty liver. During the process of metabolizing alcohol, the liver produces metabolites, ethanol, and metabolic derivatives that can cause inflammatory reactions in the liver. This inflammation damages hepatocytes and can even lead to necrosis, ultimately resulting in alcoholic hepatitis. If the damage occurring in the first and second stages is not controlled in a timely manner and is allowed to progress, it will gradually develop into nodules, fibroplasia, liver fibrosis, and cirrhosis at last. At this point, the damage to the liver is irreversible and may transform into liver cancer.

Non-alcoholic liver injury: This injury may be caused by various factors, such as drug-induced hepatitis, fatty liver, viral hepatitis, or autoimmune liver disease. These diseases can all lead to hepatocyte degeneration, necrosis, and fibrosis, and may present as non-alcoholic liver injury upon examination. For example, fatty liver is often caused by abnormal fat metabolism, which can be asymptomatic or result in non-alcoholic liver injury. Attention should be paid to control diet, strengthen exercise, and provide drug therapy if necessary.

There is a close relationship between liver injury and liver transaminases, such as aspartate aminotransferase (AST) and alanine aminotransferase (ALT). When the hepatocytes are damaged, transaminases within them are released into the bloodstream, leading to elevated transaminase levels. Therefore, AST and ALT are commonly used liver function indicators to evaluate the degree of hepatocyte damage.

Reducing transaminase activity can help treat liver injury to some extent:

(1) Alleviating hepatic inflammation: Elevated transaminase levels are a marker of liver inflammation. Reducing transaminase activity means reducing the inflammatory response of liver, which helps resolve further damage to hepatocytes.

(2) Repairing of damaged hepatocytes: Some drugs or treatment methods can reduce transaminase activity while promoting the repair and regeneration of hepatocytes. This helps to restore normal liver function, thereby improving the overall health status of patients.

(3) Delaying disease progression: By reducing transaminase activity, the progression of hepatic diseases such as hepatic cirrhosis and hepatic cancer can be postponed. It holds significant importance for improving patients' quality of life and extending survival.

Based on the critical role of transaminases in liver injury, the present disclosure further provides a product for reducing AST activity and/or ALT activity.

Wherein, the product is one for protecting liver tissue.

The present disclosure provides a use of the L-β-galactoglucan, the Agrobacterium fermentation broth containing the L-β-galactoglucan, or the crude L-β-galactoglucan polysaccharide in the manufacture of products for anti-fatigue.

Fatigue is a subjective feeling of tiredness and weakness, but objectively manifests as a loss of the ability to perform normal activities or work under equivalent conditions. Fatigue sensation is not a specific symptom; numerous diseases can cause fatigue accompanied by various neurological and psychological symptoms, yet without organic mental disorder.

Reactive Oxygen Species (ROS): ROS are highly reactive molecules generated during cellular metabolism. They include peroxide, superoxide, hydroxyl radical, singlet oxygen, and alpha-oxygen. ROS are natural by-product of normal oxygen metabolism and play a crucial role in cellular signaling and homeostasis. Under fatigue status, ROS production may increase, and leads to a rise in intracellular oxidative stress level. The oxidative stress status may damage cellular structures and functions, further exacerbating the sensation of fatigue. The accumulation of ROS is related to various fatigue-related diseases and pathological conditions, including cardiovascular diseases, diabetes, and neurodegenerative diseases.

ATP (adenosine triphosphate): ATP is the primary energy molecule in cells. It is composed of adenine, ribose, and 3 phosphate groups; its hydrolysis releases considerable energy. As the most direct energy source in organisms, it is crucial for maintaining normal cellular function and metabolism. Under fatigue condition, ATP production may decrease while the consumption may increase. The insufficient energy supply impairs cell function, further exacerbating fatigue sensation. Declining ATP level is related to various fatigue-related symptoms, including muscle fatigue, hypoprosexia, and memory decline.

Lactic acid metabolism: Lactic acid is a metabolite produced when muscles undergo anaerobic metabolism under hypoxic condition. After vigorous exercise or long-term exercise, muscle tissue may undergo anaerobic metabolism due to insufficient oxygen supply, generating a large amount of lactic acid. The accumulation of lactic acid causes local muscle soreness and fatigue, and impairs exercise capacity and recovery speed. Additionally, the accumulation of lactic

acid may lead to a disturbance of acid-base balance in the body, further intensifying the fatigue sensation.

ROS, ATP, and lactic acid metabolism all play significant roles in fatigue. The fatigue can be effectively relieved, and physical adaptability and recovery rate can be enhanced by regulating the levels of these factors.

The present disclosure provides a use of the L-β-galactoglucan, the Agrobacterium fermentation broth containing the L-β-galactoglucan, or the crude L-β-galactoglucan polysaccharide in the manufacture of products for regulating intestinal flora.

The intestinal flora refers to the bacterial communities that colonize in the guts of humans and animals, and have a long-term dependence with them. The significance of the intestinal flora in the body is primarily reflected in the following aspects:

(1) Supporting digestion: The intestinal flora decomposes and digests certain food components such as cellulose, proteins, and carbohydrates, thereby promoting nutrient absorption and utilization.

(2) Maintaining intestinal health: The intestinal flora suppresses the growth of harmful bacteria, maintains the intestinal microecological balance in the intestine, and helps prevent intestinal infections and diseases. Simultaneously, as antigens, the intestinal flora can stimulate and promote the development and functional maturation of the immune system, thereby enhancing the body's immunity.

(3) Promoting nutrient absorption: The intestinal flora can secrete various enzymes that aid in the absorption and utilization of nutrients, such as vitamins and amino acids.

The benefits of regulating the intestinal flora for humans and animals primarily manifest in improving intestinal health, enhancing immunity, promoting nutrient absorption, and preventing diseases. For example, the balance of intestinal flora can be adjusted through dietary modification and probiotic supplementation, thereby alleviating diarrhea, enhancing body resistance, promoting the absorption and utilization of nutrients, and preventing intestinal diseases and tumors. For animals, the growth performance and disease resistance can be enhanced through regulating the intestinal flora.

In one specific embodiment, the product for regulating intestinal flora described herein is one that promotes the proliferation of intestinal probiotics, thereby promoting intestinal health.

Wherein, the product is a one that increases the quantity of Lactobacillus brevis in the gut.

The present disclosure provides a use of the L-β-galactoglucan, the Agrobacterium fermentation broth containing the L-β-galactoglucan, or the crude L-β-galactoglucan polysaccharide in the manufacture of products for anti-aging.

Aging refers to the progressive decline in an organism's physical and psychological adaptability to the environment, and a trend of gradual death. Aging can be categorized into physiological aging and pathological aging. The first refers to the physiological degradation after maturation phase, while the second is aging-related changes induced by various external factors (including various diseases). Aging is the inevitable outcome of the combined effects of numerous pathological, physiological, and psychological processes, and the final biological and psychological process of an individual's growth and development.

Anti-aging refers to employing various methods to postpone function decline across bodily systems, and maintain health and extend lifespan. Anti-aging aims to prevent or mitigate age-related physiological changes, such as the accumulation of cellular damages and disturbance of gene expression. These changes may lead to organ function decrease, reduced metabolic rates, and other problems.

In senescent cells, β-galactosidase accumulates extensively, swelling of lysosomes occurs and a shift of optimal pH to 6.0 occurs, and the activity significantly increases. It is currently one of the most widely used markers in cellular senescence.

Telomeres are special structures at the ends of chromosomes. The length gradually shortens with cell division. Telomerase is an enzyme for repairing and extending telomere length. During youth, high telomerase activity maintains telomere stability, thereby preserving cell health and vitality. However, with the growth of age, telomerase activity gradually declines and telomere length progressively shortens, ultimately leading to cellular senescence and decline of body function. Therefore, by enhancing telomerase activity, the telomere length can be extended, cellular aging can be reversed, and body circulation can be restored. This helps maintain vascular elasticity and cellular viability, ultimately delaying the process of cellular senescence.

The anti-aging described herein involves inhibiting β-galactosidase activity and/or increasing telomerase activity.

The present disclosure provides a use of the L-β-galactoglucan, the Agrobacterium fermentation broth containing the L-β-galactoglucan, or the crude L-β-galactoglucan polysaccharide in the manufacture of products with at least one of the functions of hydrating and moisturizing, enhancing skin elasticity, or inhibiting melanin, including but not limited to skin care products.

The effect of a sample on enhancing skin elasticity can be determined through testing the relative expression levels of gene related to skin elasticity (skin collagen genes collala, collalb, colla2). The study shows that after the use of

products which promote skin elasticity, the relative expression levels of collala, collalb, and colla2 genes significantly increase compared to the control group.

The product described herein is one that upregulates the relative expression levels of collala and col1a1b genes.

Hydration and moisturization can maintain skin moisture balance, improve dryness and roughness, reduce fine lines, enhance skin radiance, and alleviate tightness.

Melanin is a dark brown pigment in plants and animals. It is produced by a type of special cells named melanocytes (also known as melanoblasts) and stored in such cells. It is precisely the presence of melanin that gives skin its color. The production of melanin is often related to endocrine disorders and exposure to sunlight. The number of melanocytes is primarily determined by genetics, and is also related to endocrine hormones and nutritional status. The diseases caused by melanin include freckles, chloasma, and melanosis. The functions such as whitening and freckle removal can be achieved to a certain extent through inhibiting melanin production.

The present disclosure provides the use of the L-β-galactoglucan, the Agrobacterium fermentation broth containing the L-β-galactoglucan, or the crude L-β-galactoglucan polysaccharide in the manufacture of a product for improving the health status of domestic animals.

Further, the domestic animals are mammals.

Further, the mammals are selected from livestock and pets; for example, the livestock includes pigs, cattle, and sheep; the pets include dogs and cats.

Wherein, the improvement of the health status of livestock refers to one or more of the following: promoting growth, enhancing immune function, improving the intestinal microbial flora, or reducing diarrhea.

In one specific embodiment of the present disclosure, the diarrhea rate in weaned piglets can be effectively reduced to promote piglet growth through the use of the L-β-galactoglucan, the Agrobacterium fermentation broth containing the L-β-galactoglucan, or the crude L-β-galactoglucan polysaccharide.

In one specific embodiment of the present disclosure, the dosage of L-β-galactoglucan for improving the health status of livestock is 400 to 600 grams per ton of regular feed. The above dosage refers to the dosage of crude polysaccharide (with a total sugar purity of approximately 60% or higher) in the feed. If converted to purified polysaccharide (with a total sugar purity of 90% or higher), the dosage is approximately 240 to 470 grams per ton.

The improvement of the health status of pets refers to one or more of the following: reducing obesity, improving palatability, increasing relative abundance of intestinal probiotic bacteria, improving stool shape, and improving hair quality.

In one specific embodiment of the present disclosure, the term "pet" refers to the dog.

In one specific embodiment of the present disclosure, the addition percentage of the L-β-galactoglucan in the daily ration for improving the health status of pets is 0.25-0.5%. The above dosage refers to the dosage of crude polysaccharide (with a total sugar purity of approximately 60% or higher) in the daily diet. If converted to purified polysaccharide (with a total sugar purity of 90% or higher), the dosage is approximately 0.15-0.40%.

Palatability refers to the attractiveness and acceptability of feed or food to animals in terms of taste. Animals determine their preference for a particular food based on characteristics such as taste, flavor, odor, and texture. The characteristic is named as palatability.

Enhancing feed palatability is of great significance for improving animal health. It is primarily manifested in the following aspects:

(1) Promoting appetite and feed intake: The feed with good palatability stimulates the animal's appetite, and makes them more willing to eat. This helps animals consume more nutrients to meet their growth and development demands.

(2) Enhancing nutrient absorption rate: If the feed tastes good and is easily digestible, the nutrient absorption rate will be improved accordingly. This enables animals to utilize nutrients from the feed more efficiently, reduces waste, and enhances growth rate and overall health.

(3) Reducing gastrointestinal diseases: Good palatability reduces irritation of the digestive tract by feed and lowers the incidence of gastrointestinal diseases. For instance, feed that is overly coarse or indigestible may cause gastrointestinal diseases and adversely affect health and production performance.

(4) Improving animal welfare: Improving feed palatability also contributes to animal welfare. When animals consume palatable, nutrient-rich food, their quality of life and sense of well-being are improved. This helps reduce stress responses, improve their production performance and overall health.

(5) Promoting healthy growth for animals: After the enhancement of feed palatability, animals can consume more nutrients and utilize them more efficiently. This supports healthy growth for animals, improves production performance indicators such as body weight, meat yield, and milk production.

The improvement of hair quality for pets indicates healthier skin and fur. It is typically reflected in hair luster, softness, density, and skin health condition.

For feeding pets (e.g., dogs, cats) and livestock (e.g., pigs, cattle, sheep), the demands of weight reduction or

gain primarily stem from their distinct physiological characteristics, feeding objective, and health considerations. In particular, for feeding pets such as dogs and cats, it is often required to reduce body weight:

(1) Health factors: Excessive body weight in pets can lead to a series of health issues, including heart disease, joint disorders, diabetes, and others. Therefore, a key objective in pet feeding is to maintain the animal's body weight within a healthy range.
(2) Living habits: Pets typically require more activity and playtime. Excess weight can impair their mobility and reduce their quality of life.
(3) Breed characteristics: Certain pet breeds are naturally prone to being overweight. Therefore, owners of these breeds must pay closer attention to dietary control to prevent excessive weight gain.

During the feeding of livestock such as pigs, cattle, sheep, the body weights of livestock often increase.

(1) Economic value: Weight gain in livestock is directly related to their economic worth. For instance, increased body weight in pigs, cattle, and sheep enhances meat quality and yield, thereby increasing farmers' income.
(2) Purpose of feeding: Livestock are primarily fed for meat, dairy, or other agricultural products. Thus, body weight gain is essential to meet these demands.
(3) Growth characteristics: Livestock require substantial nutritional support during the growth to meet the needs for growth and development. Promoting body weight gain and enhancing production efficiency can be achieved by providing adequate feed and nutrition.

Common skin care products include, but are not limited to hydrating sprays, moisturizing sprays, toner, skin-boosting serums, essences, lotions, creams, facial cleansers, masks, and sunscreens.
Skin care products may contain the active ingredients of the present disclosure, such as the L-β-galactoglucan, the Agrobacterium fermentation broth containing the L-β-galactoglucan, or the crude L-β-galactoglucan polysaccharide, as well as commonly used components such as:

Moisturizer: It is used to provide skin with essential moisture, and it includes hyaluronic acid (hyaluronic acid), glycerin, butylene glycol, trehalose, and other moisturizers. These components lock in moisture and maintain skin hydration.
Whitening ingredients: The common components include niacinamide (vitamin B3), arbutin, vitamin C (ascorbic acid), etc. These ingredients inhibit melanin production and brighten the skin color.
Anti-aging ingredients: It contains collagen, peptides (e.g., palmitoyl pentapeptide-4), vitamin E, retinol (a form of vitamin A), etc. These ingredients stimulate skin cell regeneration, enhance skin elasticity, and reduce striates and wrinkles.
Soothing ingredients: They may contain allantoin, aloe vera extract, chamomile extract, etc. These ingredients alleviate skin irritation and soothe redness and discomfort.
Antioxidants: They are used to protect the skin from free radical damage. Generally, they contain antioxidants such as green tea extract, grape seed extract, vitamin C, etc. These ingredients protect the skin from environmental pollution and UV damage.
Cleansing agents: Common components of cleaning agent include carbon black, kaolin clay, bentonite clay, etc. These ingredients absorb grease and dirt on the skin and deeply cleanse pores.
Preservative: It is required to add preservatives to extend the product's shelf life. Common preservatives include phenoxyethanol and methyl hydroxybenzoate. However, excessive use of preservatives may irritate the skin.
Astringents: Astringents such as aluminum chloride and citric acid help to tighten pores.
Emulsifier: The emulsifier helps to blend the aqueous and oil phases to form a stable emulsion.
Stabilizers: They are used to prevent product stratification or degradation.
Water phase: It is primarily water and may also contain water-soluble moisturizers.
Oil phase: It includes ingredients such as jojoba oil and squalane to provide oil and protective effect for skin.
Surfactants: Their main functions include cleansing and foaming. Common surfactants include soap bases, alkyl sulfates, and N-acyl methyl taurates.
Other additives: These include fragrances and colorants which are used to enhance the product's appearance and scent.

In the present disclosure, the product forms include but are not limited to pharmaceuticals, health supplements, food, cosmetics, skin care products, veterinary drugs, feed, feed additives, etc.
The products of the present disclosure include at least one of the L-β-galactoglucan, the Agrobacterium fermentation broth containing the L-β-galactoglucan, or the crude L-β-galactoglucan polysaccharide.
Appropriate excipients may be added to the products of the present disclosure as required.

The excipients referred to in the present disclosure are the general name for all additional materials other than the main components. These excipients shall have the following properties: (1) They have no toxic or harmful effects on the body, with negligible side effects; (2) They have stable chemical property, and they are not easily affected by temperature, pH, storage duration, etc.; (3) They have no incompatibility with the main drug, and they have no impact on the efficacy or quality assessment of the main drug; (4) They do not interact with package materials. The excipients in the present disclosure include but are not limited to fillers (dilutants), lubricants (flow aids or anti-caking agents), dispersants, wetting agents, binders, modifiers, solubilizers, antioxidants, antimicrobial agents, emulsifiers, disintegrants, etc. Binders include syrups, gum arabic, gelatin, sorbitol, gum tragacanth, cellulose and its derivatives (e.g., microcrystalline cellulose, sodium carboxymethyl cellulose, ethyl cellulose, or hydroxypropyl methylcellulose), gelatin paste, starch slurry, or polyvinylpyrrolidone; the fillers include lactose, sugar powder, dextrin, starch and its derivatives, cellulose and its derivatives, inorganic calcium salts (e.g., calcium sulfate, calcium phosphate, calcium hydrogen phosphate, precipitated calcium carbonate), sorbitol, or glycine; the lubricants include colloidal silicon dioxide, magnesium stearate, talc, aluminum hydroxide, boric acid, hydrogenated vegetable oil, polyethylene glycol, etc.; the disintegrants include starch and its derivatives (e.g., sodium carboxymethyl starch, sodium starch glycolate, pregelatinized starch, modified starch, hydroxypropyl starch, corn starch, etc.), polyvinylpyrrolidone, or microcrystalline cellulose, etc.; the wetting agents include sodium lauryl sulfate, water, or alcohols; the antioxidants include sodium sulfite, sodium bisulfite, sodium metabisulfite, butyl benzoate, etc.; the antiseptics include 0.5% phenol, 0.3% cresol, 0.5% trichlorobutanol, etc.; the adjusting agents include hydrochloric acid, citric acid, potassium (sodium) hydroxide, sodium citrate, and buffering agents (including sodium dihydrogen phosphate and disodium hydrogen phosphate); the emulsifiers include polysorbate-80, sorbitan oleate, poloxamer F-68, lecithin, and soy lecithin; the solubilizers include Tween-80, bile salts, and glycerin.

The administration methods for the L-β-galactoglucan, the Agrobacterium fermentation broth containing the L-β-galactoglucan, and the crude L-β-galactoglucan polysaccharide of the present disclosure are not particularly limited. Representative administration methods include, but are not limited to: oral, parenteral (intravenous, intramuscular, or subcutaneous), and topical administration.

Solid dosage forms for oral administration include capsules, tablets, pills, powders, and granules. In these solid dosage forms, the active compound is blended with at least one of the conventional inert excipients (or carriers), such as sodium citrate or dicalcium phosphate, or with the following components: (a) Fillers or bulking agents, e.g., starch, lactose, sucrose, glucose, mannitol, and silicic acid; (b) Binders such as hydroxymethyl cellulose, alginates, gelatin, polyvinylpyrrolidone, sucrose, and gum arabic; (c) Humectants such as glycerin; (d) Disintegrants such as agar, calcium carbonate, potato starch or tapioca starch, alginic acid, certain composite silicates, and sodium carbonate; (e) Retarding agents such as paraffin; (f) Absorption accelerators such as quaternary ammonium compounds; (g) Wetting agents such as cetyl alcohol and glyceryl monostearate; (h) Adsorbents, e.g., kaolin; and (i) Lubricants such as talc, calcium stearate, magnesium stearate, solid polyethylene glycol, sodium lauryl sulfate, or mixtures thereof. For capsules, tablets, and pills, the formulations may also include buffering agents.

Solid dosage forms such as tablets, sugar pills, capsules, pills, and granules may be prepared using coatings and shell materials, such as enteric coatings and other materials known in the field. They may contain opacifiers, and the release of the active compound or compounds in such compositions may be delayed in a specific portion of the digestive tract. Examples of suitable encapsulating components are polymeric substances and wax-like substances. If necessary, the active compound may also be microencapsulated with one or more of the above excipients.

Liquid dosage forms for oral administration include pharmaceutically acceptable emulsions, solutions, suspensions, syrups, or tinctures. In addition to the active compound, the liquid dosage forms may include inert diluent conventionally employed in the field, such as water or other solvents, solubilizer, and emulsifier, for example, ethanol, isopropanol, ethyl carbonate, ethyl acetate, propylene glycol, 1,3-butanediol, dimethylformamide, and oil, especially cotton seed oil, peanut oil, corn germ oil, olive oil, castor oil, and sesame oil, or mixtures thereof.

In addition to such inert diluents, the compositions may also include adjuvants such as wetting agents, emulsifiers, and suspending agents, sweeteners, flavoring agents, and fragrances.

Besides the active compound, the suspension may contain suspending agents such as ethoxylated isodecyl alcohol, polyoxyethylene sorbitan and dehydrated sorbitan esters, microcrystalline cellulose, aluminum methoxide, and agar, or mixtures thereof.

Dosage forms for topical administration of the compounds of the present disclosure include ointments, powders, patches, sprays, and inhalants. The active ingredient is mixed under aseptic conditions with a physiologically acceptable carrier and any necessary preservatives or buffers, and with propellants if required.

The primary dosage forms for skin repair include the following:

1. Creams, defined as uniform semi-solid dosage forms formed by dissolving or dispersing the drug substance in an emulsion-type base.
Excipients in creams typically include emulsifiers, stabilizers, humectants, preservatives, etc.
2. Gels, a semi-solid dosage form characterized by flowability and thixotropy under specific conditions.

Excipients for gels include gel matrix, humectant, preservative, etc. For example, recombinant human epidermal growth factor gel is a gel dosage form which is used to promote skin wound healing.
3. Solutions are clear liquid dosage form prepared by dissolving the active ingredient in a suitable solvent.
Excipients for solutions primarily include solvents, preservatives, and stabilizers. Solutions are suitable for cleansing, anti-itching, disinfection, and convergence.
4. Ointments are semi-solid topical dosage forms with drugs dissolved or dispersed in suitable matrix.
Ointment excipients primarily include bases, preservatives, and humectants. Ointments are commonly used for chronic inflammatory skin lesions such as eczema and dermatitis.
5. Dressings are materials applied to the surface of skin wounds to protect the wound surface, absorb exudate, and promote healing.
Dressings come in various types such as gauze, petrolatum gauze, synthetic fiber cloth, hydrocolloid dressing, and hydrogel dressing. Different dressings are suitable for various types of wounds and stages of healing.
6. Topical sprays are liquid dosage forms delivered via specialized devices that atomize medicinal solutions, emulsions, or suspensions for external application to the skin and mucous membranes.

Excipients in topical sprays include solvents, propellants, stabilizers, etc. This dosage form is suitable for drugs requiring uniform distribution across wound surfaces.
Among veterinary products, common dosage forms include but are not limited to:

(1) Liquid dosage form: It refers to a drug, feed, or feed additive in liquid state, such as solution or suspension. This dosage form is suitable for rapid administration or addition to drinking water, to facilitate self-administration by animals.
(2) Semi-solid dosage form: It refers to a drug, feed, or feed additive in a semi-solid state, such as an ointment or a paste. The dosage form is suitable for topical administration or application on animal skin.
(3) Solid dosage form: It refers to a drug, feed, or feed additive in a solid state, such as a tablet, a granule, or a pill. This dosage form is suitable for direct feeding or incorporation into feed; it has advantages in storage and transportation.
(4) Microcapsule dosage form: It refers to a drug, feed, or feed additive encapsulated in a microscopic capsule. The dosage form enables controlled release rate and location of the drug, and enhances stability and bioavailability.

Veterinary solution are pharmaceuticals formed by dissolving non-volatile active ingredients in specific solvents (such as water, alcohol or oil). They are suitable for oral or topical administration. Solutions of the dosage form are generally clear, but oil-soluble solutions may be cloudy.
Common excipients include:

1. Solvents:

Water: It is the most common solvent and is used to prepare aqueous solutions.
Ethanol: It is used to prepare alcoholic solutions. Sometimes, it is used to enhance drug solubility.
Glycerin: It serves as an oily solvent for preparing oil-based solutions.

2. Stabilizers:

Antioxidants: Such as vitamin E and sodium sulfite, they are used to prevent oxidation and deterioration of drugs.
Preservatives: Such as sodium benzoate, potassium sorbate, they are used to extend the shelf life of solutions.

3. Buffer agents:
They are used to regulate the pH of solutions and maintain drug stability. They include phosphate-buffered saline and acetate buffer.
4. Surfactants:
The surfactants such as Polysorbate 80, sodium lauryl sulphate are used to improve drug solubility and dispersibility.
5. Cosolvents:
Cosolvents are used to aid the dissolution of poorly soluble drugs in water, such as urea and polyethylene glycol.
6. Solubilizers:
The solubilizers such as Tween series and Poloxamer are used to increase drug solubility in solutions.
7. Flavoring agents:
The flavoring agents are used to improve the taste of solutions, such as stevioside and citric acid.
8. Colouring agents:
Colouring agents such as edible pigments are used to add color to solution for identification and differentiation.

Veterinary suspensions refer to suspensions with suspended drugs in a water or oil base to form stable suspensions suitable for animal administration. The roles of suspensions in veterinary drug applications:

(1) Extending the storage period: Suspensions effectively maintain drug stability and preserve high quality over extended periods.
(2) Enhancing drug quality: Suspensions provide excellent protection for critical drug attributes such as cellular integrity (cytoplasm), pH, and nutrient content. This reduces quality issues during subsequent use and effectively improves treatment effect.
(3) Easy to use: The suspension improves drug viscosity, and minimizes unnecessary difficulties. During animal medication, the suspension enhances drug viability and activity, further improves treatment effect.

In addition to solvent excipients, common suspension excipients also include those that help solid drug particles to be uniformly suspended in solution, such as:

(1) Physical suspension agents: These primarily form a dense dispersed phase via polymer action. It inhibits or prevents solid particle agglomeration. Common physical suspension agents include sodium carboxymethylcellulose (CMC), sodium pectin, and gelatin. As 10 examples are required, but common excipient types are insufficient to list 10 examples, only some of the examples are provided below.
(2) Physicochemical suspension agents: These form a uniformly thick shell layer through physicochemical reactions or interactions. Common physicochemical suspending agents include magnesium aluminum silicate, activated silicate, and liquid paraffin.
(3) Surfactants: By creating a dispersed phase through adsorption onto solid surfaces, they prevent particle movement and form a highly dispersed system. Surfactants are primarily classified into anionic, cationic, nonionic, and amphoteric surfactants. Specific examples may include various anionic surfactants (e.g., sodium dodecyl sulfate), cationic surfactants (e.g., cetyltrimethylammonium bromide), nonionic surfactants (e.g., polyoxyethylene alkylphenol ether), and amphoteric surfactants (e.g., dodecyl betaine).
(4) Biologically derived suspending agents: These primarily achieve dispersion by leveraging the characteristics of biological reactions to influence particle charge properties and stability. Common biologically derived suspending agents include bovine serum albumin and ovalbumin.

Additionally, they include special-function excipients such as thickeners, chelating agents, and pH adjusters, with specific selection depending on drug properties and application requirements.

Veterinary ointments refer to semi-solid topical dosage forms produced by uniformly mixing drugs with suitable bases to achieve appropriate consistency. These formulations are typically ointments of high consistency, made by mixing drugs with excipients such as petroleum jelly or oils, primarily intended for external use. The excipients in ointments primarily include:

1. Bases

Petrolatum: It is common oleaginous base with excellent moisturizing and lubricating properties.
Lanolin: It is a kind of natural animal fat with good moisturizing and stabilizing properties.
Octadecanoic acid: It is a commonly used oleaginous base, which is often combined with glyceryl stearate and similar compounds.
Paraffin wax: It is a solid hydrocarbon mixture that increases ointment viscosity and stability.
Vegetable oils: Vegetable oils such as soybean oil and peanut oil serve as oleaginous bases for ointments.

2. Thickening agents:

Magnesium aluminum silicate: It has adsorbent and thickening properties, and it is commonly used to adjust ointment consistency.
Sodium carboxymethylcellulose (CMC): It is a water-soluble cellulose derivative that can serve as a thickener for oil-in-water ointments.
Polyvinyl alcohol (PVA): It is a polymer that can be used to adjust the viscosity and consistency of ointments.

3. Preservatives:

Benzoic acid: It is a commonly used acidic preservative with inhibitory effects against various microorganisms.
Ethylparaben: It is a broad-spectrum antimicrobial agent effective against both fungi and bacteria.

Propylene glycol: It has preservative and moisturizing effects. And it is commonly used in ointments.

4. Humectants:

Glycerin: It has strong hygroscopicity. It forms hydrogen bonds with water to retain skin moisture.
Glycerol: It is similar to glycerin, and it provides excellent moisturizing effects.

5. Antioxidants:

Vitamin E: It is an antioxidant that prevents drug oxidation and deterioration.
Butylated hydroxyanisole (BHA): It is a commonly used antioxidant to prevent oxidation in oleaginous bases.

Veterinary pastes are semi-solid paste formulations with high hardness, significant water absorption capacity, and low greasiness. There are certain differences between pastes and ointments:

1. Texture and appearance:

Ointment: It is a semi-solid, milky-white gel-like substance. It is typically smooth and easy to apply.
Paste: It is a viscous liquid or semi-solid substance with higher hardness, generally firmer and drier than ointments, with lower greasiness and occlusive properties.

2. Purpose:

Ointment: It is primarily used to treat minor burns, abrasions, and other small-area skin injuries to alleviate pain and promote healing.
Paste: It is primarily suitable for the treatment of chronic skin conditions such as subacute dermatitis or eczema, with moisture-absorbing, drying, and anti-itch effects. It is also commonly applied to large-area wounds or infected surfaces, such as postoperative incisions.

3. Method of application:

Ointment: It is typically applied topically by spreading directly onto the affected area and gently massage until it is absorbed.

Paste: Apply an appropriate amount of paste directly to the wound surface, then secure with sterile gauze.

4. Key differences in excipients:

(1) Bases:

Ointment: Common bases include petrolatum, lanolin, and vegetable oil, which form softer, finer-textured ointments.
Paste: The bases are often hydrocarbon-based or water-in-oil emulsion bases. Pastes prepared by mixing with powders are typically harder and drier.

(2) Thickening agents:
Ointment: Thickening agents are used relatively sparingly, as ointments themselves have adequate viscosity.

Paste: As pastes require higher hardness and greater water absorption capacity, they may contain more thickening agents such as magnesium aluminum silicate and sodium carboxymethyl cellulose.
Solid dosage forms include capsules, tablets, pills, powders, and granules. In these solid dosage forms, the active compound is blended with at least one of the conventional inert excipients (or carriers), such as sodium citrate or dicalcium phosphate, or with the following components: (a) Fillers or bulking agents, such as starch, lactose, sucrose, glucose, mannitol, and silicic acid; (b) Binders, such as hydroxymethyl cellulose, alginates, gelatin, polyvinylpyrrolidone, sucrose, and gum arabic; (c) Humectants, such as glycerin; (d) Disintegrants, such as agar, calcium carbonate, potato starch or tapioca starch, alginic acid, certain composite silicates, and sodium carbonate; (e) Retarding solvents, such as paraffin; (f) Absorption accelerators, such as quaternary ammonium compounds; (g) Wetting agents, such as cetyl alcohol and glyceryl monostearate; (h) Adsorbents, such as kaolin; and (i) Lubricants, such as talc, calcium stearate, magnesium

stearate, solid polyethylene glycol, sodium lauryl sulfate, or mixtures thereof.

A veterinary microcapsule dosage form refers to microcapsules in which a solid or liquid drug (core material) is encapsulated with natural or synthetic polymeric materials (wall materials). The dosage form is prepared via a specific process, so that the drug is embedded in a polymeric carrier at high temperatures, and then rapidly spray-cooled to form uniform drug-loaded microspheres. These microspheres can achieve sustained, controlled, or enteric release of the drug in animals under the influence of specific triggers such as certain pH levels or intestinal lipase activity.

The excipients for veterinary microcapsules primarily include:

1. Polymeric carriers:
Such as: Polylactic acid (PLA), poly(lactic-co-glycolic acid) (PLGA), gelatin, and chitosan. These polymeric materials exhibit excellent biocompatibility and degradability, serving as the primary carrier materials for microcapsules.
2. Emulsifiers:
Such as: Polysorbate 80 (Tween 80) and sodium lauryl sulphate (SDS). Emulsifiers play a role in reducing interfacial tension, promoting drug dispersion, and stabilizing microcapsule structure during the preparation of microcapsule.
3. Thickening agents:
Such as: methylcellulose, hydroxyethyl cellulose, and polyvinyl alcohol (PVA). Thickeners enhance the viscosity and stability of the microcapsule formulation, preventing drug precipitation and stratification during preparation and storage.
4. Stabilizers:
Such as: mannitol, sorbitol, and disodium hydrogen phosphate. Stabilizers prevent physical and chemical changes in the microcapsule formulation during storage and use, ensuring drug stability and efficacy.
5. Antioxidants:
Such as: vitamin E, butylated hydroxyanisole (BHA), and propyl gallate. Antioxidants inhibit oxidative degradation of the drug in the microcapsule formulation, thereby extending the shelf life of the formulation.

The beneficial effects of the present disclosure are as follows:

(1) The present disclosure provides an L-β-galactoglucan with a novel structure;
(2) In the present disclosure, the polysaccharide fermentation process is simple and low-cost, making it suitable for industrial production;
(3) The wastewater generated during fermentation contains no toxic substances and can be reused;
(4) The polysaccharide of the present disclosure is effective not only in the treatment and/or prevention of disorders of glucose metabolism and liver injury-related diseases, but also possesses anti-aging, skin-repairing, and animal health-improving efficacy.

**Figure descriptions**

Figure 1 shows TLC results of acid-hydrolyzed L-β-galactoglucan and monosaccharide standards;
Figure 2 shows HPLC detection chromatograms of acid-hydrolyzed L-β-galactoglucan and monosaccharide standards;
Figure 3 shows FT-IR spectrum of L-β-galactoglucan;
Figure 4 shows GC chromatogram of acetylated L-β-galactoglucan;
Figure 5 shows the total ion current chromatogram of the alditol acetate derivatives of methylated L-β-galactoglucan;
Figure 6 shows 1H-NMR spectrum of L-β-galactoglucan(500 MHz, 298K);
Figure 7 shows 13C-NMR spectrum of L-β-galactoglucan (500 MHz, 298K);
Figure 8 shows DEPT135 spectrum of L-β-galactoglucan (500 MHz, 298 K);
Figure 9 shows COSY spectrum of L-β-galactoglucan (500 MHz, 298 K);
Figure 10 shows HSQC spectrum of L-β-galactoglucan. (500 MHz, 298K);
Figure 11 shows HSQC spectrum of L-β-galactoglucan. (500 MHz, 298K);
Figure 12 shows the glucose levels of zebrafish after treatment with L-β-galactoglucan in Efficacy Test 1;
Figure 13 shows fluorescence intensity of zebrafish pancreatic β-cells after L-β-galactoglucan treatment in Efficacy Test 2;
Figure 14 shows representative fluorescence intensity profile of zebrafish pancreatic β-cells after L-β-galactoglucan treatment in Efficacy Test 2;
Figure 15 shows bar graph of zebrafish pancreatic β-cell fluorescence intensity after L-β-galactoglucan treatment in Efficacy Test 2;

Figure 16 shows blood glucose line chart of mice after L-β-galactoglucan treatment in Efficacy Test 3;

Figure 17 shows schematic diagram of zebrafish tail fin length analysis after L-β-galactoglucan treatment in Efficacy Test 4;

Figure 18 shows representative zebrafish tail fin length after L-β-galactoglucan treatment in Efficacy Test 4;

Figure 19 shows bar chart of zebrafish tail fin length after L-β-galactoglucan treatment in Efficacy Test 4;

Figure 20 shows representative image of zebrafish wound site vascular regeneration after L-β-galactoglucan treatment in Efficacy Test 5;

Figure 21 shows incidence of neovascularization of zebrafish after L-β-galactoglucan treatment in Efficacy Test 5;

Figure 22 shows schematic diagram of apoptotic cells clearance analysis at the injury site of zebrafish after L-β-galactoglucan treatment in Efficacy Test 6;

Figure 23 shows representative image of apoptotic cells at the injury site of zebrafish after L-β-galactoglucan treatment in Efficacy Test 6;

Figure 24 shows bar chart of fluorescence intensity of apoptotic cells at the tissue injury site of zebrafish after L-β-galactoglucan treatment in Efficacy Test 6;

Figure 25 shows representative fluorescence intensity image of zebrafish macrophages after L-β-galactoglucan treatment in Efficacy Test 7;

Figure 26 shows bar chart of fluorescence intensity in zebrafish macrophages after L-β-galactoglucan treatment in Efficacy Test 7;

Figure 27 shows schematic diagram of residual fluorescent microsphere count analysis in zebrafish from Efficacy Test 8;

Figure 28 shows a typical graph of residual fluorescent microspheres count in zebrafish after L-β-galactoglucan treatment in Efficacy Test 8;

Figure 29 shows residual fluorescent microspheres count in zebrafish after L-β-galactoglucan treatment in Efficacy Test 8;

Figure 30 shows schematic diagram of zebrafish T-cell fluorescence intensity analysis in Efficacy Test 9;

Figure 31 shows representative fluorescence intensity image of zebrafish T cells after L-β-galactoglucan treatment in Efficacy Test 9;

Figure 32 shows bar chart of fluorescence intensity in zebrafish T cells after L-β-galactoglucan treatment in Efficacy Test 9;

Figure 33 shows representative image of neutrophils count in zebrafish tail blood vessels after L-β-galactoglucan treatment in Efficacy Test 10;

Figure 34 shows image of neutrophils count in zebrafish tail blood vessels after L-β-galactoglucan treatment in Efficacy Test 10;

Figure 35 shows AST activity diagram of zebrafish tissues after L-β-galactoglucan treatment in Efficacy Test 11;

Figure 36 shows ALT activity diagram of zebrafish tissues after L-β-galactoglucan treatment in Efficacy Test 12;

Figure 37 shows a typical image of liver tissue structure of zebrafish after L-β-galactoglucan treatment in Efficacy Test 13;

Figure 38 shows the bar chart of ROS fluorescence value of zebrafish after L-β-galactoglucan treatment in Efficacy Test 14;

Figure 39 shows the bar chart of ATP chemoluminescence value of zebrafish after L-β-galactoglucan treatment in Efficacy Test 15;

Figure 40 shows the bar chart of lactic acid content in zebrafish after L-β-galactoglucan treatment in Efficacy Test 16;

Figure 41 shows the schematic diagram of fluorescence intensity analysis of gut microbiota in zebrafish after L-β-galactoglucan treatment in Efficacy Test 17;

Figure 42 shows a typical fluorescence intensity profile of the intestinal microbiota in zebrafish after L-β-galactoglucan treatment in Efficacy Test 17;

Figure 43 shows a bar chart of Lactobacillus brevis fluorescence intensity in zebrafish intestine after L-β-galactoglucan treatment in Efficacy Test 17;

Figure 44 shows schematic diagram of tail area analysis for zebrafish after L-β-galactoglucan treatment in Efficacy Test 18;

Figure 45 shows typical diagram of tail area analysis for zebrafish after L-β-galactoglucan treatment in Efficacy Test 18;

Figure 46 shows tail area analysis for zebrafish after L-β-galactoglucan treatment in Efficacy Test 18;

Figure 47 shows schematic diagram of β-galactosidase staining intensity analysis for zebrafish after L-β-galactoglucan treatment in Efficacy Test 19;

Figure 48 shows typical diagram of β-galactosidase staining intensity for zebrafish after L-β-galactoglucan treatment in Efficacy Test 19;

Figure 49 shows β-galactosidase staining intensity for zebrafish after L-β-galactoglucan treatment in Efficacy Test 19;

Figure 50 shows the telomerase activity of zebrafish after L-β-galactoglucan treatment in Efficacy Test 20;
Figure 51 shows the relative expression levels of the collala gene in Efficacy Test 21;
Figure 52 shows the relative expression levels of col1a1b gene in Efficacy Test 21;
Figure 53 shows schematic diagram of melanin signal intensity analysis sites at tissue injury locations in zebrafish after L-β-galactoglucan treatment in Efficacy Test 22;
Figure 54 shows representative image of melanin signal intensity at tissue injury sites in zebrafish after L-β-galactoglucan treatment in Efficacy Test 22;
Figure 55 shows bar chart of melanin signal intensity at tissue injury sites in zebrafish after L-β-galactoglucan treatment in Efficacy Test 22;
Figure 56 shows bacteria abundance composition at the TOP 10 phylum level across groups;
Figure 57 shows bacteria abundance composition at the TOP 30 genus level across groups;
Figure 58 shows LEfSe analysis and LDA score histogram of L-β-galactoglucan effects on colonic microbiota composition in weaned piglets. It shows biomarker clusters (LDA score > 2);
Figure 59 shows fecal microbial composition. A: Fecal microbial genus-level Venn diagram; B: Fecal microbial β-diversity (PCOA analysis); C: Phylum-level community composition; D: Genus-level community composition; n = 8;
Figure 60 shows the impact of biopolysaccharides on the relative expression level of the IL-6 gene in Malassezia-induced HaCaT cells.

Specific embodiments

To clarify the objectives, technical scheme, and advantages of the present disclosure, the following detailed description is provided with reference to specific embodiments and efficacy tests. It should be understood that the specific embodiments and efficacy tests described herein are intended solely to illustrate the present disclosure and are not intended to limit the scope of the present disclosure. Furthermore, unless otherwise specified, all reagents used in the following embodiments are commercially available or can be synthesized with the methods described herein or known methods. Reaction conditions not listed are readily available to those skilled in the field.

The Agrobacterium sp. FN01 described in the present disclosure was isolated from soil of the western Sichuan Plateau and deposited at the China Center for Type Culture Collection (CCTCC) on March 2, 2023, with the accession number CCTCC No. 2023226, address: Wuhan University, Wuhan, China.

**Example 1 Preparation of L-β-Galactoglucan**

(1) Preparation of Agrobacterium sp. FN01 Seed Liquid

The medium formulation comprises 1.0% peptone, 0.5% yeast powder, and 1.0% sodium chloride dissolved in potable tap water. The pH was adjusted to 6.5-7.5 using sodium hydroxide solution. After sterilization at 121°C for 30 min, the cooled medium was inoculated with Agrobacterium sp. FN01 and aerobically stirred for 20 h to obtain the FN01 seed culture.

(2) Preparation of Culture Broth

Dissolve 3.0% white sugar, 0.2% sodium nitrate, 0.1% peptone, 0.2% potassium dihydrogen phosphate, 0.02% magnesium sulfate, 0.002% ferrous sulfate, 0.0012% manganese sulfate, 0.01% calcium chloride, and 0.05% defoamer in potable tap water. Adjust the pH to 6.7 using sodium hydroxide solution. Autoclave at 121°C for 30 min, cool, and prepare the culture broth.

(3) Preparation of Fermentation Broth

Inoculate the seed culture into the culture broth at a rate of 5% of the total inoculum size. Fermentation conditions are as follows: 1) Temperature is 28-32°C; 2) pH is 6.5-7.5 (adjusted with diluted HCl or NaOH solution); 3) The aeration rate:: 0-24 h, 0.2-0.4 vvm; 24-48 h, 0.5-0.7 vvm; 0.9-1.0 vvm from 48 h until fermentation completion; 4) Stirring speed: 0-24 h, 60 rpm; 24-48 h, 100 rpm; 150 rpm from 48 h until fermentation completion. 65 h after the fermentation, fermentation broth containing the L-β-galactoglucan is prepared.

(4) Preparation of Crude L-β-Galactoglucan

Add 2.5 volumes of 95% ethanol to the fermentation broth to precipitate the fermentation broth. Dry the precipitate to obtain the crude L-β-galactoglucan.

(5) Purification of L-β-galactoglucan

Dissolve the crude L-β-galactoglucan in water to form a 1.5% solution, then add 0.1% sodium hydroxide and mix thoroughly. Heat to 85°C, then add 1.5% diatomite and mix thoroughly. Remove impurities through filtration with plate and frame filter, collect the filtrate, and add 3.5 volumes of 95% ethanol to precipitate the polysaccharide; press the precipitate dry, dry at 60°C, and grind to obtain pure L-β-galactoglucan solid powder. The total sugar content is measured to be 91.7% according to the National Standard GB/T 15672-2009 "Determination of Total Sugar Content in Edible Fungi.".

**Example 2 Preparation of L-β-Galactoglucan**

(1) Preparation of Agrobacterium sp. FN01 Seed Liquid

The medium formulation comprises 1.0% peptone, 0.5% yeast powder, and 1.0% sodium chloride dissolved in potable tap water. The pH was adjusted to 6.5-7.5 using sodium hydroxide solution. After sterilization at 121°C for 30 min, the cooled medium was inoculated with Agrobacterium sp. FN01 and aerobically stirred for 20 h to obtain the FN01 seed culture.

(2) Preparation of Culture Broth

Dissolve 5.0% maltose, 0.3% potassium nitrate, 0.1% peptone, 0.1% potassium dihydrogen phosphate, 0.04% magnesium sulfate, 0.001% ferrous sulfate, 0.0006% manganese sulfate, 0.005% calcium chloride, and 0.2% defoamer in potable tap water. Adjust the pH to 6.7 with sodium hydroxide solution. Autoclave at 121°C for 30 min, cool, and prepare the culture broth.

(3) Preparation of Fermentation Broth

Inoculate the seed culture into the culture broth at a rate of 1% of the total inoculum size. Fermentation conditions are as follows: 1) Temperature is 28-32°C; 2) pH is 6.5-7.5 (adjusted with diluted HCl or NaOH solution); 3) The aeration rate: 0-24 h, 0.2-0.4 vvm; 24-48 h, 0.5-0.7 vvm; 0.9-1.0 vvm from 48 h until fermentation completion; 4) Stirring speed: 0-24 h, 60 rpm; 24-48 h, 100 rpm; 0.9-1.0 vvm from 48 h until fermentation completion. 80 h after the fermentation, fermentation broth containing the L-β-galactoglucan is prepared.

(4) Preparation of Crude L-β-Galactoglucan

Add 3 volumes of 95% ethanol to the fermentation broth to precipitate the fermentation broth. Dry the precipitate to obtain the crude L-β-galactoglucan.

(5) Purification of L-β-galactoglucan

Dissolve the crude L-β-galactoglucan in water to form a 1.5% solution, then add 0.1% sodium hydroxide and mix thoroughly. Heat to 85°C, then add 1.5% diatomite and mix thoroughly. Remove impurities through filtration with plate and frame filter, collect the filtrate, and add 4 volumes of 95% ethanol to precipitate the polysaccharide; press the precipitate dry, dry at 60°C, and grind to obtain pure L-β-galactoglucan solid powder. The total sugar content is measured to be 90.5% according to the National Standard GB/T 15672-2009 "Determination of Total Sugar Content in Edible Fungi.".

**Example 3 Preparation of L-β-Galactoglucan**

(1) Preparation of Agrobacterium sp. FN01 Seed Liquid

The medium formulation comprises 1.0% peptone, 0.5% yeast powder, and 1.0% sodium chloride dissolved in potable tap water. The pH was adjusted to 6.5-7.5 using sodium hydroxide solution. After sterilization at 121°C for 30 min, the cooled medium was inoculated with Agrobacterium sp. FN01 and aerobically stirred for 20 h to obtain the FN01 seed culture.

(2) Preparation of Culture Broth

Dissolve 5.0% maltose, 0.3% potassium nitrate, 0.1% peptone, 0.1% potassium dihydrogen phosphate, 0.04% magnesium sulfate, 0.001% ferrous sulfate, 0.0006% manganese sulfate, 0.005% calcium chloride, and 0.2% defoamer

in potable tap water. Adjust the pH to 6.7 with sodium hydroxide solution. Autoclave at 121°C for 30 min, cool, and prepare the culture broth.

(3) Preparation of Fermentation Broth

Inoculate the seed culture into the culture broth at a rate of 1% of the total inoculum size. Fermentation conditions are as follows: 1) Temperature is 28-32°C; 2) pH is 6.5-7.5 (adjusted with diluted HCl or NaOH solution); 3) The aeration rate: 0-24 h, 0.2-0.4 vvm; 24-48 h, 0.5-0.7 vvm; 0.9-1.0 vvm from 48 h until fermentation completion; 4) Stirring speed: 0-24 h, 60 rpm; 24-48 h, 100 rpm; 0.9-1.0 vvm from 48 h until fermentation completion. 80 h after the fermentation, fermentation broth containing the L-β-galactoglucan is prepared.

(4) Enzymatic Hydrolysis of Fermentation Broth

Adjust the pH of the fermentation broth to 4.5-5.5 with hydrochloric acid, heat to 45-55°C, and add 0.1% glucanase. Maintain continuous stirring throughout the enzymatic hydrolysis process.

(5) Purification of L-β-galactoglucan

Heat the enzymatically hydrolyzed fermentation broth to 85-90°C, add 1.5% diatomite, mix thoroughly, remove impurities through filtration with plate and frame filter, and collect the filtrate. Concentrate the filtrate via ultrafiltration to obtain L-β-galactoglucan concentrate. Spray dry the concentrate to obtain pure L-β-galactoglucan.

**Example 4 Structural Analysis of Polysaccharide**

(1) Polysaccharide Molecular Weight Determination

Analysis revealed the molecular weight distribution of the polysaccharide of the present disclosure is above $1 \times 10^6$ Da, with a weight-average molecular weight of approximately 4,982,327 Da.

(2) Monosaccharide Composition and Content Results

Following acid hydrolysis, neutralization, and concentration of the polysaccharide, TLC and HPLC determination results are shown in Figures 1-2. These indicate that the acid-hydrolyzed polysaccharide solution contains only glucose and galactose, with approximate percentages of 96% and 4%, respectively.

(3) FI-IR determination results

The FI-IR spectrum of the polysaccharide is shown in Figure 3. The figure shows a broad, intense peak at 3436.8 $cm^{-1}$ corresponding to the O-H stretching vibration absorption peak, and the peak at 2887.3 $cm^{-1}$ represents the C-H stretching vibration absorption peak of $-CH_2-$, characteristic peak of carbohydrates. The peak at 1616.0 $cm^{-1}$ corresponds to the carbonyl C=O stretching vibration absorption peak. The broad extension of C-O-C and C-O bonds in the range of 1000 $cm^{-1}$-1200 $cm^{-1}$ shows the presence of carbohydrates. In the FI-IR spectrum of the exopolysaccharide, absorption peaks at 1043.5 $cm^{-1}$, 1073.0 $cm^{-1}$, and 1161.0 $cm^{-1}$ correspond to C-O stretching vibrations. The absorption peak at 893.0 $cm^{-1}$ corresponds to the bending vibration of β-configured terminal C-H bonds, and it is further confirmed by NMR measurements. Infrared analysis revealed that exopolysaccharide has polysaccharide characteristic absorption peak, and β-glycosidic bond connections.

(4) Periodic acid oxidation

The molar ratio among the exopolysaccharide, consumed sodium periodate and generated formic acid is 1:0.3819:0.1411. 1 mol of hexose equivalent polysaccharide sample consumed 0.3819 mol of periodate, produced 0.1411 mol of formic acid, therefore, the sample contains 14.11% (1→) or (1->6) glycosidic bonds. The sodium periodate consumption exceeds twice the amount of formic acid generated, therefore, there is the presence of hexose residue glycosidic bonds in the structure that undergo periodate oxidation without producing formic acid. It includes 9.97% of (1→2), (1->2,6), (1→4), or (1→4,6) glycosidic bonds. Following periodic acid oxidation, the exopolysaccharide sample generates substantial glucose and galactose, therefore, there is a presence of hexose residue glycosidic bonds resistant to periodic acid oxidation. It may include (1→3), (1->3, 6), (1→2, 3), (1→2, 4), (1→3, 4), or (1→2, 3, 4) glycosidic bonds.

(5) Smith degradation

GC analysis results of the polysaccharide after periodate oxidation and Smith degradation are shown in Figure 4. Retention time comparison indicates that after periodate oxidation and Smith degradation followed by GC analysis, the sample contained trace amount of glycerin, therefore, the exopolysaccharide contains a minor proportion of (1→2) or (1->6) glycosidic bonds. Since erythritol is barely detectable in the products, the sample contains almost no (1->4) or (1→4,6) glycosidic bonds. Combined with the periodate oxidation results, it was determined that the exopolysaccharide contains 14.11% (1→) or (1→6) glycosidic bonds, 9.97% (1->2) or (1→2,6) glycosidic bonds, and potentially also (1→3), (1→3,6), (1→2,3), (1->2,4), (1→3,4), or (1→2,3,4) glycosidic bonds.

(6) GC-MS analysis of methylated and acetylated products

The total ion current chromatogram of the sugar alcohol acetate derivatives after polysaccharide methylation is shown in Figure 5. Database matching and assignment analysis of the EI-MS spectra of the major products suggest the following possible glycosidic linkage patterns in the polysaccharide structure: ① Glcp (1→, ②→3) Galp (1→, ③→6) Galp (1→, ④→4) Galp (1→, ⑤→6) Glcp (1→, ⑥→3,6) Galp (1→, ⑦→4,6) Glcp (1→, ⑧→2,3,6) Galp (1→ and ⑨→2,3,4,6) Glcp (1→.

(7) NMR results

As shown in Figure 6, after D2O exchange, the proton signals of the polysaccharide were observed mainly in the δ3.0-4.7 ppm region. The δ 4.3-4.7 ppm region corresponds to the anomeric proton resonance of polysaccharide hexose. This indicates that the polysaccharide contains only the β-configuration, which is consistent with the infrared spectroscopy analysis.

Based on the 13C-NMR and DEPT135-NMR spectra of the polysaccharide sample (Figures 7-8), its carbon signals are observed primarily in the δ60-103.5 ppm region. Combined with an in-depth analysis of the 2D-NMR spectra (COSY, HSQC, and HMBC, Figures 9-11), the major carbon and proton signals are assigned. The possible carbon-hydrogen assignments for each residue include: ① β-Galp (1→ Residue, ② →3) β-Galp (1→ Residue, ③ →2, 3) β-Glcp (1→ Residue, ④ β-Glcp (1→ and ⑤ →2) β-Glcp (1→ Residue.

In summary, the exopolysaccharide is a high-molecular-weight, highly branched polysaccharide composed of β-glucose and β-galactose. The predicted repeating unit structure is as follows, and the proposed generic name is L-β-galactoglucan:

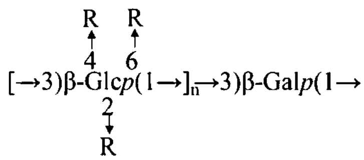

R= →2,3,4,6) β-Glcp (1→

**Efficacy Test 1 Hypoglycemic Activity of L-β-Galactoglucan**

Wild-type AB strain zebrafish at 3 dpf were randomly selected and placed in beakers, with 30 fish per beaker. L-β-galactoglucan (concentrations are listed in Table 1) was administered via aqueous exposure. A normal control group and a model control group were simultaneously established, with each beaker holding 25 mL of solution. Except for the normal control group, all other experimental groups were injected with streptozotocin (STZ) via the yolk sac. During the daytime, 0.15% egg yolk powder solution was administered via aqueous exposure, and at night, a 3% glucose solution was administered via aqueous exposure to establish a zebrafish pancreatic islet injury model. After a 2-day treatment at 28°C, zebrafish were rinsed 3 times with standard dilution water. Glucose levels were measured using a blood glucose meter. The protective efficacy of L-β-Galactoglucan against islet injury was evaluated based on the statistical analysis of this indicator. Statistical results are expressed as mean ± SE. Statistical analysis was performed using SPSS 26.0 software. A p-value of less than 0.05 ($p < 0.05$) was considered statistically significant.

Table 1 Blood Glucose Levels (n = 10)

| Group | Concentration (μg/mL) | Glucose Level (mmol/L, mean ± SE) |
|---|---|---|
| Normal Control Group | - | 1.15 ± 0.017*** |

(continued)

| Group | Concentration (μg/mL) | Glucose Level (mmol/L, mean ± SE) |
|---|---|---|
| Model Control Group | - | 3.89 ± 0.087 |
| Dimethylbiguanide | 400 | 1.39 ± 0.038*** |
| L-β-Galactoglucan | 62.5 | 3.32 ± 0.171 |
| | 125 | 2.72 ± 0.181*** |
| | 250 | 2.66 ± 0.167*** |
| Compared to the model control group, ***p < 0.001 | | |

As shown in Table 1 and Figure 12, L-β-galactoglucan exhibits hypoglycemic activity.

**Efficacy Test 2 Hypoglycemic Activity of L-β-Galactoglucan**

Transgenic zebrafish with green fluorescent pancreatic β-cells at 3 dpf were randomly selected and placed in beakers, with 30 fish per beaker. L-β-galactoglucan (concentrations are listed in Table 2) was administered via aqueous exposure. A normal control group and a model control group were simultaneously established, with each beaker holding 25 mL of solution. Except for the normal control group, all other experimental groups were injected with streptozotocin (STZ) via the yolk sac. During the daytime, 0.15% egg yolk powder solution was administered via aqueous exposure, and at night, a 3% glucose solution was administered via aqueous exposure to establish a zebrafish pancreatic islet injury model. After a 2-day treatment at 28°C, 10 zebrafish were randomly selected from each group and photographed under a fluorescence microscope, and images were saved. Data were analyzed and collected using NIS-Elements D 3.20 advanced image processing software. The fluorescence intensity of pancreatic β-cells in zebrafish was statistically analyzed to evaluate the protective efficacy of L-β-galactoglucan against islet injury. Statistical results are expressed as mean ± SE. Statistical analysis was performed using SPSS 26.0 software. A p-value of less than 0.05 ($p < 0.05$) was considered statistically significant.

Table 2 Fluorescence Intensity of Islet β-Cell (n = 10)

| Group | Concentration (μg/mL) | Fluorescence Intensity of Islet β-Cell (pixels, mean ± SE) |
|---|---|---|
| Normal Control Group | - | 31238 ± 534*** |
| Model Control Group | - | 13900 ± 1006 |
| Dimethylbiguanide | 400 | 20159 ± 1052*** |
| | 62.5 | 17733 ± 1028 |
| L-β-Galactoglucan | 125 | 17756 ± 975 |
| | 250 | 19192 ± 1690* |
| Compared to the model control group, *p < 0.05, **p < 0.001. | | |

As shown in Table 2 and Figures 13-15, L-β-galactoglucan demonstrates protective efficacy against islet injury, as specifically manifested by an increase in the fluorescence intensity of islet β-cells.

**Efficacy Test 3 Hypoglycemic Activity of L-β-Galactoglucan**

30 mice were randomly divided into three groups, with 10 mice in each group. After 24 hours of fasting, the mice were administered 1 g/kg starch, 1 g/kg L-β-galactoglucan, 1 g/kg starch + 1 g/kg L-β-galactoglucan, respectively. Blood samples were collected at 0 h (before administration), 0.5 h, 1.0 h, and 2.0 h after administration to measure blood glucose levels.

Table 3 Blood Glucose Levels (mmol/L)

| Group | 0 h | 0.5 h | 1.0 h | 2.0 h |
|---|---|---|---|---|
| Starch | 4.14±0.30 | 10.36±0.41 | 13.81±0.43 | 11.69±0.41 |
| L-β-Galactoglucan | 4.16±0.26 | 5.62±0.33 | 6.01±0.34 | 5.87±0.32 |
| Starch + L-β-Galactoglucan | 4.12±0.31 | 7.88±0.40 | 9.34±0.38 | 7.97±0.33 |

The results in Table 3 and Figure 16 indicate that L-β-galactoglucan effectively controls postprandial blood glucose increase.

**Efficacy Test 4 Evaluation of the Tissue Regeneration-Promoting Efficacy of L-β-Galactoglucan**

Wild-type AB strain zebrafish at 3 dpf were randomly selected and placed in 6-well plates, with 30 zebrafish per well. L-β-galactoglucan was administered via aqueous exposure (concentrations are listed in Table 4), with 7.00 μg/mL topical human epidermal growth factor for positive control. A normal control group and a model control group were simultaneously established, with 3 mL of solution per well. Except for the normal control group, for other experimental groups, a zebrafish tissue regeneration model was established by surgically amputating the tail fin. After a 3-day treatment at 28°C, 10 zebrafish were randomly selected from each experimental group and photographed under a anatomical microscope. The data were analyzed and collected using NIS-Elements D 3.20 advanced image processing software. The zebrafish tail fin length was measured, and the statistical analysis of the indicator were used to evaluate the tissue regeneration-promoting efficacy of L-β-galactoglucan. Statistical results are expressed as mean ± SE. Statistical analysis was performed using SPSS 26.0 software. A p-value of less than 0.05 ($p < 0.05$) was considered statistically significant.

Table 4 Evaluation of Tissue the Tissue Regeneration-Promoting Efficacy of L-(3-Galactoglucan (n = 10)

| Group | Concentration (μg/mL) | Tail Fin Length (pixels, mean ± SE) |
|---|---|---|
| Normal Control Group | - | 378 + 2.27*** |
| Model Control Group | - | 242 ± 6.96 |
| Topical Human Epidermal Growth Factor | 7.00 | 290 ± 6.10*** |
| L-β-Galactoglucan | 250 | 289 ± 7.38*** |
| | 500 | 291 ± 9.53*** |
| | 1000 | 301 ± 6.52*** |
| Compared to the model control group, *p < 0.05, **p < 0.001. | | |

As shown in Table 4 and Figures 17-19, L-β-galactoglucan exhibits tissue regeneration-promoting efficacy.

**Efficacy Test 5 Evaluation of the Wound-Healing-Promoting Efficacy of L-β-Galactoglucan**

Transgenic Flk1 zebrafish with green fluorescent vasculature at 2 dpf were randomly selected and placed in 6-well plates, with 30 fish per well. L-β-galactoglucan was administered via aqueous exposure (concentrations are listed in Table 5), with 7.00 μg/mL of topical human epidermal growth factor for positive control. A normal control group and a model control group were simultaneously established, with 3 mL of solution per well. Except for the normal control group, all experimental groups were injected with glacial acetic acid to establish a zebrafish tissue injury model. After a 2-day treatment at 28°C, the incidence of neovascularization at the wound sites of each experimental group was observed under a fluorescence microscope, and representative images were captured. The wound-healing-promoting efficacy of L-β-galactoglucan was evaluated based on the statistical analysis of the above indicators. Statistical results are expressed as mean ± SE. Statistical analysis was performed using SPSS 26.0 software. A p-value of less than 0.05 ($p < 0.05$) was considered statistically significant.

Table 5 Evaluation of the Wound Vascular Regeneration-Promoting Efficacy of L-β-Galactoglucan (n = 30)

| Group | Concentration (μg/mL) | Number of fishes exhibiting neovascularization (Tails) | Neovascularization Incidence (%) |
|---|---|---|---|
| Normal Control Group | - | 0*** | 0 |
| Model Control Group | - | 15 | 50 |
| Topical Human Epidermal Growth Factor | 7.00 | 24* | 80 |
| L-β-Galactoglucan | 250 | 14 | 47 |
| | 500 | 22 | 73 |
| | 1000 | 24* | 80 |
| Compared to the model control group, *p < 0.05, **p < 0.001. | | | |

As shown in Table 5 and Figures 20-21, L-β-galactoglucan promotes vascular regeneration at wound sites.

**Efficacy Test 6 Evaluation of the Efficacy of L-β-Galactoglucan in Clearing Apoptotic Cells at the Injury Site**

Wild-type AB strain zebrafish at 2 dpf were randomly selected and placed in 6-well plates, with 30 zebrafish per well. L-β-galactoglucan was administered via aqueous exposure (concentrations are listed in Table 6), with 7.00 μg/mL of topical human epidermal growth factor for positive control. A normal control group and a model control group were simultaneously established, with 3 mL of solution per well. Except for the normal control group, all experimental groups were injected with glacial acetic acid to establish a zebrafish tissue injury model. After a 5-hour treatment at 28°C, zebrafish from each experimental group were stained using a one-step TUNEL apoptosis detection kit. Following staining, 10 zebrafish were randomly selected from each group and photographed under a fluorescence microscope. Data were analyzed and collected using NIS-Elements D 3.20 advanced image processing software to assess the fluorescence intensity of apoptotic cells at the tissue injury site. The efficacy of L-β-galactoglucan in clearing apoptotic cells at the injury site was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 6 Evaluation of the Efficacy of L-β-Galactoglucan in Clearing Apoptotic Cells at the Injury Site (n = 10)

| Group | Concentration (μg/mL) | Fluorescence Intensity of Apoptotic Cells at Tissue Injury Sites (pixels, mean ± SE) |
|---|---|---|
| Normal Control Group | - | 140458 ± 3139*** |
| Model Control Group | - | 268573 ± 15464 |
| Topical Human Epidermal Growth Factor | 7.00 | 212892 ± 6498** |
| L-β-Galactoglucan | 250 | 231718 ± 5643 |
| | 500 | 231674 + 5260 |
| | 1000 | 217289 ± 6265* |
| Compared to the model control group, *p < 0.05, **p < 0.01, ***p < 0.001. | | |

As shown in Table 6 and Figures 22-24, L-β-galactoglucan promotes the clearance of apoptotic cells at tissue injury sites.

**Efficacy Test 7 Evaluation of the Efficacy of L-β-Galactoglucan in Increasing Macrophage Numbers**

Transgenic zebrafish with green fluorescent macrophages at 3 dpf were randomly selected and placed in 6-well plates, with 30 fish per well. L-β-galactoglucan was administered via aqueous exposure (concentrations are listed in Table 7), with 15.0 μg/mL of Bailing Capsules for positive control. A normal control group and a model control group were simultaneously established, with 3 mL of solution per well. Except for the normal control group, all experimental groups were intravenously injected with vincristine tartrate injection to establish a zebrafish model of macrophage depletion. After a 2-day treatment at 28°C, 10 zebrafish were randomly selected from each experimental group and photographed under a fluorescence microscope. Data were analyzed and collected using NIS-Elements D 3.20 advanced image processing software to assess zebrafish macrophage fluorescence intensity. The efficacy of L-β-galactoglucan in increasing macrophage numbers was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 7 Evaluation of the Efficacy of L-β-Galactoglucan in Increasing Macrophage Numbers (n = 10)

| Group | Concentration (μg/mL) | Macrophage Fluorescence Intensity (pixels, mean ± SE) |
|---|---|---|
| Normal Control Group | - | 34377 ± 1090*** |
| Model Control Group | - | 26570 ± 1026 |
| Bailing Capsules | 15.0 | 33820 ± 1507*** |

(continued)

| Group | Concentration (μg/mL) | Macrophage Fluorescence Intensity (pixels, mean ± SE) |
|---|---|---|
| | 250 | 30000 ± 1336 |
| L-β-Galactoglucan | 500 | 31156 ± 1219* |
| | 1000 | 33238 ± 1329** |
| Compared to the model control group, *p < 0.05, **p < 0.01, ***p < 0.001. | | |

As shown in Table 7 and Figures 25-26, L-β-galactoglucan increases macrophage numbers.

**Efficacy Test 8 Evaluation of the Efficacy of L-β-Galactoglucan in Enhancing Macrophage Phagocytosis**

Wild-type AB strain zebrafish at 3 dpf were randomly selected and placed in 6-well plates, with 30 zebrafish per well. L-β-galactoglucan was administered via aqueous exposure (concentrations are listed in Table 8), with 15.0 μg/mL of Bailing Capsules for positive control. A normal control group and a model control group were simultaneously established, with 3 mL of solution per well. All experimental groups were intravenously injected with vincristine tartrate injection and fluorescent microspheres to establish a model of impaired macrophage phagocytosis. The normal control group received only fluorescent microspheres. After a 4-day treatment at 28°C, 10 zebrafish were randomly selected from each group and photographed under a fluorescence microscope. The number of fluorescent microspheres retained by zebrafish was collected and analyzed using ImageJ software. The efficacy of L-β-galactoglucan in enhancing macrophage phagocytosis was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 8 Evaluation of the Efficacy of L-β-Galactoglucan in Enhancing Macrophage Phagocytosis (n = 10)

| Group | Concentration (μg/mL) | Number of residual fluorescent microspheres (pcs, mean ± SE) |
|---|---|---|
| Normal Control Group | - | 18.6 ± 1.49*** |
| Model Control Group | - | 55.7 ± 1.35 |
| Bailing Capsules | 15.0 | 43.1 ± 3.71** |
| | 250 | 46.2 ± 4.15 |
| L-β-Galactoglucan | 500 | 43.0 ± 2.27* |
| | 1000 | 42.2 ± 4.04* |
| Compared to the model control group, *p < 0.05, **p < 0.01, ***p < 0.001. | | |

As shown in Table 8 and Figures 27-29, L-β-galactoglucan enhances macrophage phagocytosis.

**Efficacy Test 9 Amelioration of T-Cell Depletion by L-β-Galactoglucan**

Transgenic zebrafish with red fluorescent T-cells at 3 dpf were randomly selected and placed in 6-well plates, with 30 zebrafish per well. L-β-galactoglucan was administered via aqueous exposure (concentrations are listed in Table 9), with 15.0 μg/mL of Bailing Capsules for positive control. A normal control group and a model control group were simultaneously established, with 3 mL of solution per well. Except for the normal control group, all experimental groups were intravenously injected with vincristine tartrate injection to establish a model of T-cell depletion. After a 2-day treatment at 28°C, 10 zebrafish were randomly selected from each group and photographed under a fluorescence microscope. Data were analyzed and collected using NIS-Elements D 3.20 advanced image processing software to assess T-cell fluorescence intensity. The efficacy of L-β-galactoglucan in ameliorating T-cell depletion was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 9 Evaluation of Efficacy of L-β-Galactoglucan in Ameliorating T-Cell depletion (n = 10)

| Group | Concentration (μg/mL) | T-Cell Fluorescence Intensity (pixels, mean ± SE) |
|---|---|---|
| Normal Control Group | - | 22537 ± 1136*** |
| Model Control Group | - | 7446 ± 473 |

(continued)

| Group | Concentration (μg/mL) | T-Cell Fluorescence Intensity (pixels, mean ± SE) |
|---|---|---|
| Bailing Capsules | 15.0 | 11340 ± 839*** |
| | 250 | 7601 ± 1174 |
| L-β-Galactoglucan | 500 | 12144 ± 1045** |
| | 1000 | 12436 ± 876** |
| Compared to the model control group, **p < 0.01, ***p < 0.001. | | |

As shown in Table 9 and Figures 30-32, L-β-galactoglucan ameliorates T-cell depletion.

**Efficacy Test 10 Amelioration of Neutropenia by L-β-Galactoglucan**

Transgenic zebrafish with green fluorescent neutrophils (MPX) at 3 dpf were randomly selected and placed in 6-well plates, with 30 fish per well. L-β-galactoglucan (concentrations are listed in Table 10) was administered via aqueous exposure, with Bailing Capsules (15.0 μg/mL) for positive control. A normal control group and a model control group were simultaneously established, with 3 mL of solution per well. Except for the normal control group, all experimental groups were intravenously injected with vincristine tartrate injection to establish a zebrafish model of neutropenia. After a 2-day treatment at 28°C, 10 zebrafish were randomly selected from each experimental group and photographed under a fluorescence microscope. Data were analyzed and collected using NIS-Elements D 3.20 advanced image processing software to assess tail-end neutrophil counts. The efficacy of L-β-galactoglucan in ameliorating neutropenia was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 10 Evaluation of the Efficacy of L-β-Galactoglucan in Ameliorating Neutropenia (n = 10)

| Group | Concentration (μg/mL) | Neutrophil Count in Caudal Vessels (units, mean ± SE) |
|---|---|---|
| Normal Control Group | - | 58.8 ± 3.08*** |
| Model Control Group | - | 39.0 ± 1.78 |
| Bailing Capsules | 15.0 | 52.9 ± 2.02*** |
| | 250 | 44.2 ± 1.47 |
| L-β-Galactoglucan | 500 | 51.3 ± 1.80*** |
| | 1000 | 50.9 ± 1.36*** |
| Compared to the model control group, ***p < 0.001 | | |

As shown in Table 10 and Figures 33-34, L-β-galactoglucan ameliorates neutropenia.

**Efficacy Test 11 Evaluation of the Adjuvant Protective Efficacy of L-β-Galactoglucan Against Alcoholic Liver Injury (AST Activity)**

Wild-type AB strain zebrafish at 3 dpf were randomly selected and placed in 6-well plates, with 30 zebrafish per well. L-β-galactoglucan was administered via aqueous exposure (concentrations are listed in Table 11), with 298 μg/mL metadoxine for positive control. A normal control group and a model control group were simultaneously established, with 3 mL of solution per well. Except for the normal control group, the other experimental groups were administered anhydrous ethanol via aqueous exposure to establish a model of alcoholic liver injury. Triplicate experiments were conducted in parallel. After a 2-day treatment at 28°C, zebrafish samples were collected according to the instructions of the AST assay kit. AST levels were measured using a multifunctional microplate reader to analyze AST activity in zebrafish from each experimental group. The adjuvant protective efficacy of L-β-galactoglucan against alcoholic liver injury was evaluated based on statistical analysis of the indicator. Statistical results are expressed as mean ± SE. Statistical analysis was performed using SPSS 26.0 software. A p-value of less than 0.05 ($p < 0.05$) was considered statistically significant.

Table 11 Evaluation of the Adjuvant Protective Efficacy of L-β-Galactoglucan Against Alcoholic Liver Injury(AST Activity) (n = 3)

| Group | Concentration (μg/mL) | Tissue AST Activity (U/gprot, mean ± SE) |
|---|---|---|
| Normal Control Group | - | 40.6 ± 0.367*** |
| Model Control Group | - | 49.3 ±0.621 |
| Metadoxine | 298 | 41.5 ±0.760** |
| L-β-Galactoglucan | 62.5 | 45.1 ±0.712* |
| | 125 | 45.8 ±1.19* |
| | 250 | 41.4 ±0.364*** |
| Compared to the model control group, *p < 0.05, **p < 0.01, ***p < 0.001. | | |

As shown in Table 11 and Figure 35, L-β-galactoglucan reduces AST activity in tissue.

**Efficacy Test 12 Evaluation of the Adjuvant Protective Efficacy of L-β-Galactoglucan Against Alcoholic Liver Injury (ALT Activity)**

Wild-type AB strain zebrafish at 3 dpf were randomly selected and placed in 6-well plates, with 30 zebrafish per well. L-β-galactoglucan was administered via aqueous exposure (concentrations are listed in Table 12), with 298 μg/mL metadoxine for positive control. A normal control group and a model control group were simultaneously established, with 3 mL of solution per well. Except for the normal control group, the other experimental groups were administered anhydrous ethanol via aqueous exposure to establish a model of alcoholic liver injury. Triplicate experiments were conducted in parallel. After a 2-day treatment at 28°C, zebrafish samples were collected according to the instructions of the ALT assay kit. ALT levels were measured using a multifunctional microplate reader to analyze ALT activity in zebrafish from each experimental group. The adjuvant protective efficacy of L-β-galactoglucan against alcoholic liver injury was evaluated based on statistical analysis of the indicator. Statistical results are expressed as mean ± SE. Statistical analysis was performed using SPSS 26.0 software. A p-value of less than 0.05 ($p < 0.05$) was considered statistically significant.

Table 12 Evaluation of the Adjuvant Protective Efficacy of L-β-Galactoglucan Against Alcoholic Liver Injury (ALT Activity) (n = 3)

| Group | Concentration (μg/mL) | Tissue ALT Activity (U/gprot, mean ± SE) |
|---|---|---|
| Normal Control Group | - | 10.8 ± 0.484*** |
| Model Control Group | - | 20.3 ± 0.277 |
| Metadoxine | 298 | 18.5 ± 0.294** |
| L-β-Galactoglucan | 62.5 | 19.5 ± 0.574 |
| | 125 | 18.9 ± 0.821 |
| | 250 | 18.1 ± 0.229* |
| Compared to the model control group, *p < 0.05, **p < 0.01, ***p < 0.001. | | |

As shown in Table 12 and Figure 36, L-β-galactoglucan reduces ALT activity in tissue.

**Efficacy Test 13 Effect on Liver Histological Structure**

Wild-type AB strain zebrafish at 3 dpf were randomly selected and placed in 6-well plates, with 30 zebrafish per well. L-β-galactoglucan was administered via aqueous exposure (concentrations are listed in Table 12), with 298 μg/mL metadoxine for positive control. A normal control group and a model control group were simultaneously established, with 3 mL of solution per well. Except for the normal control group, the other experimental groups were administered anhydrous ethanol via aqueous exposure to establish a model of alcoholic liver injury. After a 2-day treatment at 28 °C, zebrafish from each experimental group were fixed in 4% formaldehyde solution and processed through a series of steps including dehydration, embedding, sectioning, staining, and mounting. Histopathological analysis was then performed using hematoxylin and eosin (H&E) staining. Through liver histopathological analysis, the adjuvant protective efficacy of L-β-galactoglucan against alcoholic liver injury was evaluated.

As shown in Figure 37, zebrafish liver histopathological examination for animals in the normal control group revealed well-defined hepatocyte nuclei without visible enlargement or vacuolation, with normal cellular architecture and

clear borders. In contrast, the model control group exhibited blurred hepatocyte structures and fatty vacuolar degeneration (black arrows) in the liver tissue, indicating successful model establishment. The positive control (metadoxine) group showed clearer hepatocyte structures and reduced fatty vacuolar degeneration compared to the model control group, demonstrating the ameliorative effect of metadoxine on alcohol-induced liver injury in zebrafish. In the 62.5 and 125 μg/mL L-β-galactoglucan groups, zebrafish liver histopathological examination revealed blurred hepatocyte structures and fatty vacuolar degeneration in liver tissue. However, the 250 μg/mL L-β-galactoglucan group demonstrated clearer hepatocyte structures and reduced fatty vacuolar degeneration compared to the model control group. This indicates that L-β-galactoglucan has a protective effect against alcohol-induced liver injury in zebrafish.

**Efficacy Test 14 Evaluation of ROS Scavenging Activity**

Wild-type AB strain zebrafish at 4 dpf were randomly selected and placed in 6-well plates, with 30 zebrafish per well. L-β-galactoglucan was administered via aqueous exposure (concentrations are listed in Table 13), with 1000 μg/mL of Zhonghua Dieda Wan for positive control. A normal control group and a model control group were simultaneously established, with a volume of 3 mL per well. After a 1-day treatment at 28°C, CM-H2DCFDA was added for each experimental group. Except for the normal control group, the other experimental groups were administered sodium sulfite anhydrous via aqueous exposure to establish a zebrafish fatigue model. Following administration of anhydrous sodium sulfite, zebrafish were immediately transferred to a 96-well enzyme label plate. After co-exposure with anhydrous sodium sulfite for a specified period, ROS fluorescence values were measured for each experimental group using a multifunctional microplate reader. The ROS scavenging activity of L-β-galactoglucan was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 13 Evaluation of the ROS Scavenging Activity of L-β-Galactoglucan (n = 10)

| Group | Concentration (μg/mL) | ROS fluorescence value (mean ± SE) |
|---|---|---|
| Normal Control Group | - | 25.0 ± 0.632*** |
| Model Control Group | - | 15490 + 428 |
| Zhonghua Di Tai Wan | 1000 | 7772 ± 143*** |
| L-β-Galactoglucan | 250 | 13778 ± 211 |
| | 500 | 12336 ± 224** |
| | 1000 | 10618 ± 76.8*** |
| Compared to the model control group, **$p < 0.01$, ***$p < 0.001$. | | |

As shown in Table 13 and Figure 38, L-β-galactoglucan exhibits ROS scavenging activity.

**Efficacy Test 15 Evaluation of the ATP Synthesis-Promoting Efficacy of L-β-Galactoglucan**

Wild-type AB strain zebrafish at 4 dpf were randomly selected and placed in 6-well plates, with 30 zebrafish per well. L-β-galactoglucan was administered via aqueous exposure (concentrations are listed in Table 14), with 1000 μg/mL of Zhonghua Dieda Wan for positive control. A normal control group and a model control group were simultaneously established, with a volume of 3 mL per well. After a 1-day treatment at 28°C, the other experimental groups except for the normal control were administered anhydrous sodium sulfite via aqueous exposure to establish a zebrafish fatigue model. After co-exposure with anhydrous sodium sulfite for a specified period, 10 zebrafish were randomly selected from each experimental group. ATP chemiluminescence value in zebrafish body was measured using an ATP content assay kit. The ATP synthesis-promoting efficacy of L-β-galactoglucan was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 14 Evaluation of the ATP Synthesis-Promoting Efficacy of L-β-Galactoglucan (n = 10)

| Group | Concentration (μg/mL) | ATP chemiluminescence value (mean ± SE) |
|---|---|---|
| Normal Control Group | - | 180908 ± 10280*** |
| Model Control Group | - | 115634 + 9005 |
| Zhonghua Di Tai Wan | 1000 | 147335 ± 9624* |

(continued)

| Group | Concentration (μg/mL) | ATP chemiluminescence value (mean ± SE) |
|---|---|---|
| L-β-Galactoglucan | 250 | 121366 ± 5116 |
| | 500 | 177624 ± 16234* |
| | 1000 | 191221 ± 15945** |
| Compared to the model control group, *p < 0.05, **p < 0.01, ***p < 0.001. | | |

As shown in Table 14 and Figure 39, L-β-galactoglucan promotes ATP synthesis.

**Efficacy Test 16 Evaluation of the Lactic Acid Metabolism-Promoting Efficacy of L-β-Galactoglucan**

Wild-type AB strain zebrafish at 4 dpf were randomly selected and placed in 6-well plates, with 30 zebrafish per well. L-β-galactoglucan was administered via aqueous exposure (concentrations are listed in Table 15), with 1000 μg/mL of Zhonghua Dieda Wan for positive control. A normal control group and a model control group were simultaneously established, with a volume of 3 mL per well. Three parallel experiments were conducted. After a 1-day treatment at 28°C, the other experimental groups except for the normal control were administered anhydrous sodium sulfite via aqueous exposure to establish a zebrafish fatigue model. After co-treatment with sodium metabisulfite for a specified period, zebrafish samples were collected per the lactate assay kit instructions. Data were collected using a multifunctional microplate reader to analyze lactate levels in zebrafish across experimental groups. The lactic acid metabolism-promoting efficacy of L-β-galactoglucan was evaluated based on statistical analysis of the indicator. Statistical results are expressed as mean ± SE. Statistical analysis was performed using SPSS 26.0 software. A p-value of less than 0.05 ($p < 0.05$) was considered statistically significant.

Table 15 Evaluation of the Lactic Acid Metabolism-Promoting Efficacy of L-β-Galactoglucan (n = 3)

| Group | Concentration (μg/mL) | Lactic Acid Content (mmol/gprot, mean ± SE) |
|---|---|---|
| Normal Control Group | - | 0.229 ± 0.001*** |
| Model Control Group | - | 0.299 ± 0.002 |
| Zhonghua Di Tai Wan | 1000 | 0.258 ± 0.002*** |
| L-β-Galactoglucan | 250 | 0.269 ± 0.001*** |
| | 500 | 0.269 ± 0.002*** |
| | 1000 | 0.254 ± 0.002*** |
| Compared to the model control group, ***p < 0.001 | | |

As shown in Table 15 and Figure 40, L-β-galactoglucan promotes lactic acid metabolism.

**Efficacy Test 17 Regulation of Gut Microbiota**

A zebrafish gut microbiota (Lactobacillus brevis) model was established by feeding wild-type AB strain zebrafish at 5 dpf with Lactobacillus brevis labeled with CM-Dil red fluorescent dye at a concentration of 1 × 108 CFU/mL. After the culture at 28°C until 6 dpf, the Lactobacillus brevis was removed and the zebrafish were randomly assigned to 6-well plates, with 30 fish per well. L-β-galactoglucan was administered via aqueous exposure (concentrations are listed in Table 16), with 2000 μg/mL of Bacillus subtilis duplex living bacterium for positive control. A model control group was simultaneously established, with 3 mL of solution per well. After a 6-hour treatment at 28°C, 10 zebrafish were randomly selected from each experimental group and photographed under a fluorescence microscope. Data were collected using NIS-Elements D 3.20 advanced image processing software to analyze intestinal Lactobacillus brevis fluorescence intensity. The efficacy of L-β-galactoglucan in modulating gut microbiota (Lactobacillus brevis) was evaluated based on statistical analysis of the indicator. Statistical results are expressed as mean ± SE. Statistical analysis was performed using SPSS 26.0 software. A p-value of less than 0.05 ($p < 0.05$) was considered statistically significant.

Table 16 Evaluation of the Gut Microbiota-Regulating Efficacy of L-β-Galactoglucan (n = 10)

| Group | Concentration(μg/mL) | Lactobacillus brevis fluorescence intensity (pixels, mean ± SE) |
|---|---|---|
| Model Control Group | - | 395929 ± 36791 |
| Bacillus subtilis duplex living bacterium | 2000 | 809397 ± 90617** |
| | 250 | 611879 ± 57981 |
| L-β-Galactoglucan | 500 | 688166 ± 120744 |
| | 1000 | 1039653 ± 78889*** |
| Compared to the model control group, **p < 0.01, ***p < 0.001. | | |

As shown in Table 16 and Figures 41-43, L-β-galactoglucan increases the number of intestinal Lactobacillus brevis.

**Efficacy Test 18 Evaluation of the Hydrating and Moisturizing Efficacy of L-β-Galactoglucan**

Randomly selected 4 dpf zebrafish from the melanin allele mutation translucent Albino strain were placed in 6-well plates, with 30 fish per well. L-β-galactoglucan (concentrations are listed in Table 17) was administered via aqueous exposure, with urea at 600 μg/mL for positive control. A normal control group and a model control group were simultaneously established, with 3 mL of solution per well. After an 18-hour treatment at 28°C, elution was performed. Except for the normal control group, the other experimental groups were administered sodium chloride via aqueous exposure to establish a zebrafish dehydration model. After a 6-hour treatment at 28°C, 10 zebrafish were randomly selected from each experimental group and imaged under a dissecting microscope. Photographs were taken and stored. Data were analyzed and collected using NIS-Elements D 3.20 advanced image processing software. The tail area of zebrafish was measured. The hydrating and moisturizing efficacy of L-β-galactoglucan was evaluated based on the statistical analysis of the indicator. Statistical results are expressed as mean ± SE. Statistical analysis was performed using SPSS 26.0 software. A p-value of less than 0.05 (p < 0.05) was considered statistically significant.

Table 17 Evaluation of the Hydrating and Moisturizing Efficacy of L-β-Galactoglucan (n = 10)

| Group | Concentration (μg/mL) | Tail Area (pixels, mean ± SE) |
|---|---|---|
| Normal Control Group | - | 576194 ± 5588*** |
| Model Control Group | - | 519445 ± 6282 |
| Urea | 600 | 554839 ± 4158*** |
| | 250 | 547971 ± 3330* |
| L-β-Galactoglucan | 500 | 564052 ± 6038*** |
| | 1000 | 569622 ± 5586*** |
| Compared to the model control group, *p < 0.05, **p < 0.001. | | |

As shown in Table 17 and Figures 44-46, L-β-galactoglucan exhibits hydrating and moisturizing efficacy.

**Efficacy Test 19 Evaluation of the Inhibitory Effect of L-β-Galactoglucan on β-Galactosidase Activity**

Wild-type AB strain zebrafish at 6hpf were randomly selected and placed in 6-well plates, with 30 fish per well. L-β-galactoglucan was administered via aqueous exposure (concentrations are listed in Table 18), with 2000 μg/mL catalase for positive control. A normal control group and a model control group were simultaneously established, with 3 mL of solution per well. Except for the normal control group, the other groups were administered hydrogen peroxide via aqueous exposure to establish a zebrafish aging model, with daily medium changes. After a 6-day treatment at 28°C, zebrafish from each experimental group were stained using a cellular senescence β-galactosidase staining kit. Following staining, 10 zebrafish were randomly selected from each group and photographed under a dissecting microscope. Data were collected using NIS-Elements D 3.20 advanced image processing software to analyze β-galactosidase staining intensity. The inhibitory effect of L-β-galactoglucan on β-galactosidase activity was evaluated with statistical analysis of the indicator. Statistical results are presented as mean ± SE. Statistical analysis was performed using SPSS 26.0 software. A p-value of less than 0.05 (p < 0.05) was considered statistically significant.

Table 18 Evaluation of the Inhibitory Effect of L-β-Galactoglucan on β-Galactosidase Activity (n = 10)

| Group | Concentration (μg/mL) | β-Galactoglucan Staining Intensity (pixels, mean ± SE) |
|---|---|---|
| Normal Control Group | - | 53854 ± 706*** |
| Model Control Group | - | 59073 ± 629 |
| Catalase | 2000 | 53500 ± 729*** |
| | 125 | 57506 ± 1159 |
| L-β-Galactoglucan | 250 | 55882 ± 1169 |
| | 500 | 53114 ± 1408** |
| Compared to the model control group, **p < 0.01, ***p < 0.001. | | |

As shown in Table 18 and Figures 47-49, L-β-galactoglucan demonstrates inhibitory effects on β-galactosidase activity.

**Efficacy Test 20 L-β-Galactoglucan Increases Telomerase Activity**

Wild-type AB strain zebrafish at 6 hpf were randomly selected and placed in 6-well plates, with 30 fish per well. L-β-galactoglucan was administered via aqueous exposure (concentrations are listed in Table 19), with 2000 μg/mL catalase for positive control. A normal control group and a model control group were simultaneously established, with 3 mL of solution per well. Three parallel experiments were conducted. Except for the normal control group, the other groups were administered hydrogen peroxide via aqueous exposure to establish a zebrafish aging model, with daily medium changes. After a 6-day treatment at 28°C, zebrafish samples were collected according to the instructions for use of Zebrafish TE Elisa Kit. Data were collected using a multifunctional microplate reader to analyze telomerase activity in zebrafish from each experimental group. The efficacy of L-β-galactoglucan in increasing telomerase activity was evaluated based on the statistical analysis of the indicator. Statistical results are expressed as mean ± SE. Statistical analysis was performed using SPSS 26.0 software. A p-value of less than 0.05 ($p < 0.05$) was considered statistically significant.

Table 19 L-β-Galactoglucan Increases Telomerase Activity (n = 3)

| Group | Concentration (μg/mL) | Telomerase Activity (IU/gprot, mean ± SE) |
|---|---|---|
| Normal Control Group | - | 3.06 ± 0.091** |
| Model Control Group | - | 2.24 ± 0.098 |
| Catalase | 2000 | 3.06 ± 0.222* |
| | 125 | 2.74 ± 0.145 |
| L-β-Galactoglucan | 250 | 2.83 ± 0.084 |
| | 500 | 3.25 ± 0.228** |
| Compared to the model control group, *p < 0.05, **p < 0.01 | | |

As shown in Table 19 and Figure 50, L-β-galactoglucan increases telomerase activity.

**Efficacy Test 21 L-β-Galactoglucan Enhances Skin Elasticity**

Wild-type AB strain zebrafish at 4 dpf were randomly selected and placed in 6-well plates, with 30 zebrafish per well. L-β-galactoglucan (concentrations are listed in Table 20) was administered via aqueous exposure, with verisol collagen (250 μg/mL) for positive control. A normal control group was included, with each well containing 3 mL. Triplicate experiments were conducted in parallel. After a 1-day treatment at 28°C, total RNA for zebrafish samples in each group was extracted using the Universal RNA Extraction TL Kit C. Total RNA concentration and purity were measured using a UV-visible spectrophotometer. Total RNA of 2.00 μg of zebrafish was taken. 20.0 μL of cDNA was synthesized following the instructions for use of cDNA first-strand synthesis kit. Gene expression of β-actin, collala, col1a1b, eln1, and eln2 was tested via q-PCR. The relative RNA expression levels of collala, collalb, eln1, and eln2 genes were calculated using β-actin as an internal control for gene expression. Statistical results are expressed as mean ± SE. Statistical analysis was performed using SPSS 26.0 software. A p-value of less than 0.05 ($p < 0.05$) was considered statistically significant.

At the experimental endpoint, total RNA of zebrafish was extracted and analyzed using a UV-visible spectrophotometer to determine RNA concentration and the A260/A280 ratio (Table 23). The A260/A280 ratio was in the range of

1.8-2.2, therefore, the quality of total RNA for zebrafish is high, and is suitable for subsequent q-PCR experiments. Primer sequences are listed in Table 21.

Table 20 Total RNA Concentration and A260/A280 Ratio (n = 3)

| Group | Concentration (μg/mL) | RNA concentration (μg/μL) | | | A260/A280 | | |
|---|---|---|---|---|---|---|---|
| | | Sample I | Sample II | Sample III | Sample I | Sample II | Sample III |
| Normal Control Group | - | 0.664 | 0.739 | 0.745 | 1.92 | 1.98 | 1.97 |
| Verisol Collagen | 250 | 0.709 | 0.829 | 0.795 | 1.94 | 1.99 | 1.98 |
| | 250 | 0.705 | 0.723 | 0.730 | 1.95 | 2.00 | 2.03 |
| L-β-Galactoglucan | 500 | 0.693 | 0.760 | 0.764 | 1.95 | 1.96 | 1.94 |
| | 1000 | 0.738 | 0.737 | 0.751 | 1.97 | 1.93 | 1.96 |

Table 21 Primer Sequence Information

| Gene | Primer Sequence | |
|---|---|---|
| β-actin | Forward | 5'-TCGAGCAGGAGATGGGAACC-3' |
| | Reverse | 5'-CTCGTGGATACCGCAAGATTC-3' |
| collala | Forward | 5'-CCAGACGGCACCAAGAAGAA-3' |
| | Reverse | 5'-TGCCATACTCGAACTGGAAGC-3' |
| col1a1b | Forward | 5'-TTTCTGCTAGGGTCTGGATTGG-3' |
| | Reverse | 5'-AGCTGCAGTAACTTCGTCGG-3' |

Table 22 Evaluation of the Skin Elasticity-Enhancing Efficacy of L-β-Galactoglucan (col1a1a and col1a1b) (n = 3)

| Group | Concentration (μg/mL) | Relative Expression Levels of collala Gene (mean ± SE) | Relative Expression Levels of col1a1b Gene (mean ± SE) |
|---|---|---|---|
| Normal Control Group | - | 1.00 ± 0.106 | 1.00 ± 0.044 |
| Verisol Collagen | 250 | 1.92 ± 0.091** | 2.11 ± 0.194** |
| | 250 | 2.11 ± 0.004*** | 1.91 ± 0.115* |
| L-β-Galactoglucan | 500 | 1.95 ± 0.060*** | 2.34 ± 0.102** |
| | 1000 | 2.23 ± 0.099*** | 3.24 ± 0.313*** |
| Compared to the normal control group, *p < 0.05, **p < 0.01, ***p < 0.001. | | | |

As shown in Table 22 and Figures 51-52, L-β-galactoglucan demonstrated efficacy in enhancing skin elasticity, specifically manifested by upregulating the relative expression levels of collala and collalb genes.

**Efficacy Test 22 L-β-Galactoglucan Inhibits Melanin Recruitment**

Transgenic Flk1 zebrafish with green fluorescent vasculature at 2 dpf were randomly selected and placed in 6-well plates, with 30 fish per well. L-β-galactoglucan was administered via aqueous exposure (concentrations are listed in Table 23), with 7.00 μg/mL of topical human epidermal growth factor for positive control. A normal control group and a model control group were established simultaneously, with 3 mL of solution per well. Except for the normal control group, all experimental groups were injected with glacial acetic acid to establish a zebrafish tissue injury model. After a 2-day treatment at 28°C, 10 zebrafish were randomly selected from each experimental group and photographed under a fluorescence microscope. Data were analyzed and collected using NIS-Elements D 3.20 advanced image processing software to assess melanin signal intensity at the tissue injury site. The efficacy of L-β-galactoglucan in inhibiting melanin recruitment was evaluated based on the statistical analysis of the above indicators. Statistical results are expressed as mean ± SE. Statistical analysis was performed using SPSS 26.0 software. A p-value of less than 0.05 ($p < 0.05$) was considered statistically significant.

Table 23 Evaluation of the Inhibitory Effect of L-β-Galactoglucan on Melanin Recruitment (n = 10)

| Group | Concentration (μg/mL) | Melanin signal intensity at tissue injury sites (pixels, mean ± SE) |
|---|---|---|
| Normal Control Group | - | 11665 ± 153*** |
| Model Control Group | - | 18976 ± 709 |
| Topical Human Epidermal Growth Factor | 7.00 | 16483 ± 524* |
| L-β-Galactoglucan | 250 | 16959 + 962 |
| | 500 | 16570 + 895 |
| | 1000 | 15893 ± 714* |

Compared to the model control group, *p < 0.05, **p < 0.001.

As shown in Table 23 and Figures 53-55, L-β-galactoglucan inhibits melanin recruitment.

**Efficacy Test 23 Application Study of L-β-Galactoglucan in Weaned Piglets**

**1 Experimental Objective**

To investigate the impact of L-β-galactoglucan on the growth performance and intestinal health indicators of weaned piglets, and to effectively evaluate its application in weaned piglets.

**2 Test Samples and Source**

The test sample was a crude L-β-galactoglucan (water-soluble, total sugar purity 62.14%), which was prepared according to the method described in Example 1 and supplied by Chengdu Saidike Biotechnology Co., Ltd.

**3 Test Animals and Design**

In the study, 120 healthy weaned three-way crossbred piglets (7.46 ± 1.52 kg) were randomly divided into 3 groups, with 8 replicates per group (5 pigs per replicate). The test was conducted at Changsha Kelikang Agriculture & Animal Husbandry Technology Co., Ltd. After a 3-day pre-feeding period, the formal test was commenced with a 42-day experimental period. All test pigs had free access to feed and water. The pigsty was cleaned and disinfected regularly according to the farm's routine regulation, and the pigsty was clean and dry, with appropriate temperatures. All other husbandry procedures were carried out according to the pig farm requirements.
The grouping and experimental formulations are as follows:

Table 24 Study Grouping

| Group | Diet |
|---|---|
| A | Commercial Feed (control group) |
| B | Commercial Feed + 400 g/t L-β-Galactoglucan |
| C | Commercial Feed + 600 g/t L-β-Galactoglucan |

Table 25 Feed Formulation

| | Teaching Feed Additive Dosage (kg) | | Nursery Feed |
|---|---|---|---|
| Corn | 25 | Corn | 57.82 |
| Soybean Meal | 8.55 | Soybean Meal | 18 |
| Puffed Corn | 30.5 | Soybean Protein Concentrate | 7.4 |
| Soybean Protein Concentrate | 8 | Whey Powder | 4 |
| Fish Meal | 6 | Soybean Oil | 6 |
| Whey Powder | 8 | Whey Powder | |
| Glucose | 5 | Glucose | |
| Soybean Oil | 2 | Soybean Oil | |

(continued)

| | Teaching Feed Additive Dosage (kg) | | Nursery Feed |
|---|---|---|---|
| Calcium Lactate | 0.6 | Calcium Lactate | 0.6 |
| White Sugar | 2 | White Sugar | |
| Limestone Powder | 0.2 | Limestone Powder | 0.25 |
| Dicalcium Phosphate | 0.5 | Dicalcium Phosphate | 1.1 |
| Choline Chloride | 0.1 | Choline Chloride | 0.18 |
| Citric Acid | 0.8 | Citric Acid | 2 |
| Zinc Oxide | 0.2 | Zinc Oxide | 0.02 |
| Dairy Salt | 0.4 | Dairy Salt | 0.6 |
| Lysine | 0.62 | Lysine | 0.52 |
| Methionine | 0.09 | | 0.26 |
| Threonine | 0.25 | | 0.18 |
| Tryptophan | 0.06 | | 0.02 |
| Piglet Core Diet | 1.1a | Core Diet[b] | 1.05 |
| Total | 100 | | 100 |

a. The core teaching feed includes 0.1 kg multivitamins, 0.16 kg choline, 0.05 kg antioxidant, 0.05 kg mold inhibitor, 0.05 kg copper pentahydrate, 0.03 kg manganese monohydrate, 0.03 kg zinc monohydrate, 0.06 kg ferrous sulfate monohydrate, 0.001 kg 1% iodine, 0.001 kg 1% selenium, 0.001 kg 1% cobalt, and 0.567 kg zeolite powder.

b. The core nursery feed includes 0.1 kg multivitamins, 0.05 kg mold inhibitor, 0.05 kg copper pentahydrate, 0.03 kg manganese monohydrate, 0.03 kg zinc monohydrate, 0.06 kg ferrous sulfate monohydrate, 0.001 kg 1% iodine, 0.001 kg 1% selenium, 0.001 kg 1% cobalt, and 0.727 kg zeolite powder.

**4 Test Parameters and Data Analysis**

On days 28 and 42 of the trial, pigs were weighed with empty stomach in the pigsty, and the data were recorded. Daily feed intake was recorded throughout the trial, and the feed conversion ratio was calculated. Pig diarrhea and mortality were documented. Daily observations of pig feeding, water intake, and growth were documented, with detailed monitoring and recording of adverse reactions. Diarrhea Index = Total Diarrhea Score / (Number of Test Pigs) * Number of days * Daily Scoring Frequency)

Table 26 Criteria for Scoring of Piglet Diarrhea Index

| Feces Appearance | Diarrhea Severity | Diarrhea Score |
|---|---|---|
| Formed or granular | Normal | 0 |
| Soft, formed | Mild | 1 |
| Pasty, unformed | Moderate | 2 |
| Liquid, unformed | Severe | 3 |

**4.1 Blood Biochemical Indicators**

On Day 42 of the trial, one pig with a body weight close to the group's average weight was selected from each group and slaughtered. Blood was collected for serum biochemical analysis.

**4.2 Organ Index**

On Day 42 of the trial, one pig with a body weight close to the group's average weight was selected from each group and slaughtered. At slaughter, the liver, spleen, and kidneys were weighed to calculate relative weight (relative weight = organ weight / slaughter weight).

**4.3 Intestinal Morphology**

During slaughter, 2-cm segments of the middle duodenum, anterior 1/4 of the jejunum, and middle ileum were collected. The contents were washed, the specimens were fixed in paraformaldehyde, sectioned, and stained with hematoxylin-eosin (HE). For each section, a field with intact, straight villi was selected to measure the height of 10 complete villi and the depth of their crypts.

**4.4 Intestinal Digestive Enzyme Activity, Antioxidant Capacity, and Immune Factor Expression**

The concentrations or activities of intestinal alkaline phosphatase, malondialdehyde (MDA), total antioxidant capacity (T-AOC), superoxide dismutase (SOD), and secretory IgA (sIgA) were measured. The mRNA expression levels of intestinal IL-10, TNF-α, IL-6, TGF-β1, MUC2, and ZO1 were tested.

4.5 Intestinal Microbial Analysis

Ileal 16S gene sequencing was performed at Beijing Nuohe Zhiyuan Technology Co., Ltd.

**4.6 Statistical Analysis of Data**

Data were preliminarily processed using Excel and analyzed by analysis of variance (ANOVA) with SAS statistical software (SAS 9.4). Results were expressed as mean ± standard deviation (Mean ± SD). A p-value of less than 0.05 ($p < 0.05$) was considered statistically significant.

**5 Results and Analysis**

**5.1 Growth Performance**

The piglets were weighed with empty stomach on Day 28 and Day 42 of the trial. Piglet growth performance and diarrhea incidence are shown in Table 27.

Table 27 Impact of L-β-Galactoglucan on Body Weight of Weaned Piglets

| Body Weight/kg | 0.00g/t | 400g/t | 600g/t | SEM | P-value |
|---|---|---|---|---|---|
| BW0 | 7.43 | 7.48 | 7.48 | 1.49 | 0.915 |
| BW28 | 17.52 | 18.08 | 17.54 | 2.97 | 0.332 |
| BW42 | $25.46^{b}$ | $27.17^{a}$ | $26.04^{ab}$ | 3.96 | 0.046 |
| Average Daily Feed Intake / kg | | | | | |
| D0-28 | 0.73 | 0.78 | 0.77 | 0.12 | 0.337 |
| D28-42 | $0.87^{b}$ | $1.01^{a}$ | $0.99^{a}$ | 0.11 | 0.006 |
| D0-42 | 0.82 | 0.87 | 0.84 | 0.13 | 0.177 |
| Average Daily Weight Gain / kg | | | | | |
| D0-28 | 0.36 | 0.38 | 0.37 | 0.07 | 0.393 |
| D28-42 | 0.57 | 0.65 | 0.61 | 0.14 | 0.064 |
| D0-42 | $0.43^{b}$ | $0.47^{a}$ | $0.44^{ab}$ | 0.09 | 0.043 |
| Feed-to-Gain Ratio (g/g) | | | | | |
| D0-42 | 1.85 | 1.81 | 1.86 | 0.05 | 0.697 |
| Diarrhea Index | | | | | |
| D0-42 | $0.12^{a}$ | $0.11^{a}$ | $0.02^{b}$ | 0.05 | 0.007 |

The results indicate that compared to the control group and the 600 g/t dosage group, the 400 g/t dosage of L-β-galactoglucan significantly increased body weight and average daily weight gain for 21-day-old weaned piglets after 42 days of feeding ($P < 0.05$). The feed intake of piglets in 400 g/t and 600 g/t dosage groups was significantly higher than that in the control group during 28-42 days of feeding ($P < 0.05$). The diarrhea index of animals in 600 g/t L-β-galactoglucan group showed significant differences compared to those of control group and 400 g/t group, therefore, the high-dose L-β-galactoglucan has anti-diarrheal effects.

### 5.2 Impact of L-β-Galactoglucan on the Blood Biochemistry of Weaned Piglets

On the day of slaughter, blood was collected and serum levels of the following were measured: total protein (TP), albumin (ALB), alanine aminotransferase (ALT), aspartate aminotransferase (AST), alkaline phosphatase (ALP), gamma-glutamyltransferase (GGT), lactate dehydrogenase (LDH), blood urea nitrogen (BUN), creatinine (CREA), glucose (GLU), triglycerides (TG), cholesterol (CHOL), low-density lipoprotein cholesterol (LDL-C), high-density lipoprotein-cholesterol (HDL-C), diamine oxidase (DAO), blood ammonia ($NH_3L$), immunoglobulin G (IGG), and Immunoglobulin M (IGM) in serum.

Table 28 Impact of L-β-Galactoglucan on Blood Biochemistry in Weaned Piglets

| | 0.00g/t | 400g/t | 600g/t | SEM | P-value |
|---|---|---|---|---|---|
| **TP** | 59.30 | 61.71 | 57.93 | 2.80 | 0.085 |
| **ALB** | 37.80 | 42.26 | 42.21 | 4.47 | 0.156 |
| **ALT** | 76.64 | 73.56 | 64.86 | 16.46 | 0.378 |
| **AST** | 43.29 | 60.86 | 60.86 | 15.89 | 0.116 |
| **ALP** | 287.71 | 259.43 | 285.43 | 58.73 | 0.634 |
| **GGT** | 64.71 | 53.14 | 58.86 | 22.14 | 0.642 |
| **LDH** | 813.43 | 1062.57 | 1011.71 | 213.17 | 0.082 |
| **BUN** | 2.81 | 2.73 | 2.24 | 0.77 | 0.214 |
| **CREA** | 74.83 | 78.14 | 81.71 | 8.74 | 0.394 |
| **GLU** | 7.07 | 6.84 | 7.33 | 0.77 | 0.439 |
| **TG** | 0.33 | 0.32 | 0.37 | 0.08 | 0.538 |
| **CHOL** | 2.27 | 2.51 | 2.56 | 0.29 | 0.082 |
| **LDLC3** | 1.23 | 1.35 | 1.47 | 0.21 | 0.035 |
| **HLDC4** | 1.12 | 1.15 | 1.14 | 0.07 | 0.719 |
| **DAO** | 1.23 | 1.27 | 1.38 | 0.32 | 0.703 |
| **$NH_3L$** | 218.13 | 184.26 | 199.33 | 41.10 | 0.059 |
| **IGG** | 1.36 | 1.69 | 1.38 | 0.19 | 0.051 |
| **IGM** | 0.52 | 0.58 | 0.43 | 0.12 | 0.063 |

The results showed that compared to animals in the control group or the 600 g/t dosage group, animals in the 400 g/t dosage group showed a trend of increased serum TP, LDH, and IGG levels ($0.05 < P < 0.1$).

### 5.3 Impact of L-β-Galactoglucan on Organ Weight in Weaned Piglets

Table 29 Impact of L-β-Galactoglucan on Organ Index in Weaned Piglets

| g/kg | 0.00g/t | 400g/t | 600g/t | SEM | P-value |
|---|---|---|---|---|---|
| Liver Index | 29.05 | 26.18 | 27.38 | 2.44 | 0.134 |
| Kidney Index | 4.88 | 4.86 | 4.69 | 0.33 | 0.537 |
| Spleen Index | 1.89 | 1.76 | 1.74 | 0.34 | 0.646 |

Table 29 indicates that L-β-galactoglucan had no significant impact on liver, kidney, or spleen indicators of weaned piglets ($P > 0.05$).

### 5.4 Impact of L-β-Galactoglucan on Intestinal Morphology of Weaned Piglets

The following table indicates that L-β-galactoglucan has no significant impact on intestinal morphology of piglets. The animals from 600 g/t dosage group showed a trend of reduced jejunal villus-to-crypt ratio compared to that of control group ($0.05 < P < 0.1$).

Table 30 Impact of L-β-Galactoglucan on Intestinal Morphology in Weaned Piglets

| | 0.00g/t | 400g/t | 600g/t | SEM | P-value |
|---|---|---|---|---|---|
| | | Duodenum | | | |
| Crypts, μm | 167.90 | 162.06 | 196.67 | 34.77 | 0.085 |
| Villi, μm | 189.31 | 184.88 | 225.61 | 25.14 | 0.057 |
| Villi-Crypt Ratio | 1.25 | 1.18 | 1.12 | 0.22 | 0.752 |
| | | Jejunum | | | |
| Crypts, μm | 124.55 | 125.94 | 128.73 | 11.49 | 0.799 |
| Villi, μm | 211.05 | 193.92 | 180.23 | 10.69 | 0.116 |
| Villi-Crypt Ratio | 1.62 | 1.55 | 1.35 | 0.15 | 0.062 |
| | | Ileum | | | |
| Crypts, μm | 109.79 | 107.39 | 101.50 | 14.82 | 0.572 |
| Villi, μm | 167.58 | 161.29 | 154.30 | 21.78 | 0.539 |
| Villi-Crypt Ratio | 1.55 | 1.53 | 1.52 | 0.22 | 0.974 |

**5.5 Impact of L-β-galactoglucan on Intestinal Enzyme Activity and Antioxidant Capacity of Weaned Piglets**

L-β-Galactoglucan has no significant impact on alkaline phosphatase activity, T-AOC, MDA, or sIgA levels in the jejunum and ileum of piglets. The 600 g/t of L-β-galactoglucan significantly increased SOD activity in the ileum of piglets ($P < 0.05$).

Table 31 Impact of L-β-galactoglucan on intestinal T-AOC

| Unit / (mmol/g) | 0.00g/t | 400g/t | 600g/t | SEM | P-value |
|---|---|---|---|---|---|
| Jejunum | 0.11 | 0.11 | 0.25 | 0.14 | 0.332 |
| Ileum | 0.18 | 0.21 | 0.30 | 0.07 | 0.155 |

Table 32 Impact of L-β-galactoglucan on intestinal MDA content

| Unit / (mol/mgprot) | 0.00g/t | 400g/t | 600g/t | SEM | P-value |
|---|---|---|---|---|---|
| Jejunum | 8.06 | 8.98 | 9.88 | 2.79 | 0.110 |
| Ileum | 23.06 | 16.38 | 17.70 | 8.16 | 0.270 |

Table 33 Impact of L-β-galactoglucan on intestinal alkaline phosphatase activity

| Unit / (king-armstrong unit) / gprot) | 0.00g/t | 400g/t | 600g/t | SEM | P-value |
|---|---|---|---|---|---|
| Jejunum | 1.82 | 1.64 | 1.30 | 0.40 | 0.337 |
| Ileum | 0.78 | 0.42 | 0.55 | 0.25 | 0.113 |

Table 34 Impact of L-β-galactoglucan on intestinal SOD activity

| Unit / (U/mgprot) | 0.00g/t | 400 g/t | 600 g/t | SEM | P-value |
|---|---|---|---|---|---|
| Jejunum | 13.76 | 19.84 | 16.62 | 6.12 | 0.272 |
| Ileum | $27.23^{b}$ | $24.00^{b}$ | $32.85^{a}$ | 3.94 | 0.002 |

Table 35 Impact of L-β-galactoglucan on intestinal sIgA content

| Unit / (μg/ml) | 0.00g/t | 400 g/t | 600 g/t | SEM | P-value |
|---|---|---|---|---|---|
| Jejunum | 9.56 | 9.34 | 8.48 | 1.15 | 0.241 |
| Ileum | 9.71 | 9.96 | 10.27 | 1.03 | 0.613 |

**5.6 Impact of L-β-galactoglucan on gene expression of inflammatory factor pathways in the duodenum and jejunum of weaned piglets**

As shown in the table below, L-β-galactoglucan had no significant impact on the expression of TGF, TNF-α, IL-6, IL-10, ZO1, and MUC2 genes in the duodenum, jejunum, and ileum of piglets ($P > 0.05$).

Table 36 Impact of L-β-Galactoglucan on Intestinal Cytokines and Tight Junction Protein

| | 0.00g/t | 400g/t | 600g/t | SEM | P-value |
|---|---|---|---|---|---|
| | | Duodenum | | | |
| **TGF-β1** | 1.00 | 0.82 | 0.90 | 0.26 | 0.190 |
| **TNF-α** | 1.00 | 1.11 | 1.07 | 0.41 | 0.841 |
| **IL6** | 1.00 | 1.36 | 1.35 | 0.38 | 0.148 |
| **IL10** | 1.00 | 1.11 | 0.94 | 0.35 | 0.602 |
| **ZO1** | 1.00 | 1.19 | 1.21 | 0.16 | 0.077 |
| **MUC2** | 1.00 | 1.15 | 1.24 | 0.46 | 0.636 |
| Jejunum | | | | | |
| **TGF-β1** | 1.00 | 0.87 | 1.06 | 0.25 | 0.363 |
| **TNF-α** | 1.00 | 0.60 | 0.77 | 0.32 | 0.133 |
| **IL6** | 0.78 | 0.65 | 0.76 | 0.17 | 0.367 |
| **IL10** | 1.00 | 0.64 | 0.68 | 0.29 | 0.105 |
| **ZO1** | 1.00 | 0.95 | 0.94 | 0.13 | 0.501 |
| **MUC2** | 1.00 | 0.81 | 0.77 | 0.29 | 0.354 |
| **Ileum** | | | | | |
| **TGF-β1** | 1.00 | 1.02 | 0.90 | 0.27 | 0.622 |
| **TNF-α** | 1.00 | 0.91 | 0.89 | 0.37 | 0.761 |
| **IL6** | 1.00 | 0.71 | 0.77 | 0.28 | 0.233 |
| **IL10** | 0.92 | 0.83 | 0.93 | 0.26 | 0.607 |
| **ZO1** | 1.00 | 1.06 | 1.02 | 0.14 | 0.801 |
| **MUC2** | 1.00 | 0.87 | 0.86 | 0.43 | 0.776 |

**5.7 Intestinal Microbial Diversity Assessment**

To evaluate the impact of L-β-galactoglucan on the colonic microbiota of weaned piglets, the study compared theα- and β-diversity among the three groups. Based on the OTU, Chao, Shannon, and Simpson indices, dietary supplementation with L-β-galactoglucan had no significant impact on the abundance or evenness of the colonic microbiota. The predominant bacterial phyla in the piglet colon were Firmicutes and Proteobacteria. Dietary supplementation with L-β-galactoglucan reduced Firmicutes abundance while increasing Proteobacteria abundance, although the differences among the three groups were not statistically significant. Further analysis of the top 30 most abundant genera revealed that dietary L-β-galactoglucan supplementation decreased the abundance of Ornithinicoccus, Bacillus, and Frisingicoccus, while increasing the abundance of the short-chain fatty acid-producing genera Lachnoclostridium and Subdoligranulum.

Additionally, linear discriminant analysis (LDA) effect size (LEfSe) was employed to analyze the intestinal microbial composition. The results showed that Prevotella_9, Subdoligranulum, Phascolarctobacterium, and Ornithinicoccus exhibited significant differences between the 600 g/t L-β-galactoglucan group and the control group. Furthermore, Prevotella_9, Subdoligranulum, and Phascolarctobacterium were significantly enriched in the L-β-galactoglucan group, while Ornithinicoccus was significantly enriched in the control group.

Based on KEGG level-3 taxonomy 16S rRNA sequencing results, PICRUSt was applied for construction of metagenome. The results showed significant differences in functional pathway distribution among the groups. The study selected differentially regulated pathways related to microbial metabolism for analysis. Compared to the control group, the L-β-galactoglucan group exhibited significantly enhanced functional activity of genes associated with amino acid metabolism (e.g., alanine, aspartic acid, glutamic acid, glycine, serine, and threonine), while functional activity of genes related to glycolysis, gluconeogenesis, starch, and sucrose metabolism was significantly reduced.

Table 37 Impact of L-β-Galactoglucan on Colonic Bacterial Community Diversity in Piglets

| | 0.00g/t | 400g/t | 600g/t | SEM | P-value |
|---|---|---|---|---|---|
| chao | 135.83 | 165.11 | 167.60 | 16.895 | 0.522 |
| OUT | 134.86 | 164.00 | 166.71 | 16.732 | 0.479 |
| shannon | 3.49 | 3.24 | 3.71 | 0.116 | 0.309 |
| simpson | 0.84 | 0.77 | 0.86 | 0.016 | 0.117 |

**6 Experimental Conclusions**

The results showed that compared to the negative control group, the administration of 400 g/t L-β-galactoglucan dose significantly increased piglet body weight and average daily gain after 42 days of feeding. Compared to those of the control group or the 600 g/t dose group, the 400 g/t group showed a trend of increased serum TP, LDH, and IGG levels ($0.05 < P < 0.1$). The 600 g/t of L-β-galactoglucan significantly reduced diarrhea incidence in weaned piglets and markedly increased ileal SOD activity ($P < 0.05$). L-β-galactoglucan had no significant impact on the microbial diversity of the piglet colon. However, 600 g/t L-β-galactoglucan significantly enriched the abundance of Prevotella 9, Subdoligranulum, and Phascolarctobacterium, enhanced microbial amino acid metabolism, and reduced the functional expression of genes related to energy metabolism ($P < 0.05$).

In summary, dietary supplementation with L-β-galactoglucan (400 g/t) promote the growth of weaned piglets, with effects which may be apparent after approximately 1.5-2 months. The likely mechanism of action involves enhancing immune function. Concurrently, high dose of (600 g/t) L-β-galactoglucan has impact on intestinal microbial metabolism in piglets, and significantly reduces diarrhea incidence in weaned piglets.

**Efficacy Test 24 Impact on Canine Growth Performance, Palatability, and Health**

China's pet market is large, with the pet consumption market of CNY 279.3 billion in 2023 and an annual increase trend. In recent years, issues such as pet obesity, poor coat quality, diarrhea, and compromised immunity have got significant attention from pet owners. Prebiotics, which cannot be digested or absorbed by the body but can promote the proliferation of beneficial gut bacteria, have attracted researchers' interest due to their potential to improve overall health. Among these, β-glucan, as a high-quality prebiotic, has been extensively studied and applied in humans, mice, and livestock. Unlike β-glucan, galactoglucan is a heteropolysaccharide primarily found in plant seed endosperm. Its main chain consists of glucose and mannose units linked by β-1,4 bonds, while the side chains are attached to hexose residues on the main chain via α-1,6 bonds. Study showed that galactoglucan exhibits acid and alkali resistance, tolerance to high salt concentrations, high viscosity at low concentrations, and stability across a broad temperature range.

The intestinal microbiota includes bacteria, viruses, fungi, and protozoa, with bacteria of over 98% of the population. The intestinal flora of dogs and cats is dominated by strict anaerobes and facultative anaerobes, primarily comprised of Firmicutes, Fusobacteria, and Bacteroidetes. The intestinal microbiota of dogs and cats can convert diet and drugs into microbial metabolites. It is considered as a vital metabolic organ. A balanced intestinal flora can regulate the host's immune system through metabolic products, protect the host from pathogen invasion, and provide nutrients to promote host health.

Although studies have demonstrated the beneficial effects of β-glucan supplementation on canine health, the efficacy of β-galactoglucan supplementation in dogs remains unclear. Therefore, this study used Beagle dogs as the experimental animals to evaluate the impact of β-galactoglucan on growth performance, nutrient apparent digestibility, palatability, complete blood count, blood biochemistry, fecal quality, and intestinal flora composition, and provided reference for its application in canine diets.

**1 Materials and Methods**

**1.1 Test Materials**

The test sample was crude L-β-galactoglucan (water-soluble, total sugar purity 62.14%), which was prepared according to the method described in Example 1 and supplied by Chengdu Saidike Biotechnology Co., Ltd.

**1.2 Study Design**

32 healthy Beagle dogs were randomly divided into four groups (eight animals in each group). Diets were administered at fixed time points on each day. The daily diet for animals in control group was prepared according to the

standard recommended by NRC (2006), with nutritional levels shown in Table 38. The experimental diets were prepared by supplementing the control diet with β-galactoglucan at 0.25%, 0.5%, or 1%. Thus, the four groups were: : (1) Control group (n = 8), (2) 0.25% β-galactoglucan group (n = 8), (3) 0.5% β-galactoglucan group (n = 8), and (4) 1% β-galactoglucan group (n = 8), with 28 days of trial period. At the end of the trial, blood samples, hair samples, and fecal samples were collected for the analysis of blood biochemical parameters, microbial flora, and digestibility.

Table 38 Nutritional Levels of the Base Diet

| Item | Content, % |
|---|---|
| Crude protein | 21.89 |
| Ether extract | 8.5 |
| Ash | 7.5 |
| Crude fiber | 2.6 |
| Calcium | 1.96 |
| Acid insoluble ash | 0.9 |
| Phosphorus | 0.78 |
| GE / (MJ/kg) | 19.14 |

### 1.3 Measurement Indicators and Methods

#### 1.3.1 Growth Performance and Fecal Quality Scoring

Record weekly body weight and daily feed intake of dogs; Score daily feces according to the fecal scoring criteria and document the times of diarrhea episodes during the trial period. Fecal Score follows Carciofi's approach: 1 point: soft stool with no distinct shape; 2 points: soft stool with poor shape; 3 points: soft stool with moist shape; 4 points: good and consistent stool shape; 5 points: good stool form, firm and dry.

On Day 28 of the trial, hair samples were collected from the back of each dog. Scores of 1, 2, or 3 were assigned based on hair brightness, smoothness, and skin condition, respectively. The overall coat quality score was the sum of the three scores. The scoring criteria are shown in Table 39.

Based on scoring standard, with reference of Table 39:

Table 39 Coat Scoring Criteria

| Hair scoring items | State | Fraction | State | Fraction | State | Fraction |
|---|---|---|---|---|---|---|
| Wool brightness | Dull | 1 | General | 2 | Light | 3 |
| Hair compliance | Coarse | 1 | General | 2 | Soft | 3 |
| Skin state | Dry, flaky | 1 | General | 2 | Moist, glossy | 3 |

#### 1.3.2 Apparent Nutrient Digestibility

On Day 25 of the trial, all feces for 3 consecutive days were collected, weighed and stored at -20°C for analysis. Crude protein (GB/T 6432-2018), crude fat (GB/T 6433-2006), crude fiber (GB/T 6434-2006), and crude ash (GB/T 6438-2007) content in fecal samples were determined. The calculation formula of apparent digestibility of nutrient is as follows:

$$\text{Apparent digestibility of certain nutrient X (\%)} = 100-[100\times(b\times c)/(a\times d)]$$

In the formula: a refers to the content of nutrient X in the feed; b refers to the content of nutrient X in the fecal sample; c refers to the acid-insoluble ash content in the feed; d refers to the acid-insoluble ash content in the fecal sample.

#### 1.3.3 Blood Biochemistry

The venous blood was collected on Day 0 and Day 28. A portion of blood underwent complete blood count analysis, while the rest of the isolated serum was used for blood biochemistry and immunoglobulin testing. Immunoglobulins IgA, IgG, and IgM were tested using ELISA kits.

### 1.3.4 Gut Microbiota Composition

Fecal microbial DNA was extracted using the E.Z.N.A. ® Soil DNA Kit. Amplification was performed using universal primers 341F (5'-CCTACGGGRSGCAGCAG-3') and 806R (5'-GGACTACVVGGGTATCTAATC-3') to construct a library. High-throughput sequencing of 16S rDNA was conducted using the Illumina MiSeq platform. The sequencing service was provided by Shanghai Meiji Biotechnology Co., Ltd. The sequences were clustered into operational taxonomic units (OTU) based on ≥ 97% similarity.

### 1.3.5 Palatability

The daily feed intake of each dog and preferred diet was documented through the internationally accepted Two-Pan test method, to calculate the intake ratio. The intake ratio of 0.5 shows no preference difference between the two diets. The food intake ratio is as follows: Feed Intake Ratio (Diet A/B) = Feed Intake (Diet A/B) / Total Feed Intake (Diet A + Diet B).

### 1.3.6 Data Processing and Analysis

One-way Analysis of Variance (ANOVA) was performed on the data using SPSS software (version 26.0). The results are expressed as "mean ± standard error", with statistical significance defined as $P \leq 0.05$ and non-significance as $P > 0.05$.

## 2 Results

### 2.1. Impact of β-Glucan on Canine Growth Performance

Table 40 shows no significant differences among the groups in fecal score, initial body weight, final body weight, or hair score. Compared with the control group, dogs in all β-galactoglucan supplementation groups exhibited significantly reduced body weight ($P \leq 0.05$). Dogs in the 0.5% β-galactoglucan group showed an increase trend in hair score and fecal score, with improved fecal consistency.

Table 40 Impact of β-Galactoglucan on Growth Performance

| Items | β-galactoglucan | | | | P-value |
|---|---|---|---|---|---|
| | Control | 0.25% | 0.5% | 1% | |
| Fecal score | 2.63±0.08 | 2.68±0.04 | 2.73±0.17 | 2.65±0.05 | 0.067 |
| Initial BW, kg | 13.41±1.76 | 13.78±2.38 | 12.09±2.78 | 13.69±1.33 | 0.381 |
| Final BW, kg | 13.71±1.87 | 13.26±2.61 | 11.54±2.78 | 13.25±1.19 | 0.247 |
| Weight change, kg | 0.35±0.35a | -0.51±0.28b | -0.55±0.58b | -0.56±0.28b | <0.01 |
| Hair Score | 6.34±1.74 | 7.16±1.09 | 7.59±0.55 | 6.72±0.57 | 0.146 |

Note: The values are expressed as mean ± standard error, n = 8. Data points with no letter or identical letters indicate no significant difference ($P > 0.05$); different letters indicate significant differences ($P \leq 0.05$).

### 2.2 Impact of β-Galactoglucan on Apparent Digestibility of Dogs Nutrient

Table 41 shows that after 28 days of treatment, compared with that of the control group, apparent digestibility of crude fiber of animals in 0.25% β-galactoglucan supplementation group exhibited a significant decrease ($P \leq 0.05$); the addition of different levels of β-galactoglucan had no significant effect on the apparent digestibility of crude protein, crude fat, and crude ash ($P > 0.05$).

Table 41 Impact of β-Galactoglucan on Apparent Digestibility

| Items | β-galactoglucan | | | | P-value |
|---|---|---|---|---|---|
| | Control | 0.25% | 0.5% | 1% | |
| Crude Protein CP, % | 95.49±1.12 | 95.64±0.85 | 95.49±0.69 | 95.12±1.14 | 0.738 |
| Crude Fat EE, % | 95.24±1.24 | 95.48±0.16 | 95.25±0.69 | 95.66±0.64 | 0.698 |
| Crude Ash, % | 81.24±2.82 | 82.98±1.66 | 83.70±1.95 | 82.81±1.08 | 0.110 |

(continued)

| Items | β-galactoglucan | | | | P-value |
|---|---|---|---|---|---|
| | Control | 0.25% | 0.5% | 1% | |
| Crude Fiber CF, % | 71.29±5.04a | 61.28±9.89b | 66.46±9.42ab | 71.08±3.78a | 0.040 |
| Note: The values are expressed as mean ± standard error, n = 8. Data points with no letter or identical letters indicate no significant difference (P > 0.05); different letters indicate significant differences (P ≤ 0.05). | | | | | |

**2.3 Impact of β-Galactoglucan on the Complete Blood Count of Dogs**

Table 42 shows that after 28 days of treatment, there were no significant differences (P > 0.05) in white blood cell count, red blood cell count, hemoglobin content, platelet count, hematocrit, mean corpuscular volume, and mean corpuscular hemoglobin content between the β-galactoglucan supplementation groups and the control group.

Table 42 Impact of β-Galactoglucan on the Complete Blood Count

| Items | β-galactoglucan | | | | P-value |
|---|---|---|---|---|---|
| | Control | 0.25% | 0.5% | 1% | |
| White Blood Cell Count WBC / ($\times 10^9$/L) | 11.42±2.40 | 11.43±2.32 | 11.67±3.57 | 12.04±2.62 | 0.967 |
| Red Blood Cell Count RBC / ($\times 10^{12}$/L) | 7.17±0.86 | 7.11±0.46 | 7.24±0.88 | 7.17±0.95 | 0.992 |
| Hemoglobin HGB / (g/L) | 163.63±22.83 | 158.25±16.76 | 156.63±22.52 | 154.25±22.80 | 0.841 |
| Platelet Count | 274.75±75.58 | 293.13±88.93 | 341.75±68.21 | 295.00±51.99 | 0.312 |
| PLT/($\times 10^9$/L) | | | | | |
| Hematocrit | 47.65±5.24 | 46.1±2.73 | 47.39±5.83 | 46.93±6.97 | 0.944 |
| Mean Corpuscular Volume MCV/fL | 64.83±1.41 | 63.64±2.18 | 65.45±1.67 | 65.3±2.04 | 0.216 |
| Mean Corpuscular Hemoglobin MCH/pg | 21.65±0.57 | 21.46±0.52 | 21.59±0.53 | 21.46±0.55 | 0.869 |
| Note: The values are expressed as mean ± standard error, n = 8. Data points with no letter or identical letters indicate no significant difference (P > 0.05); different letters indicate significant differences (P ≤ 0.05). | | | | | |

**2.4 Impact of β-Galactoglucan on Canine Serum Biochemistry**

Table 43 shows that there were no significant differences in blood glucose, total protein, albumin, globulin, alanine aminotransferase, blood lipid, and high-density lipoprotein levels between the β-galactoglucan supplementation groups and the control group. Compared with the control group and the 1% β-galactoglucan group, the 0.5% β-galactoglucan group showed a significant decrease in low-density lipoprotein (LDL) levels (P ≤ 0.05).

Table 43 Impact of β-Galactoglucan on Blood Biochemistry

| Items | β-galactoglucan | | | | P-value |
|---|---|---|---|---|---|
| | Control | 0.25% | 0.5% | 1% | |
| Blood Glucose Glu / (mmol/L) | 5.64±0.45 | 5.57±0.31 | 6.07±0.70 | 5.98±0.46 | 0.142 |
| Total Protein TP / (g/L) | 59.06±1.82 | 59.73±3.41 | 61.86±3.76 | 61.24±4.30 | 0.352 |
| Albumin ALB / (g/L) | 32.66±1.54 | 32.16±1.95 | 31.78±2.60 | 31.38±1.74 | 0.616 |
| Globulin LOB / (g/L) | 26.40±2.29 | 27.56±2.78 | 30.09±3.71 | 29.86±2.97 | 0.052 |
| Alanine Aminotransferase ALT / (U/L) | 38.63±14.45 | 40.63±17.97 | 50.50±33.46 | 33.50±15.40 | 0.474 |
| Blood Lipid TG/(mg/dL) | 60.46±17.42 | 56.15±10.93 | 49.95±10.87 | 42.46±10.33 | 0.06 |
| High-Density Lipoprotein HDL-C / (mg/dL) | 150.39±36.14 | 124.26±5.08 | 127.64±20.13 | 135.92±20.49 | 0.157 |

(continued)

| Items | β-galactoglucan | | | | P-value |
|---|---|---|---|---|---|
| | Control | 0.25% | 0.5% | 1% | |
| Low-Density Lipoprotein LDL-C/(mg/dL) | 10.75±4.13[a] | 8.26±1.87[ab] | 6.20±1.28[b] | 9.40±2.96[a] | 0.033 |
| Aspartate Transaminase AST / (U/L) | 31.35+8.59[bc] | 41.96±10.48[a] | 41.40±13.72[ab] | 26.3±3.82[c] | 0.007 |

Note: The values are expressed as mean ± standard error, n = 8. Data points with no letter or identical letters indicate no significant difference ($P > 0.05$); different letters indicate significant differences ($P \leq 0.05$).

**2.5 Impact of β-Galactoglucan on Canine Serum Immunoglobulin**

Table 44 shows that the IgA, IgG, and IgM levels in all β-galactoglucan supplementation groups showed no significant differences compared with those in the control group ($P > 0.05$).

Table 44 Impact of β-Galactoglucan on Serum Immunoglobulins

| Items | β-galactoglucan | | | | P-value |
|---|---|---|---|---|---|
| | Control | 0.25% | 0.5% | 1% | |
| Immunoglobulin A IgA, μg/mL | 1.42±0.09 | 1.40±0.08 | 1.46±0.13 | 1.45±0.09 | 0.576 |
| Immunoglobulin G IgG, μg/mL | 1.15±0.05 | 1.11±0.03 | 1.13±0.04 | 1.12±0.03 | 0.216 |
| Immunoglobulin M IgM, μg/mL | 49.62±2.41 | 48.15±2.96 | 46.20±3.69 | 47.37±3.25 | 0.189 |

Note: The values are expressed as mean ± standard error, n = 8. Data points with no letter or identical letters indicate no significant difference ($P > 0.05$); different letters indicate significant differences ($P \leq 0.05$).

**2.6 Impact of β-Galactoglucan on Canine Fecal Microbes**

Compared with the control group, the animals in the 0.5% β-galactoglucan supplementation group showed a significantly increased microbial α-diversity (Chao index, $P \leq 0.05$) and significantly decreased Simpson index ($P \leq 0.05$). There was no significant difference in Shannon index between the control group and the supplemented groups.

Venn diagrams were used to statistically analyze the number of shared and unique species across multiple groups or samples (Figure 59). As shown in Figure 59A (Venn diagram of fecal microbiota), 114 bacterial species were shared between the control and the experimental groups. Each group also contained a unique intestinal flora: 4 in the control group, 17 in the 0.25% β-galactoglucan group, 22 in the 0.5% β-Galactoglucan group, and 23 in the 1% β-Galactoglucan group. As shown in Figure 59B (fecal microbial β-diversity, PCOA analysis), the microbial community structures in the β-galactoglucan supplementation groups with different concentrations exhibited a tendency to diverge from the control group. As shown in Figure 59C, at the phylum level, the fecal microbiota was dominated by Firmicutes, followed by Actinobacteria and Bacteroidetes. Compared with the control group, the 0.5% β-galactoglucan group showed a decreasing trend in the relative abundance of Firmicutes, while the relative abundances of Actinobacteria and Bacteroidetes increased. As shown in Figure 59D, at the genus level, the fecal microbiota primarily included Lactobacillus as the dominant genus, Peptoclostridium, Blautia, Turicibacter, Romboutsia, unclassified _f_Peptostreptococca, and Collinsella. Compared with the control group, the relative abundance of Lactobacillus showed a decrease trend in animals from the 0.25% β-galactoglucan addition group and the 0.5% β-galactoglucan addition group; the relative abundance of Blautia and Peptoclostridium significantly increased in the 0.25% β-galactoglucan addition group ($P \leq 0.05$), while Turicibacter and unclassified_f_Peptostreptococca showed an increase trend.

**2.7 Impact of β-Galactoglucan on Palatability in Dogs**

Table 45 shows that in the palatability comparison among diets with different concentrations of β-galactoglucan, the intake rate was significantly higher in the 0.25% β-galactoglucan group than in the control group ($P \leq 0.05$). Feed intake and the preference rate showed an upward trend. Compared with the 1% β-galactoglucan supplementation group, dogs in the 0.5% β-Galactoglucan supplementation group showed increased feed intake, intake rate, and preference rate. Dogs in the 0.5% β-galactoglucan supplementation group demonstrated improved palatability.

Table 45 Impact of β-Galactoglucan on Palatability

| Items | Test I | | P-value | Test II | | P-value |
|---|---|---|---|---|---|---|
| | Control | 0.25% | | 0.5% | 1% | |
| Daily Intake, g/d | 43.40±37.86 | 50.31±35.38 | 0.554 | 52.55±37.45 | 34.42±24.30 | 0.083 |
| Intake Rate, % | 42.31±19.14[b] | 57.70±19.14[a] | 0.018 | 53.63±21.47 | 46.37±21.47 | 0.304 |
| Preference Rate, % | 47.37% | 52.63% | | 61.54% | 38.46% | |

Note: The values are expressed as mean ± standard error, n = 8. Data points with no letter or identical letters indicate no significant difference (P > 0.05); different letters indicate significant differences (P ≤ 0.05).

### 3 Discussion

### 3.1. Impact of β-Glucan on Canine Growth Performance

Fecal quality, body weight change, and coat condition scores provide convenient indicators for assessing pet dog health. The study investigated the impact of β-galactoglucan on canine growth performance. Compared to the control group, addition of β-galactoglucan had no significant impact on fecal scores or coat scores. However, the body weight decreases significantly (P ≤ 0.05) for dogs in β-galactoglucan supplementation groups. Wu et al. showed that after replacing corn starch with 5.83% oat β-glucan in the diet of 21-day-old weaned piglets, the average daily feed intake and average daily gain decreased. The dry matter, crude protein, crude fiber, crude fat, and apparent digestibility of calcium and phosphorus in feces are decreased. Furthermore, Santos et al. showed that the addition of dehydrated yeast culture makes the apparent digestibility of crude fiber in dogs increased, and makes the digestibility of crude protein and nitrogen-free extract (P < 0.05) increased. Another study showed that the addition of oat β-glucan extract tends to reduce the apparent digestibility of crude protein, dry matter, and minerals in dogs. Sweeney et al. found that the addition of 250 mg/kg yeast or seaweed β-glucan to diets had no impact on growth performance or digestibility of dry matter, organic matter, and minerals in piglets. Therefore, β-glucan supplementation does not alter animal natural growth patterns or compromise health. Under the experimental conditions, compared with the control group, the apparent digestibility of crude protein, crude fat, and crude ash in dogs supplemented with β-galactoglucan showed no significant difference. The 0.25% β-galactoglucan group exhibited a significantly reduced apparent digestibility of crude fiber. This may be due to the fact that β-galactoglucan increases the viscosity of digesta in the digestive tract and prolongs gastric emptying, thereby indirectly affecting body weight change and apparent digestibility. Therefore, it shows potential for application in the feeding of obese animals.

### 3.2. Impact of β-Galactoglucan on Canine Blood Biochemistry

Animal health status and metabolism can be judged based on serum biochemistry and complete blood count tests. Low-density lipoprotein (LDL) is an apolipoprotein with main function of transporting cholesterol from the liver to other body tissues. Low content of low-density lipoprotein is generally considered an indicator of good health. Under the experimental conditions, compared to the control group and the 1% β-galactoglucan group, the 0.5% β-galactoglucan group exhibited a significantly reduced low-density lipoprotein level (P ≤ 0.05). The result showed that dogs in the 0.5% β-galactoglucan group had a lower risk of cardiovascular disease. Ferreira et al. demonstrated that dogs in the 1% oat β-glucan supplementation group exhibited lower serum total cholesterol, low density and very low-density lipoprotein (VLDL) concentrations (P < 0.05) compared to the control group.

The primary immunoglobulins in blood include IgA, IgM, and IgG. Study on the mechanism of immune function by Hofer et al. showed that glucans stimulate immune cell proliferation and enhance immunity through specifically binding to immune cells in the body. Shao et al. found that dietary supplementation with a certain dose of β-glucan increased serum IgA and IgG levels in broiler chickens. Stuyven et al. demonstrated that daily supplementation of yeast β-glucan for 4 weeks resulted in significantly reduced serum IgA levels and significantly elevated serum total IgM levels in experimental dogs compared to controls, with no impact on IgG levels. These effects disappeared one week after discontinuing β-glucan supplementation. Under the experimental conditions, compared with the control group, the immunoglobulin content in dogs administered β-galactoglucan did not show significant changes. The possible cause is that β-galactoglucan indirectly affects the overall immune system function by activating immune cells, thereby exerting minimal direct impact on immunoglobulin production.

### 3.3. Impact of β-Galactoglucan on Palatability in Dogs

Palatability is a crucial indicator for evaluating pet diets. Pet diets with good palatability can increase owners' willingness to purchase, ensure adequate pet intake, and thereby promote pet health. Pet diet palatability is influenced by animal factors (e.g., species, breed, weight, gender), dietary factors (e.g., raw materials, nutritional components, feed additives, processing methods), and environmental factors (e.g., season, temperature, humidity). It is primarily manifested in its effects on feeding behaviors such as feeding enthusiasm and frequency. Under the experimental conditions, compared with the control group, dogs in the 0.25% β-galactoglucan supplementation group showed a significantly increased intake rate ($P \leq 0.05$), with increasing trends in intake amount and preference rate. Compared to the 1% β-galactoglucan group, dogs in the 0.5% β-galactoglucan group showed increasing trends in intake amount, intake rate, and preference rate. A comprehensive analysis of the experimental results indicated that dogs exhibited good palatability for β-galactoglucan with concentrations of 0.25% and 0.5%.

### 3.4. Impact of β-Galactoglucan on Canine Fecal Microbes

The gut microbiota, which coexists with the host, comprises diverse and abundant bacterial species and is crucial for host health. Intestinal microbial diversity is commonly reflected by alpha diversity and beta diversity. Alpha diversity is used to assess species richness and diversity within an individual sample. The Chao1 index represents species richness in a sample, while the Simpson index and the Shannon index reflect species diversity. Higher values of these indices indicate greater diversity.

The study results demonstrated that β-galactoglucan does not change the composition of the core microbiota or the structure of normal flora at the phylum level in the canine intestine. At the genus level, compared to the control group, the 0.25% β-galactoglucan supplementation group showed a significant increase in the relative abundance of Blautia and Peptoclostridium ($P \leq 0.05$), while the relative abundance of Turicibacter and unclassified _f_Peptostreptococca showed an increasing trend. In summary, β-galactoglucan may promote gut health by enhancing the proliferation of beneficial bacteria such as Blautia, Peptoclostridium, and Turicibacter, and by shaping a slightly acidic intestinal environment, thereby improving palatability and overall health in pets.

### 4 Conclusion

In this trial, the addition of β-galactoglucan of different concentrations significantly reduced body weight in dogs. Therefore, it has a potential for use in feeding obese animals. β-galactoglucan improves feed palatability. compared to the other groups, dogs in the 0.25% and 0.5% β-galactoglucan supplementation groups exhibited superior palatability. β-galactoglucan supplementation reduces the risk of cardiovascular disease. Compared to the control group and the 1% β-galactoglucan group, dogs in the 0.5% β-galactoglucan group exhibited significantly lower LDL levels ($P \leq 0.05$). β-galactoglucan promotes probiotic proliferation. Compared to other groups, the animals in 0.25% β-galactoglucan group showed increased relative abundance of beneficial bacteria, including Blautia, Peptoclostridium, and Turicibacter. Additionally, intestinal species richness was enhanced in dogs fed β-galactoglucan.

β-galactoglucan demonstrates application value in pet diets. It contributes to obesity reduction, palatability enhancement, increased relative abundance of beneficial gut bacteria, and improvements in stool consistency and coat quality. The recommended inclusion level of β-galactoglucan in dog food is 0.25-0.5%.

### Efficacy Test 25: Impact of L-β-Galactoglucan on Malassezia

Malassezia was cultured in a medium containing maltose agar, bovine bile (dried), Tween 40, glycerin monolaurate, and chloramphenicol. Malassezia cells were harvested, washed with sterile PBS, and prepared into a bacterial suspension of $1 \times 10^7$ cells/mL for subsequent use. Then, HaCaT keratinocytes in the logarithmic growth phase were taken and a cell suspension of $1\times10^5$ cells/mL was prepared using DMEM for later use. A 0.1% biopolysaccharide stock solution was prepared using DMEM medium for later use. For the blank control group, 2 mL of HaCaT cell suspension and 1 mL of DMEM medium were added, mixed thoroughly, and inoculated into a 35 mm culture dish. For the model control group, 200 μL of Malassezia suspension, 2 mL of HaCaT cell suspension, and 800 μL DMEM medium were mixed thoroughly and inoculated into a 35 mm culture dish. For the 0.0039% biopolysaccharide group, 200 μL of Malassezia suspension, 2 mL of HaCaT cell suspension, 117 μL of the 0.1% biopolysaccharide stock solution, and 683 μL of DMEM medium were combined, mixed thoroughly, and inoculated into a 35 mm culture dish. For the 0.0078% biopolysaccharide group, 200 μL of Malassezia suspension, 2 mL of HaCaT cell suspension, 234 μL of the 0.1% biopolysaccharide stock solution, and 566 μL of DMEM medium were combined, mixed thoroughly, and inoculated into a 35 mm culture dish. For the 0.0156% biopolysaccharide group, 200 μL of Malassezia suspension, 2 mL of HaCaT cell suspension, 468 μL of the 0.1% biopolysaccharide stock solution and 332 μL of DMEM medium were combined, mixed thoroughly, and inoculated

into a 35 mm culture dish. All groups of cells were placed in a 37°C $CO_2$ incubator for continuous culture for 24 hours. The cells of all groups were collected and washed three times with PBS. 1 mL of Trizol was added to each group for RNA extraction. RNA was reverse-transcribed into cDNA using a reverse transcription kit. The mRNA expression level of the IL-6 gene was quantified by RT-qPCR using the SYBR Green method. Statistical results are expressed as mean ± SEM. Statistical analysis was performed using GraphPad Prism 8.0 software. A p-value of less than 0.05 ($p < 0.05$) was considered statistically significant.

Test results are shown in Table 46 and Figure 60. Following Malassezia processing, the relative expression level of IL-6 gene in the model control group cells was 4089.85 ± 424.1%, representing a 3989.85% upregulation ($p < 0.001$), and confirming the successful establishment of the Malassezia-induced inflammatory model. Under equivalent conditions, relative IL-6 gene expression levels in cells which were processed with 0.0039%, 0.0078%, and 0.0156% biopolysaccharide were 3153.66 ± 281.0%, 1440.53 ± 209.3 and 739.05 ± 44.83%, respectively. Compared with the model control group, these values represented downregulation of 936.19% ($p > 0.05$), 2649.32% ($p < 0.001$), and 3350.8% ($p < 0.001$), respectively, indicating that biopolysaccharide at these concentrations significantly inhibits Malassezia-induced IL-6 gene expression. Under the experimental conditions, the biopolysaccharide exhibited scalp-soothing efficacy.

Table 46 Impact of the biopolysaccharide on relative IL-6 gene expression level in Malassezia-induced HaCaT cells.

| Group | Blank Control | Model ControlGroup | 0.0039% | 0.0078% | 0.0156% |
|---|---|---|---|---|---|
| Relative expression levels of IL-6 gene (%) | 100.00 ± 6.43 | 4089.85 ± 424.1 ### | 3153.66 ± 281.0 | 1440.53 ± 209.3*** | 739.05 ± 44.83* ** |
| P-value | / | <0.001 | >0.05 | <0.001 | <0.001 |
| Note: Compared with the blank control group: #p < 0.05, ##p < 0.01, ###p < 0.001; Compared with the model control group: *p < 0.05, **p < 0.01, ***p < 0.001. | | | | | |

## Claims

1. An L-β-galactoglucan, wherein its repeating unit structural formula I is as follows:

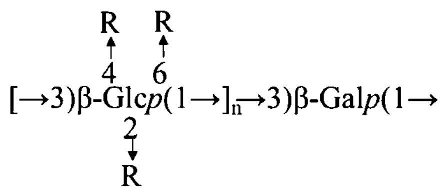

R= →2,3,4,6) β-Glcp (1→

I

wherein, *Glcp* is glucose, *Galp* is galactopyranose, and n is an integer not less than 30;
wherein, the content of glucose is 94%-98%, and the content of galactose is 2%-6%.

2. The L-β-galactoglucan according to claim 1, wherein the L-β-galactoglucan is levorotatory and has a molecular weight of at least 5000 Da.

3. The L-β-galactoglucan according to claim 1, wherein its weight-average molecular weight is 4.5 million to 5.5 million Da.

4. A method for preparing the L-β-galactoglucan according to claim 1, wherein the method comprises the following steps:

(1) Inoculating *Agrobacterium* sp. FN01 into a culture broth for fermentation to obtain a fermentation broth;
(2) Precipitating the fermentation broth with alcohol and oven-drying to obtain a crude polysaccharide, dissolving the crude polysaccharide, adding a base and a filter aid, and then filtering, precipitating the filtrate with alcohol and drying to obtain a pure polysaccharide; or enzymatically hydrolyzing the fermentation broth with glucanase, then

filtering, concentrating by ultrafiltration, and spray drying to obtain the pure polysaccharide.

5. A crude L-β-galactoglucan polysaccharide, wherein it is prepared as follows:

(1) Inoculating *Agrobacterium* sp. FN01 into a culture broth for fermentation to obtain a fermentation broth;
(2) Precipitating the fermentation broth with alcohol and oven-drying to obtain a crude polysaccharide.

6. Use of the L-β-galactoglucan, the *Agrobacterium* sp. FN01 fermentation broth containing the L-β-galactoglucan, or the crude L-β-galactoglucan polysaccharide in the manufacture of one of the following products:

(1) A product for treating and/or preventing the diseases related to glucose metabolic disorders;
(2) A product with the function of promoting skin repair;
(3) A product for enhancing non-specific immune function;
(4) A product for treating and/or preventing liver injury;
(5) An anti-fatigue product;
(6) A product for regulating the intestinal flora;
(7) An anti-aging product;
(8) A product with at least one of the functions of hydrating and moisturizing, increasing skin elasticity, or whitening and freckle removal;
(9) A product for improving the health status of a domestic animal; and
(10) A product for inhibiting *Malassezia* spp.

7. The use according to claim 6, wherein the product for treating and/or preventing diseases related to glucose metabolic disorders is a product for protecting pancreatic β cells and/or decreasing blood glucose.

8. The use according to claim 6, wherein the product for enhancing non-specific immune function that achieve the said efficacy by increasing the number of immune cells and/or regulating the function of immune cells.

9. The use according to claim 6, wherein the product for enhancing non-specific immune function that achieve the said efficacy by increasing the number of macrophages, neutrophils, and lymphocytes.

10. The use according to claim 6, wherein the liver injury comprises alcoholic liver injury and non-alcoholic liver injury.

11. The use according to claim 6, wherein the product for treating and/or preventing liver injury is a product for reducing liver transaminase activity and protecting liver tissue.

12. The use according to claim 6, wherein the anti-fatigue product refers to a product with at least one of the functions of clearing ROS, promoting ATP synthesis, and promoting lactic acid metabolism.

13. The use according to claim 6, wherein the product for regulating the intestinal flora is a product for promoting the proliferation of intestinal probiotic bacteria and promoting intestinal health.

14. The use according to claim 6, wherein the anti-aging refers to inhibiting β-galactosidase activity and/or increasing telomerase activity.

15. The use according to claim 6, wherein the increasing skin elasticity refers to upregulating the relative expression levels of *col1a1a* and *col1a1b* genes.

16. The use according to claim 6, wherein the domestic animals are selected from livestock and pets; the livestock comprises pigs, cattle, sheep; and the pets comprise dogs and cats.

17. The use according to claim 16, wherein the improving the health status of livestock refers to one or more functions of promoting growth, promoting body immunity, improving intestinal microflora, and reducing diarrhea; and the improving the health status of pets refers to one or more functions of reducing obesity, improving palatability, increasing relative abundance of intestinal probiotic bacteria, improving stool shape, and improving hair quality.

18. The use according to claim 17, wherein the dosage of the L-β-galactoglucan in improving the health status of the livestock is 400-600 grams/ton of regular feed; and the addition percentage of the L-β -galactoglucan in the daily ration

for improving the health status of pets is 0.25-0.5%.

**19.** The use according to claim 6, wherein the promoting skin repair comprises at least one of the functions of promoting tissue regeneration, wound healing, and apoptotic cell clearance.

**20.** The use according to claim 6, wherein the whitening and freckle removal comprises inhibiting melanin production.

**21.** The use according to claim 6, wherein the inhibiting *Malassezia* spp. refers to inhibiting the expression of inflammatory factors induced by *Malassezia* spp.

**22.** The use according to claim 6, wherein the product for inhibiting *Malassezia* spp. is a product for treating skin infection caused by *Malassezia* spp.

**23.** The use according to claim 22, wherein the product for inhibiting *Malassezia* spp. is a product for treating a scalp infection induced by *Malassezia* spp.

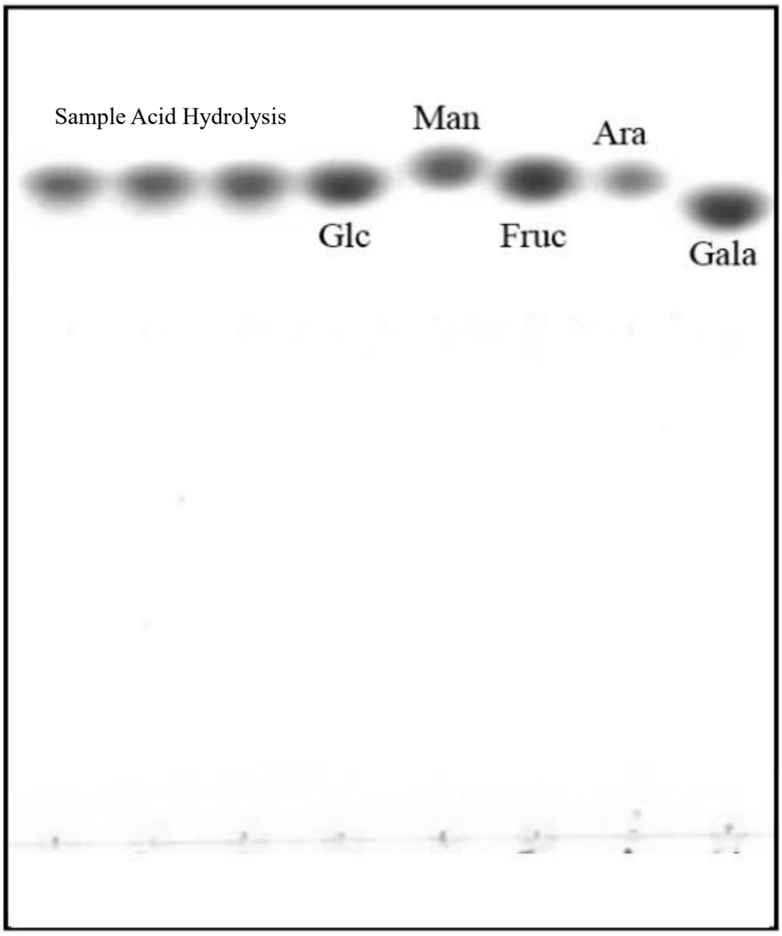


Figure 1

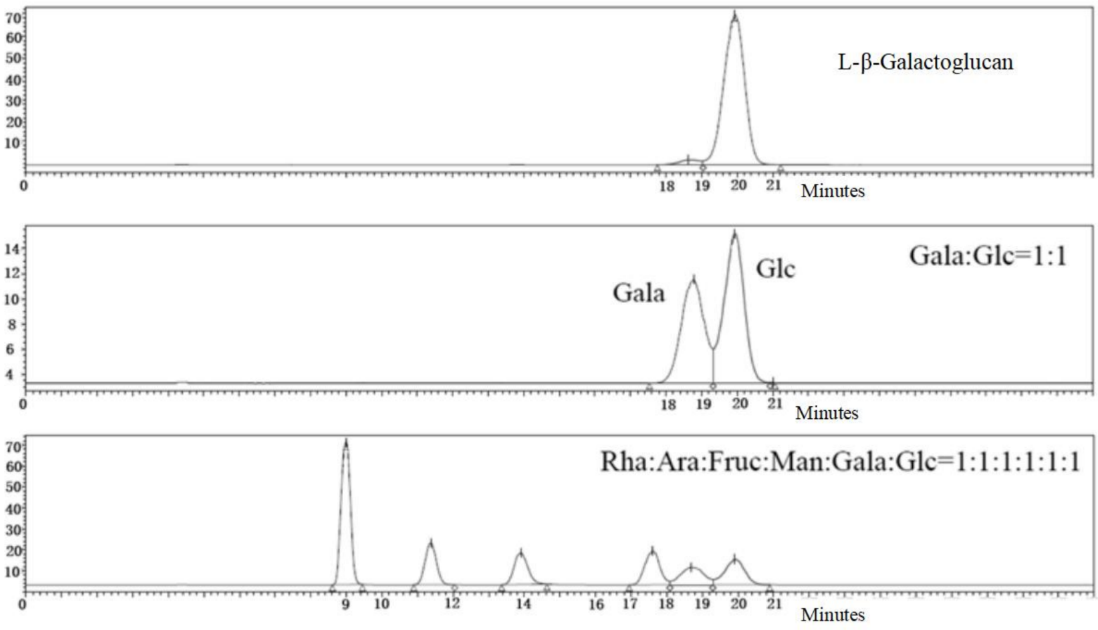


Glc: Glucose; Man: Mannose; Fruc: Fructose; Ara: Arabinose; Gala: Galactose; Rha: Rhamnose

Figure 2

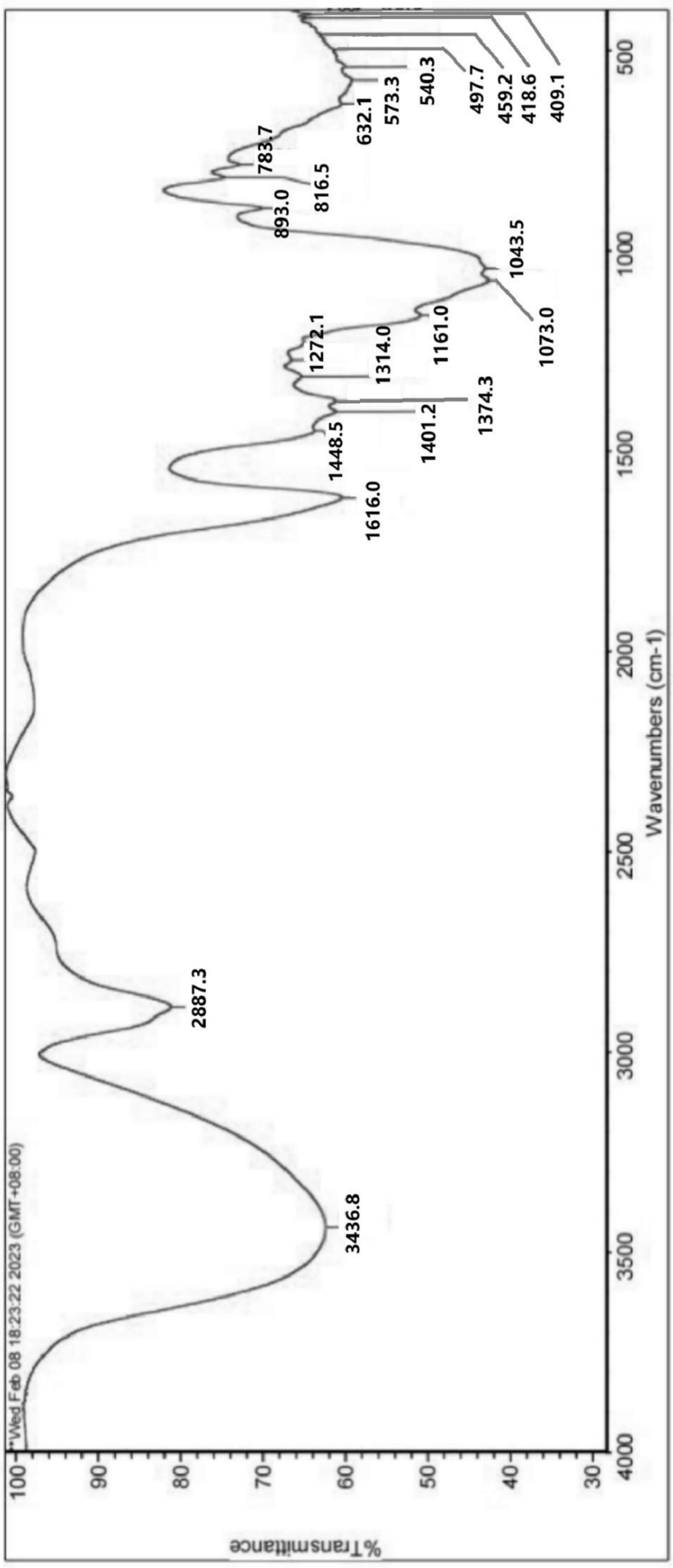
*Wed Feb 08 18:23:22 2023 (GMT+08:00)
%Transmittance
Wavenumbers (cm-1)
100
90
80
70
60
50
40
30
4000
3500
3000
2500
2000
1500
1000
500
3436.8
2887.3
1616.0
1448.5
1401.2
1374.3
1314.0
1272.1
1161.0
1073.0
1043.5
893.0
816.5
783.7
632.1
573.3
540.3
497.7
459.2
418.6
409.1

Figure 3

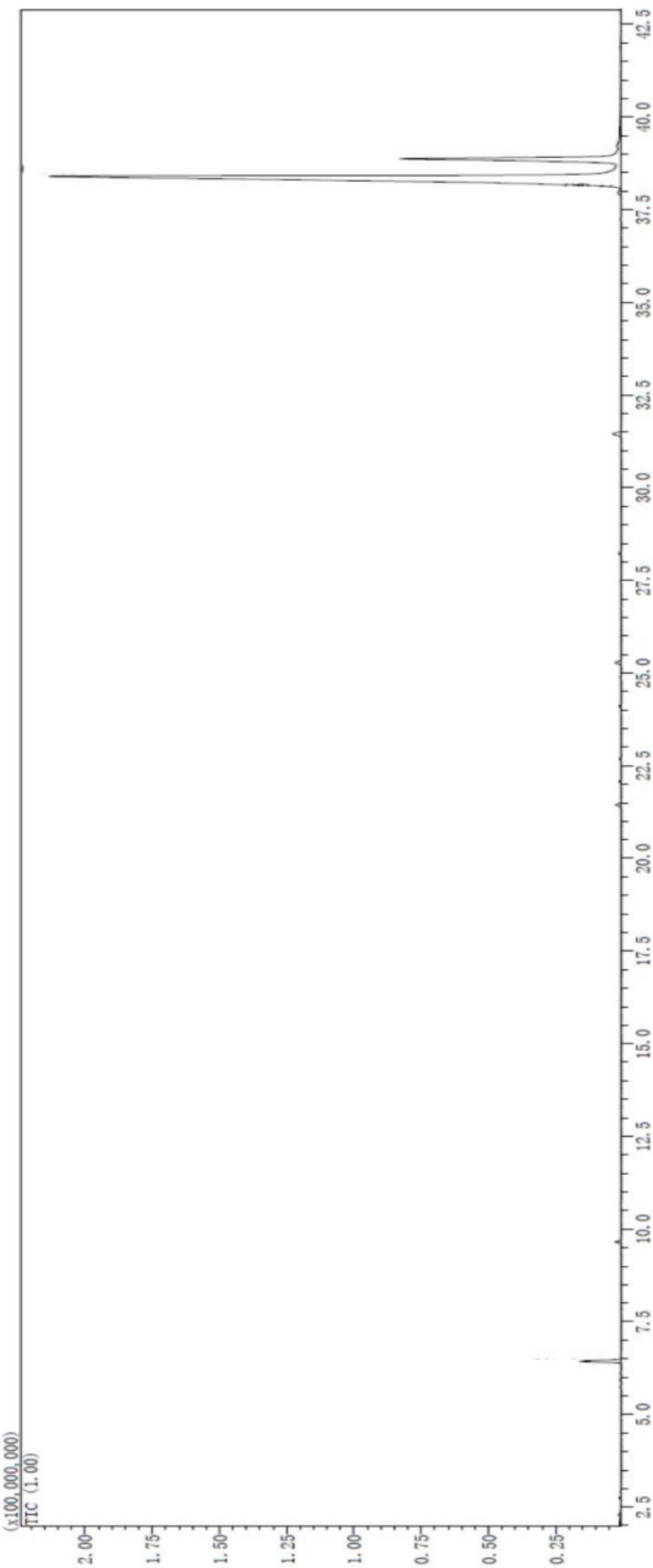
(x100,000,000)
TIC (1.00)
2.00
1.75
1.50
1.25
1.00
0.75
0.50
0.25
2.5
5.0
7.5
10.0
12.5
15.0
17.5
20.0
22.5
25.0
27.5
30.0
32.5
35.0
37.5
40.0
42.5

Figure 4

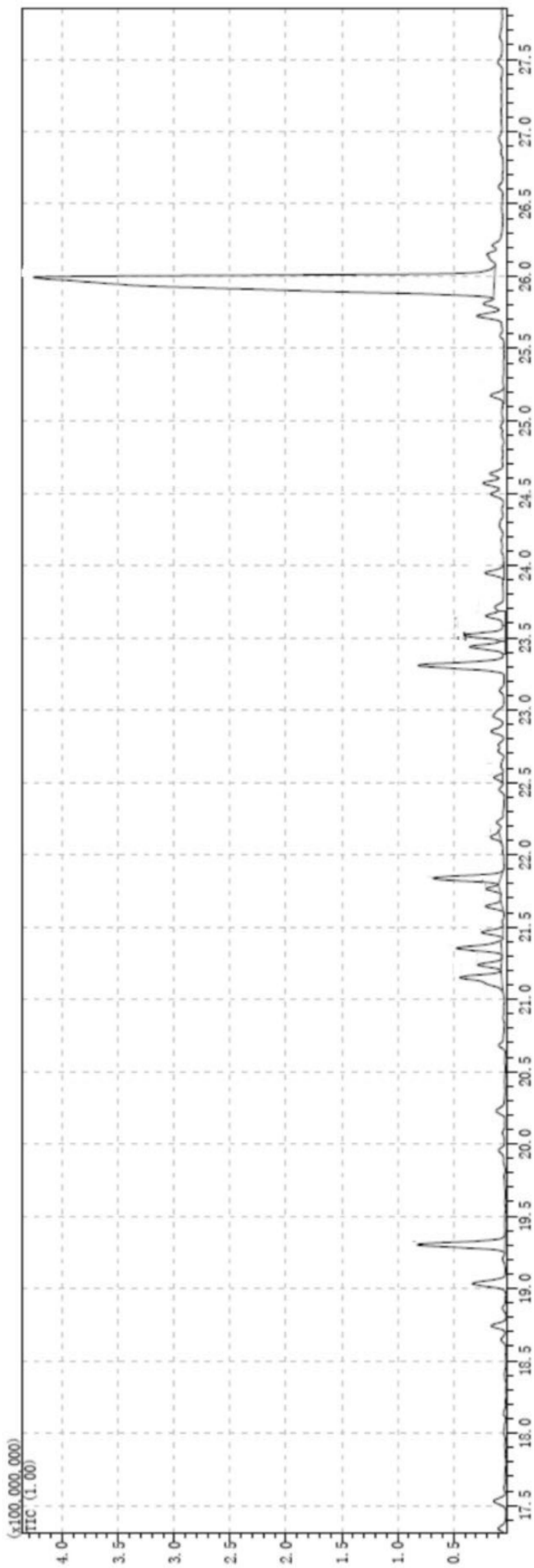
(x100,000,000)
TIC (1.00)
4.0
3.5
3.0
2.5
2.0
1.5
1.0
0.5
17.5
18.0
18.5
19.0
19.5
20.0
20.5
21.0
21.5
22.0
22.5
23.0
23.5
24.0
24.5
25.0
25.5
26.0
26.5
27.0
27.5

Figure 5

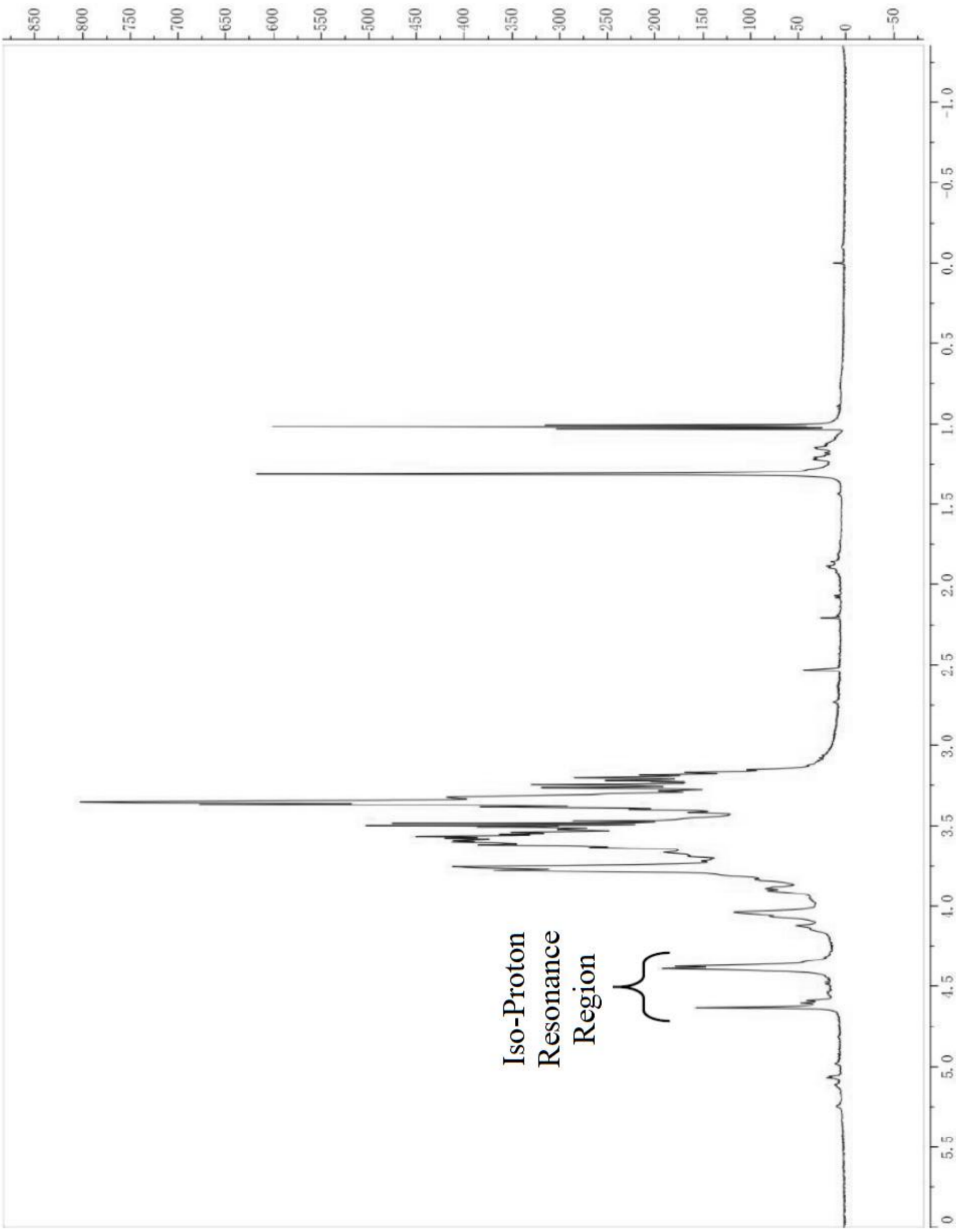


Figure 6

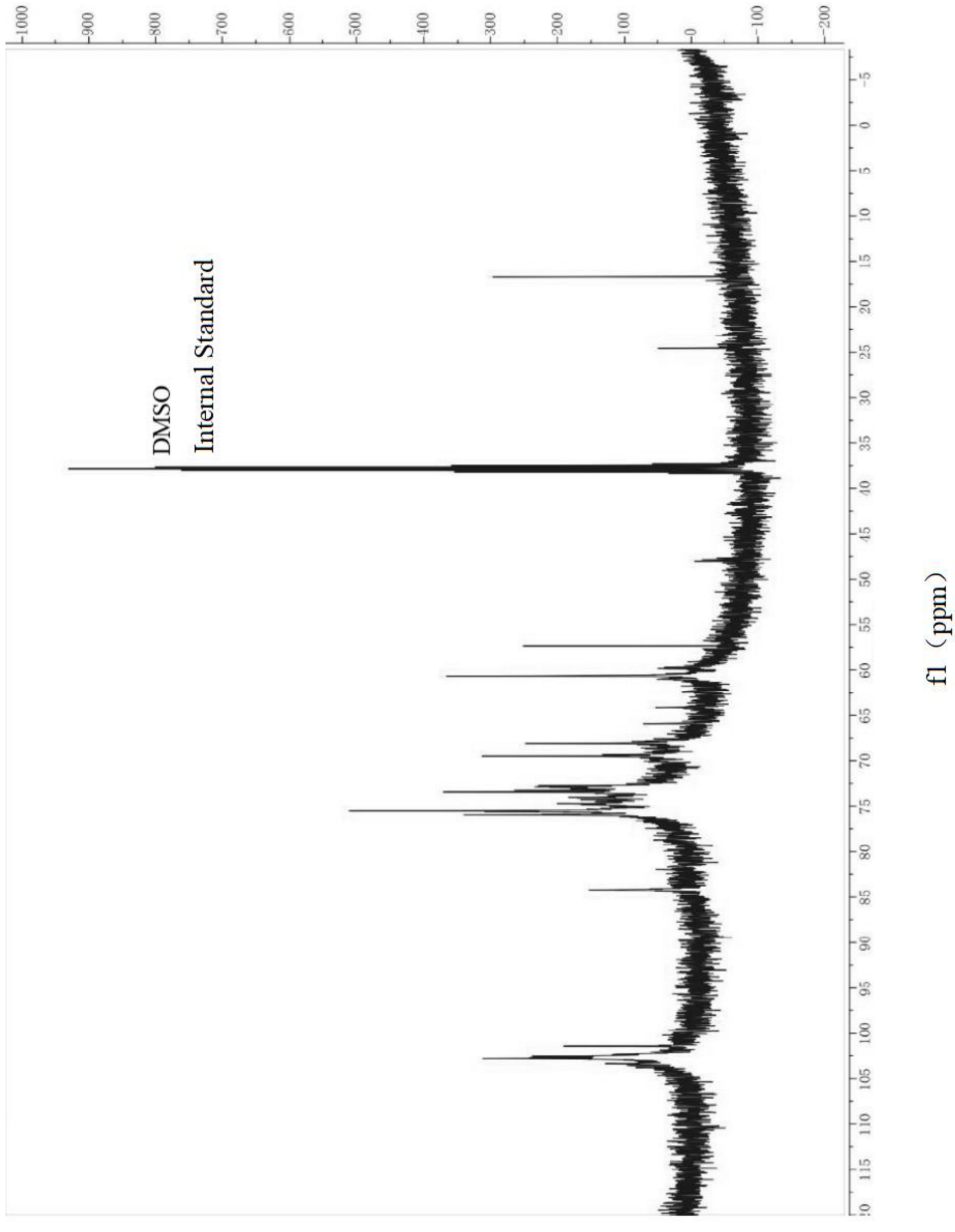


Figure 7

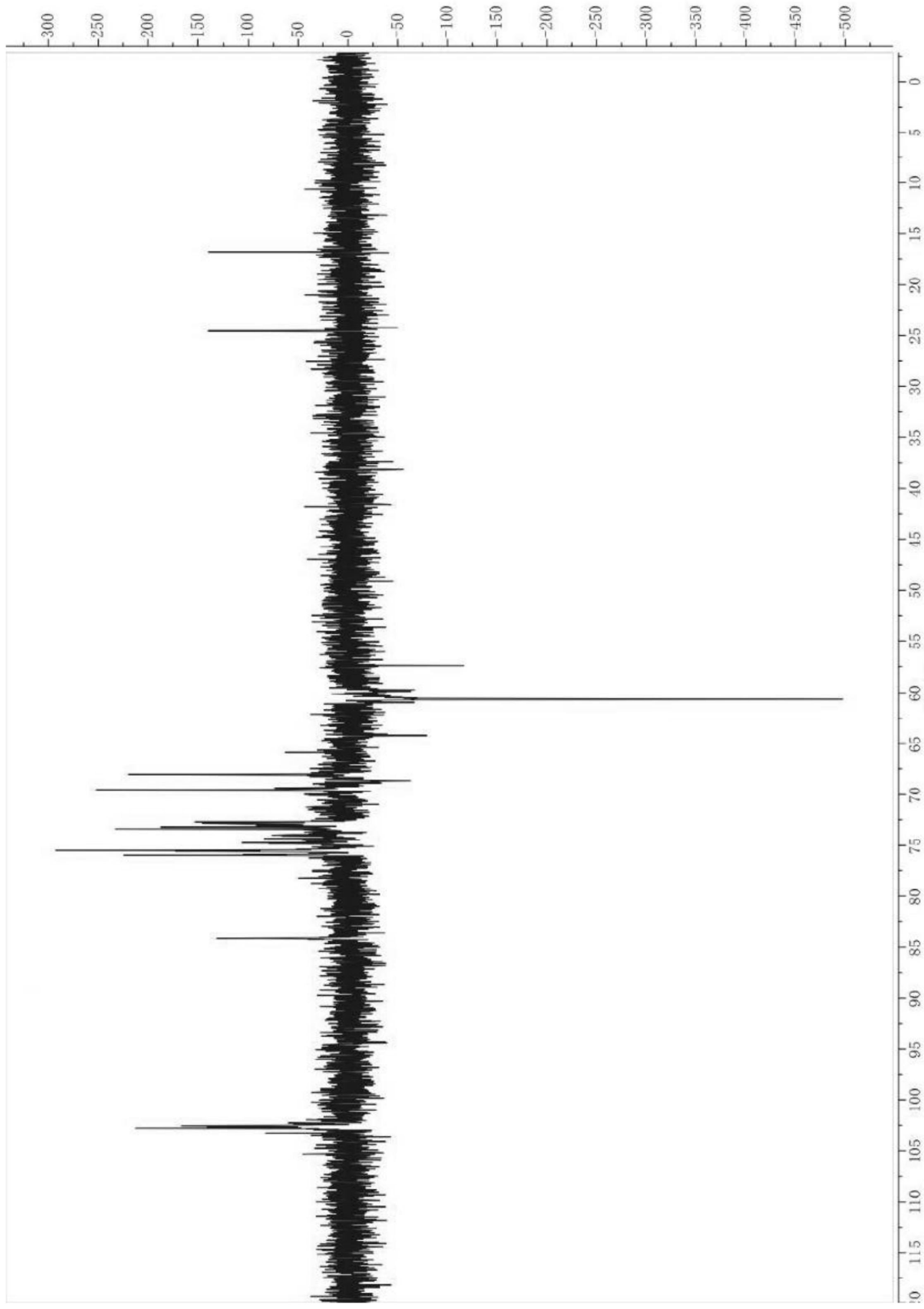


Figure 8

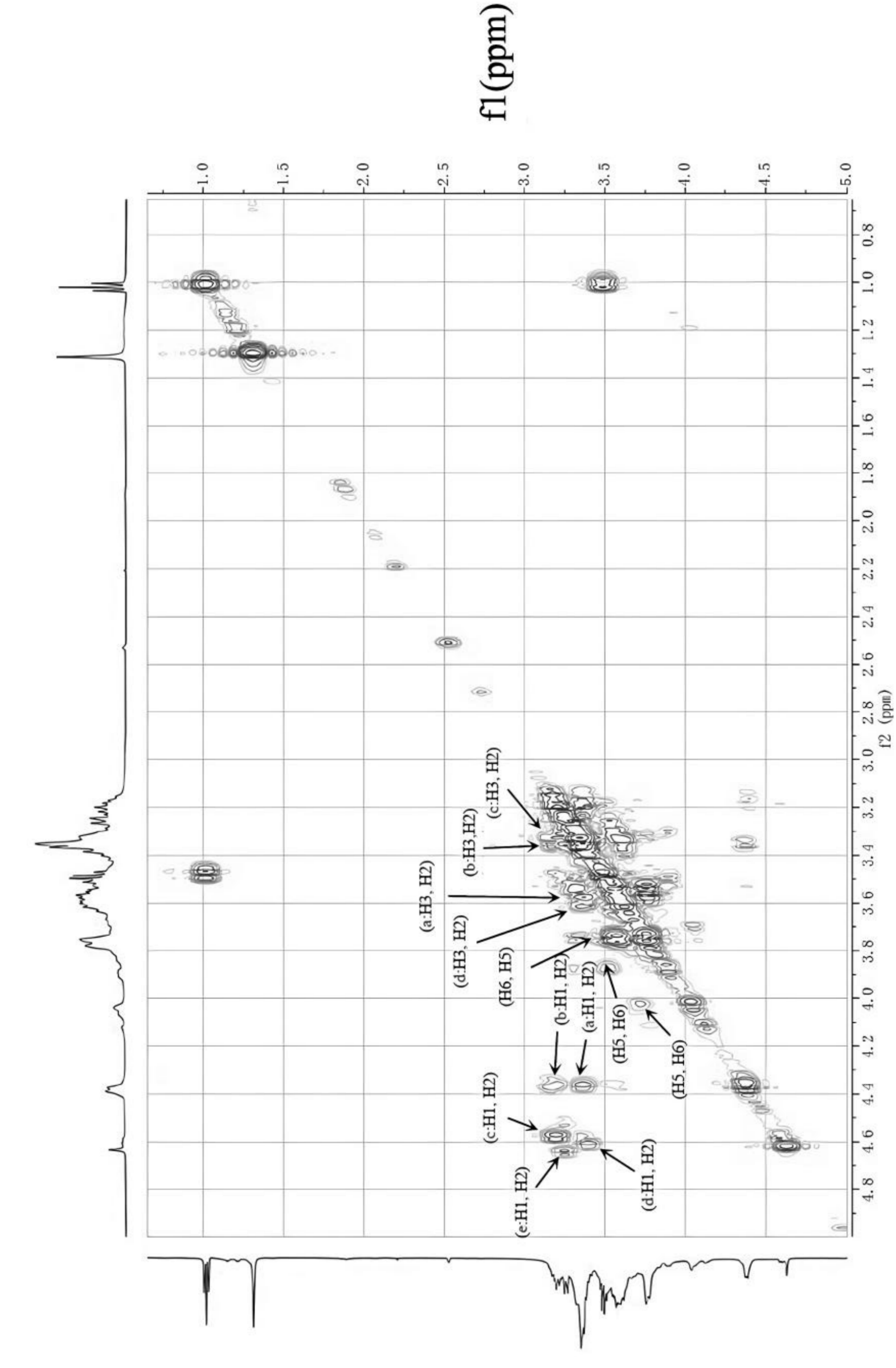
f1(ppm)
f2 (ppm)
(c:H3, H2)
(b:H3,H2)
(a:H3, H2)
(d:H3, H2)
(H6, H5)
(b:H1, H2)
(a:H1, H2)
(H5, H6)
(H5, H6)
(c:H1, H2)
(e:H1, H2)
(d:H1, H2)

Figure 9

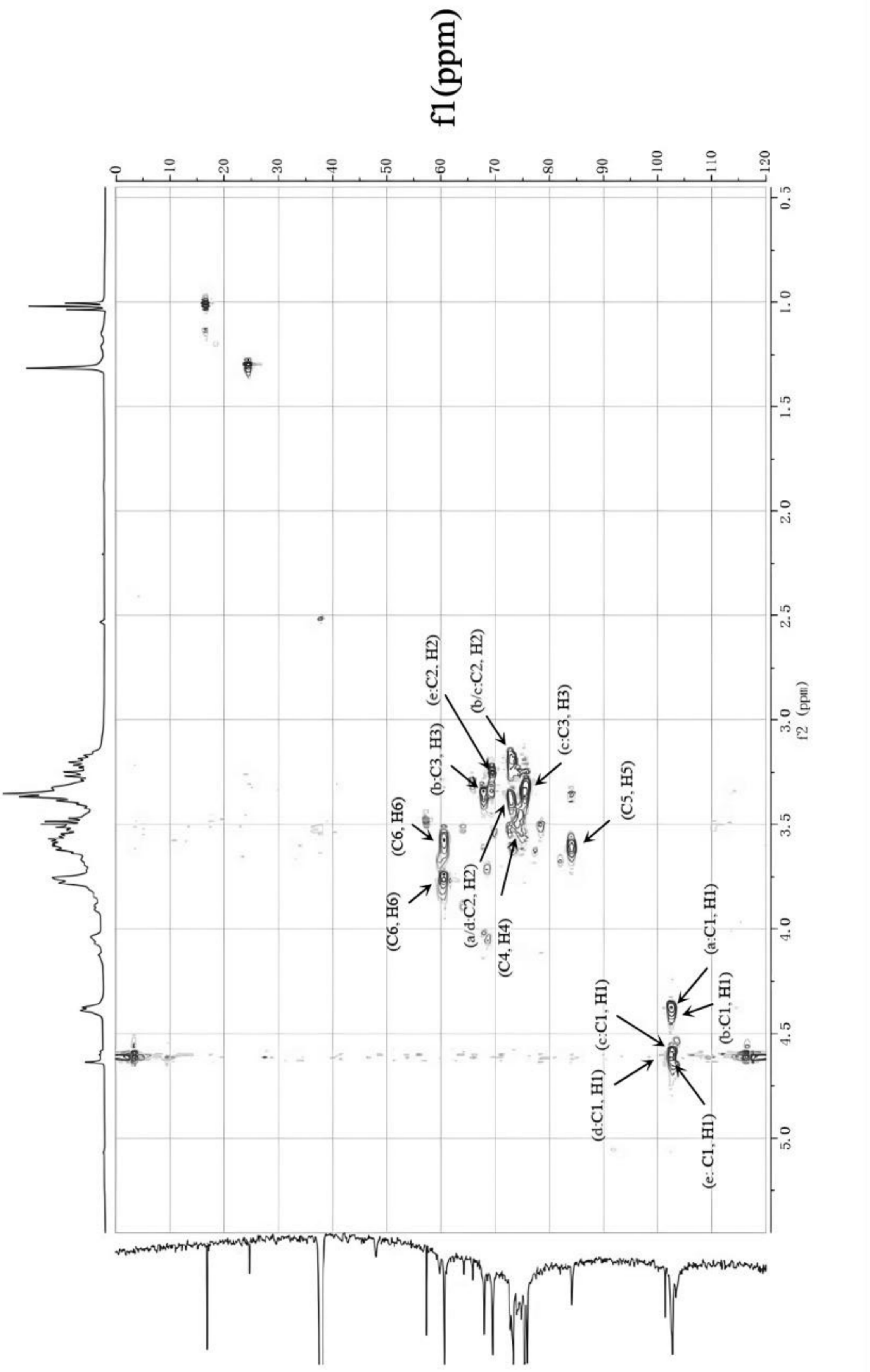
f1(ppm)
f2 (ppm)
(e:C2, H2)
(b/c:C2, H2)
(b:C3, H3)
(c:C3, H3)
(C5, H5)
(C6, H6)
(C6, H6)
(a/d:C2, H2)
(C4, H4)
(a:C1, H1)
(b:C1, H1)
(c:C1, H1)
(d:C1, H1)
(e: C1, H1)

Figure 10

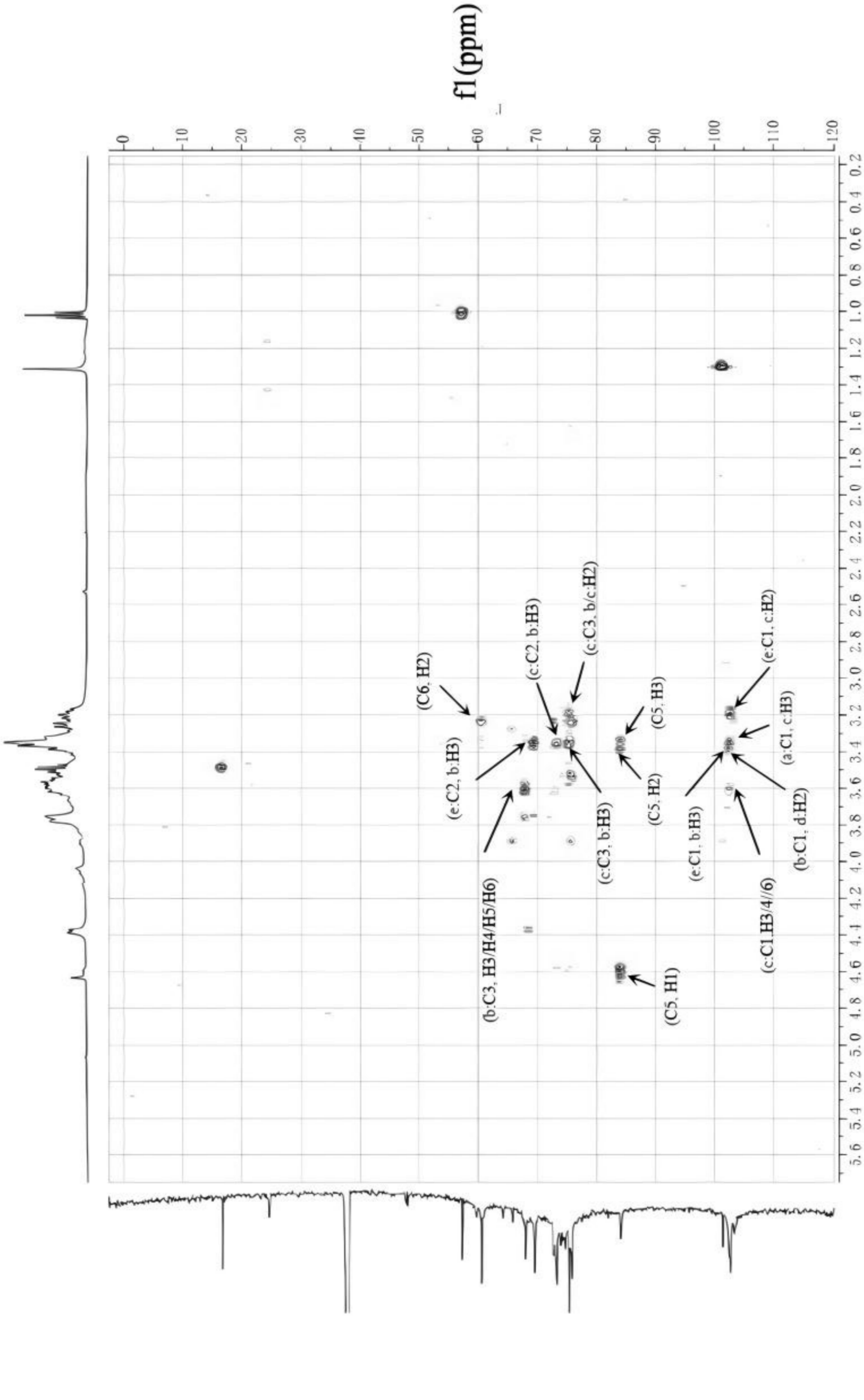
f1(ppm)
f2 (ppm)
(C6, H2)
(c:C2, b:H3)
(c:C3, b/c:H2)
(e:C1, c:H2)
(C5, H3)
(a:C1, c:H3)
(e:C2, b:H3)
(c:C3, b:H3)
(C5, H2)
(e:C1, b:H3)
(b:C1, d:H2)
(b:C3, H3/H4/H5/H6)
(c:C1,H3/4//6)
(C5, H1)

Figure 11

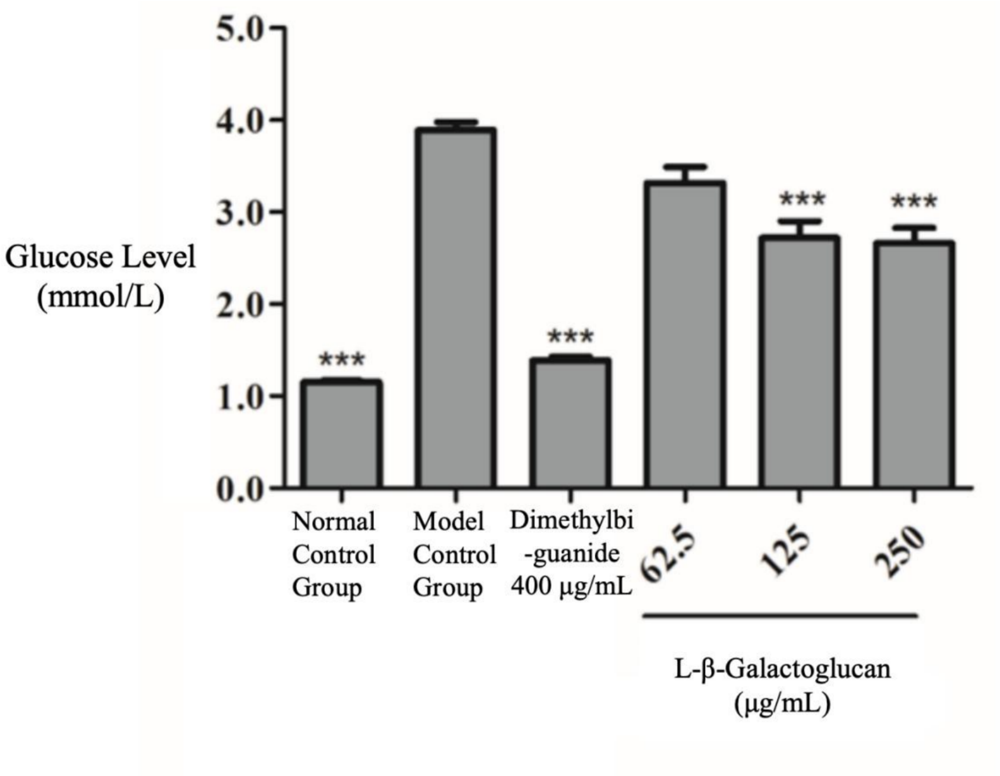


Figure 12

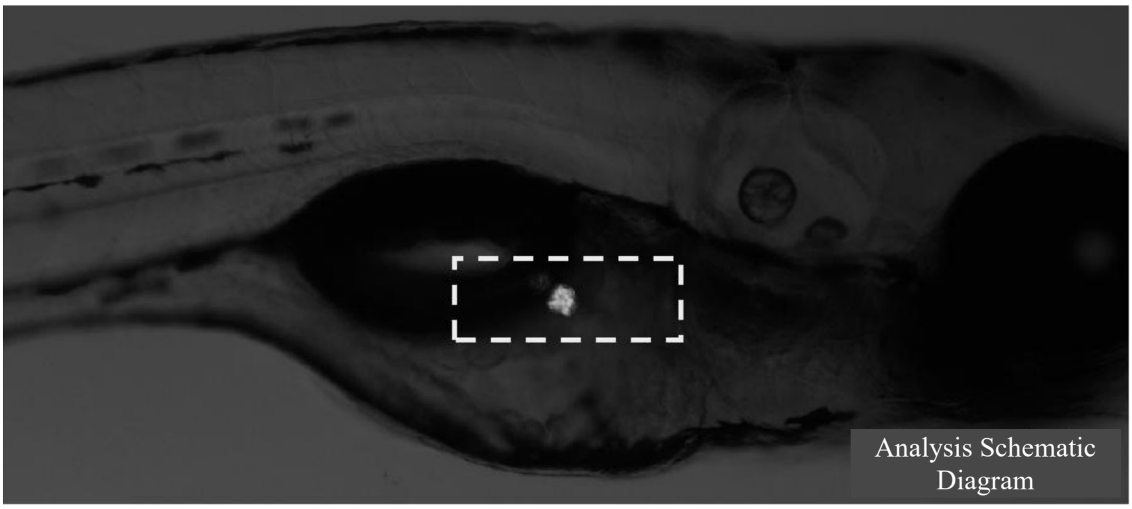


Figure 13

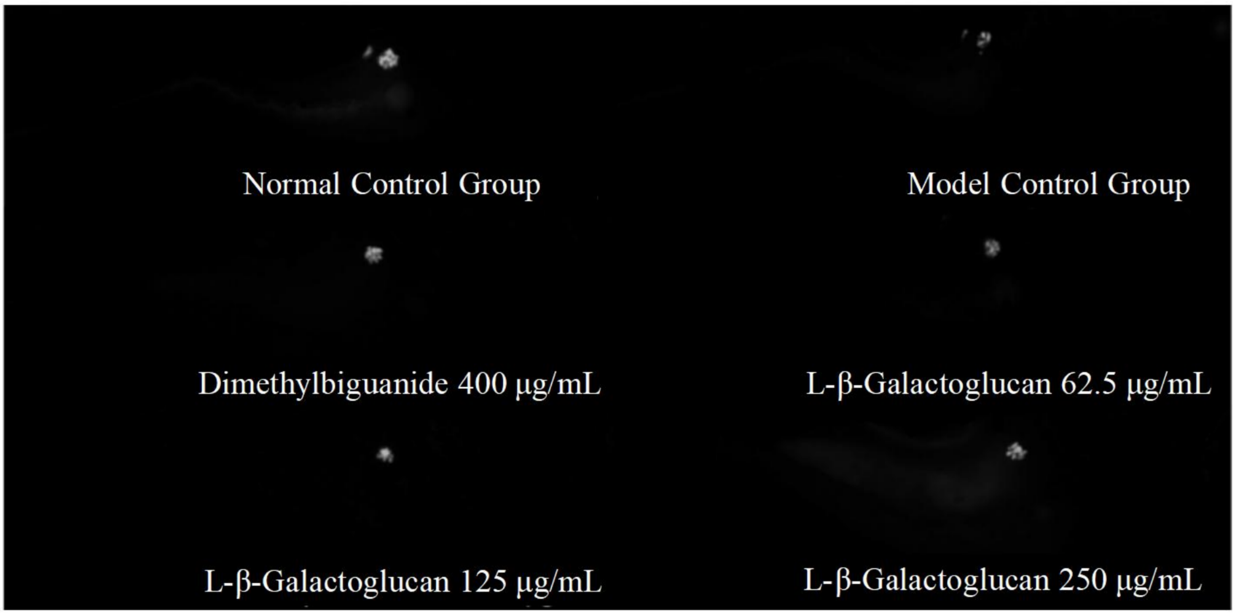


Figure 14

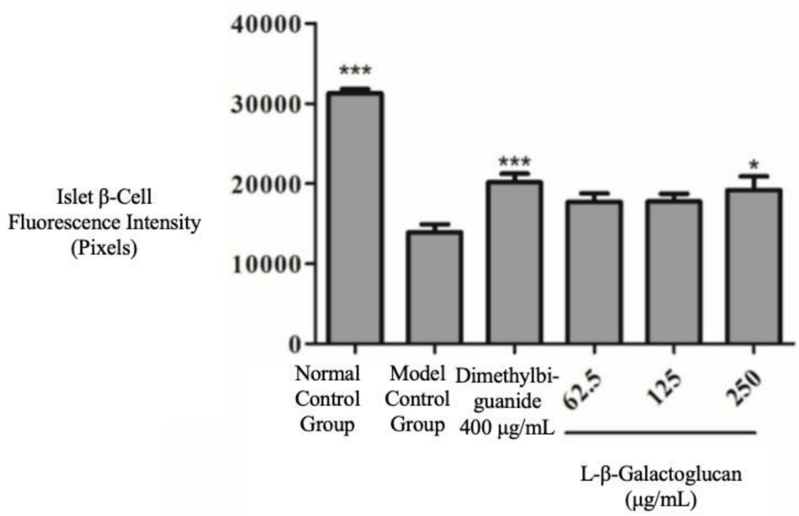


Figure 15

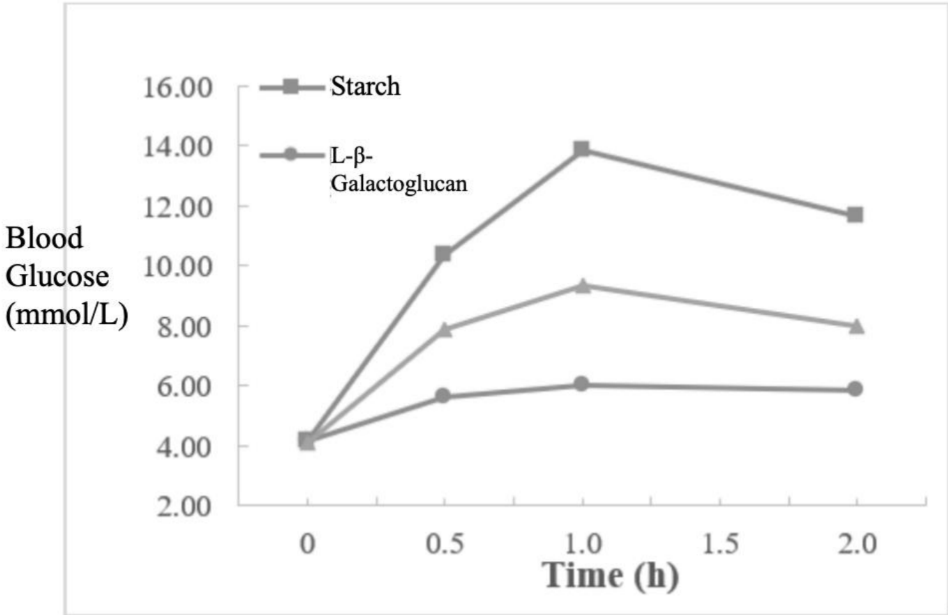


Figure 16

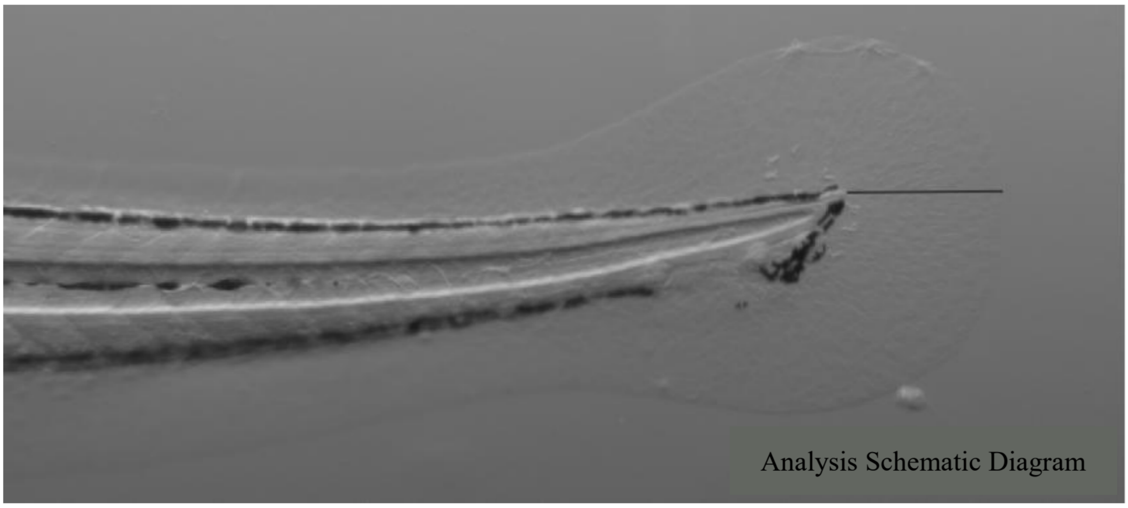


Figure 17

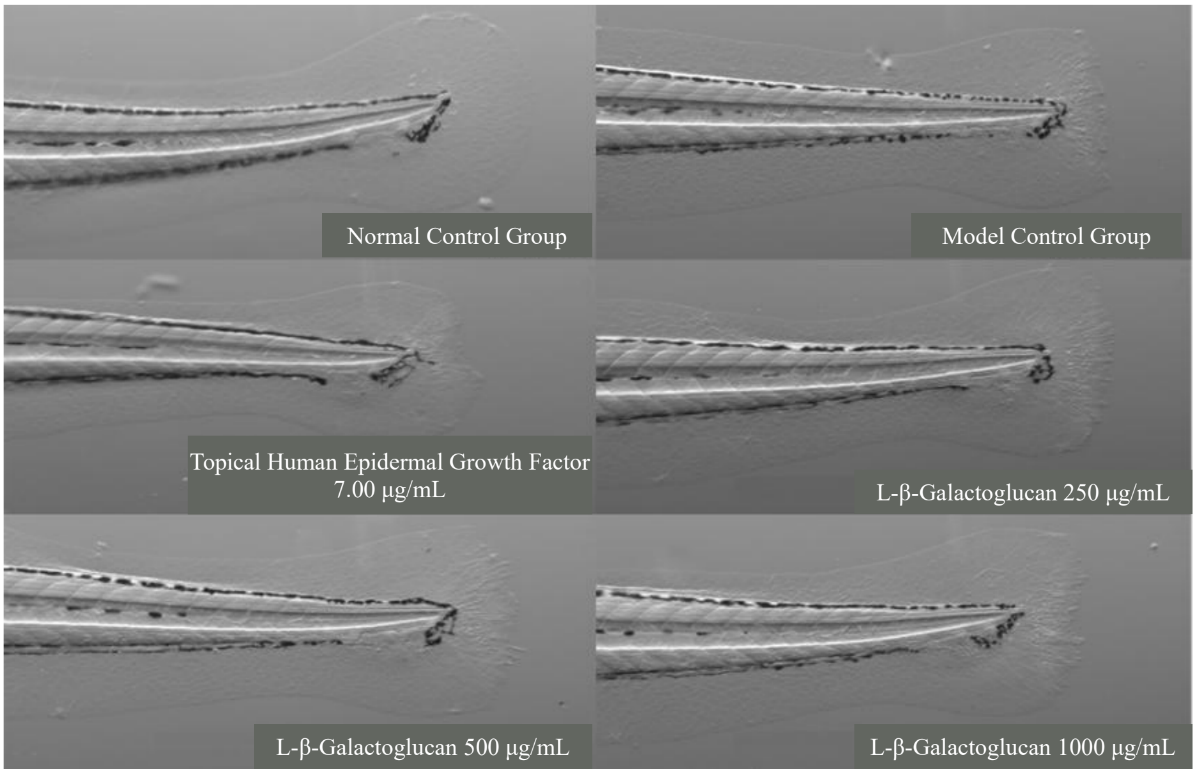


Figure 18

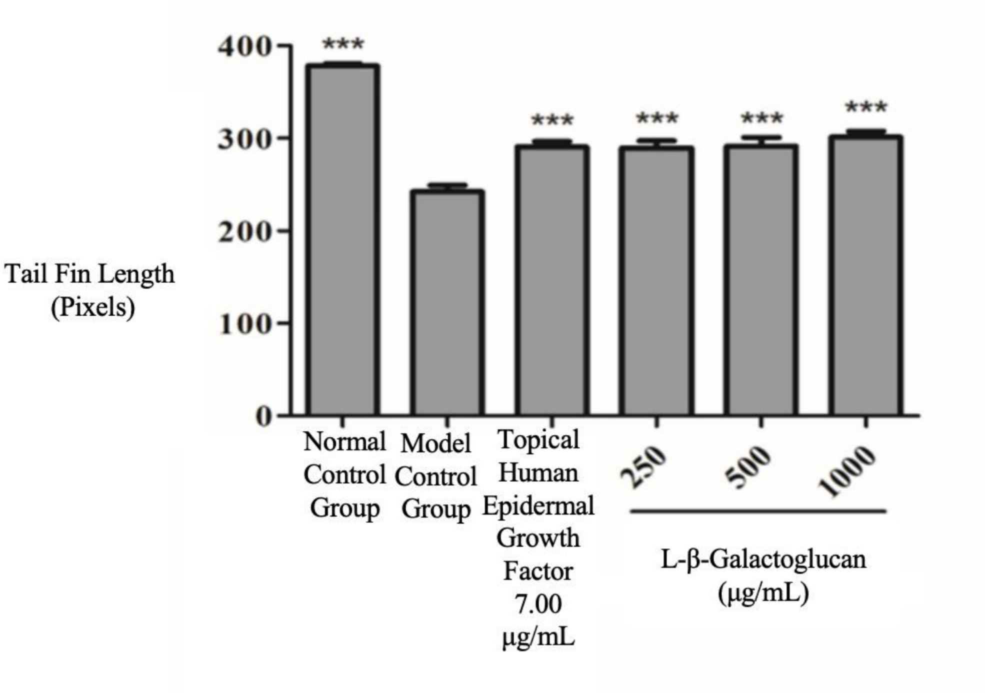


Figure 19

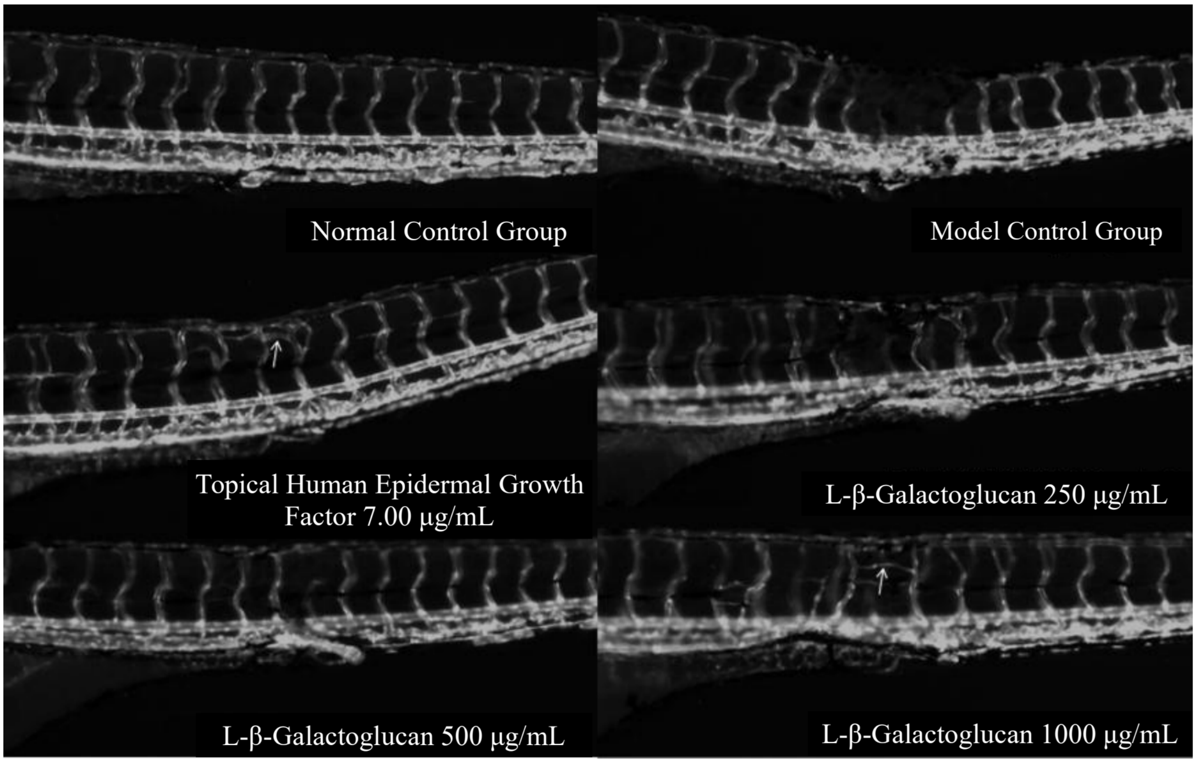


Figure 20

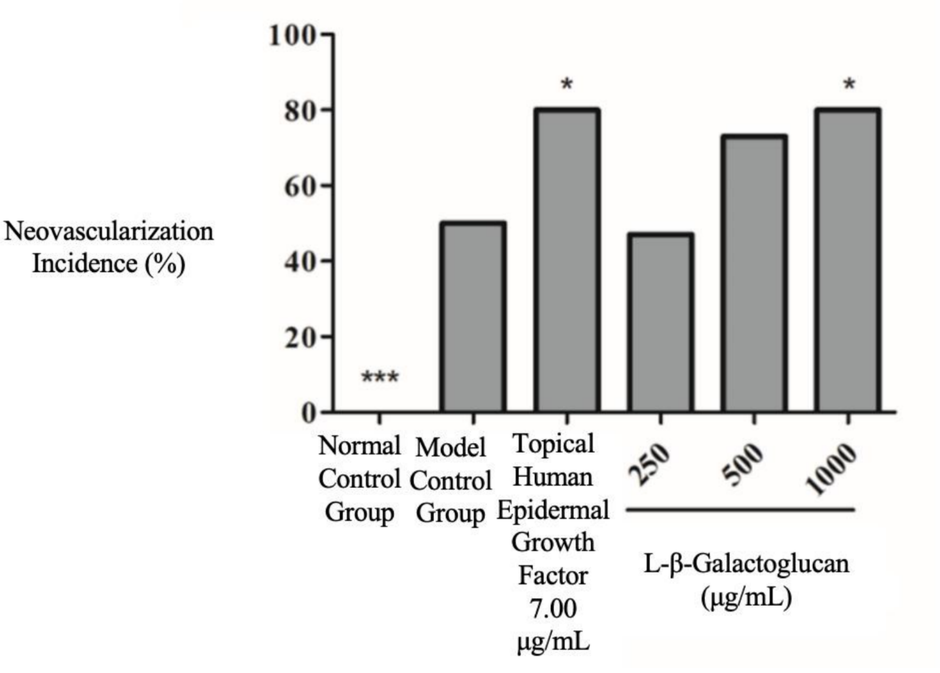


Figure 21

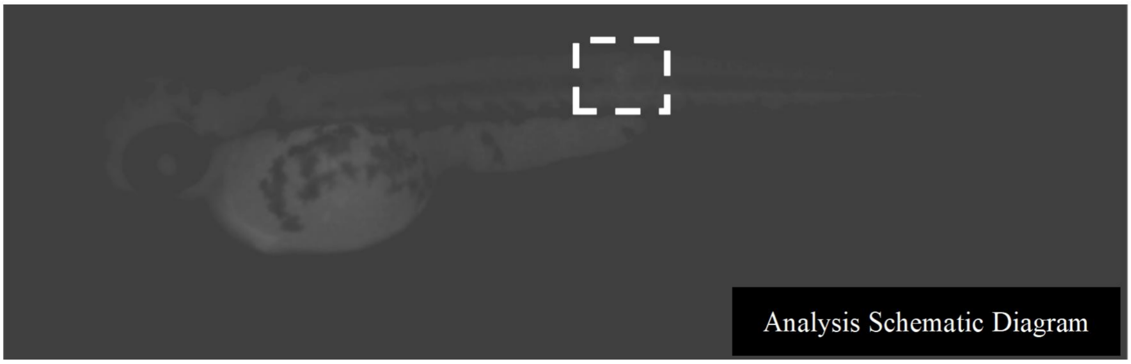


Figure 22

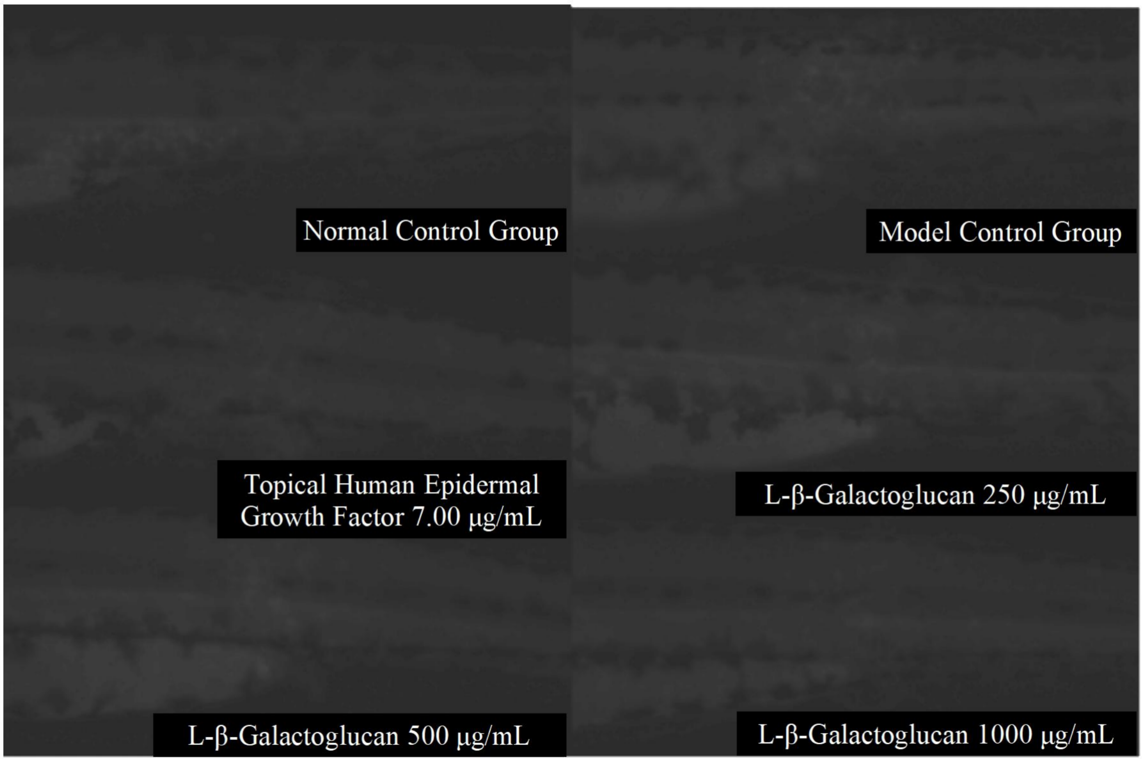


Figure 23

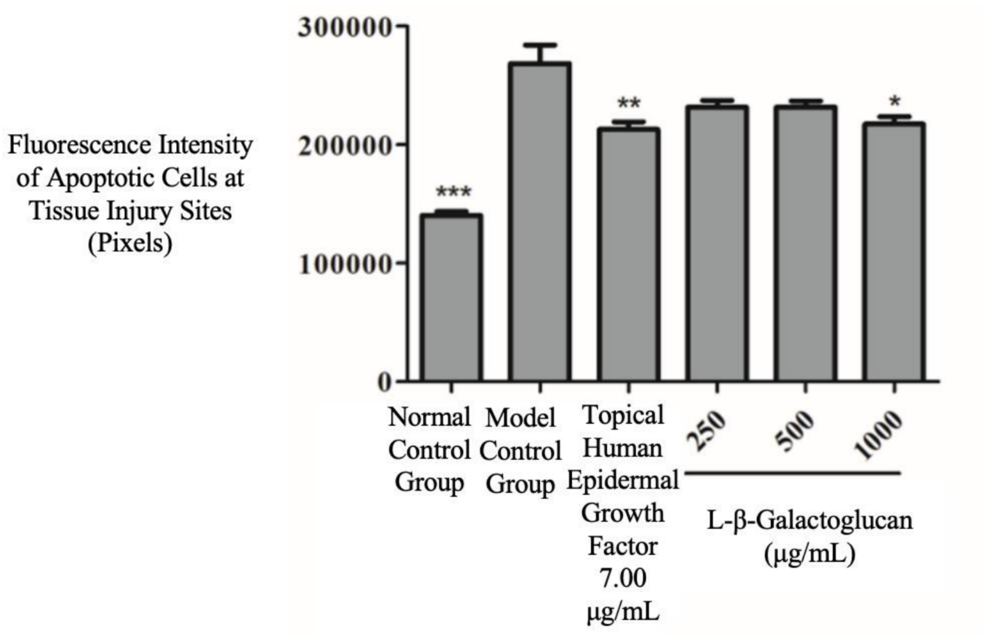


Figure 24

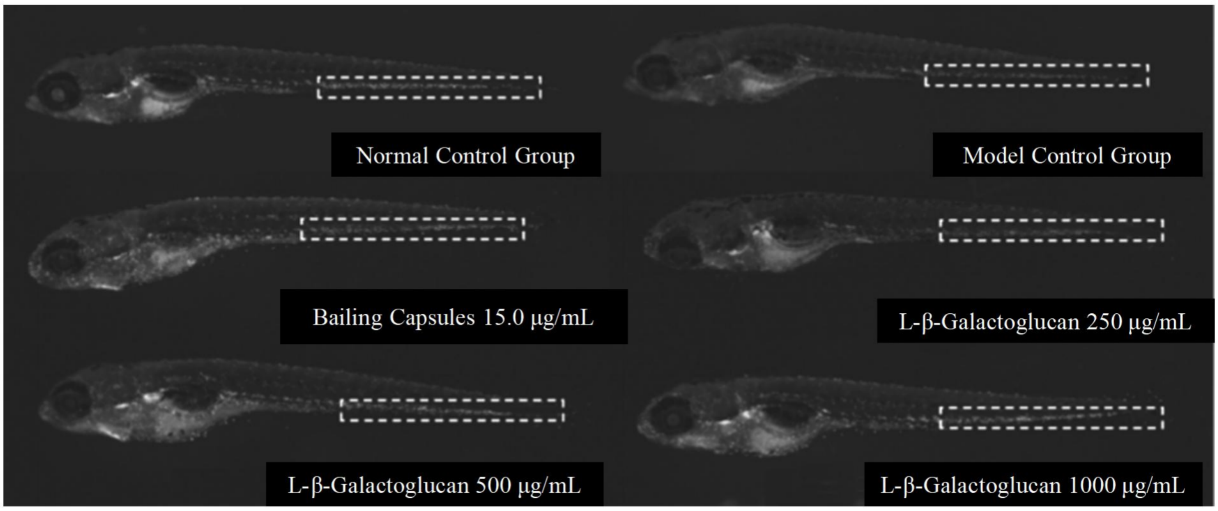


Figure 25

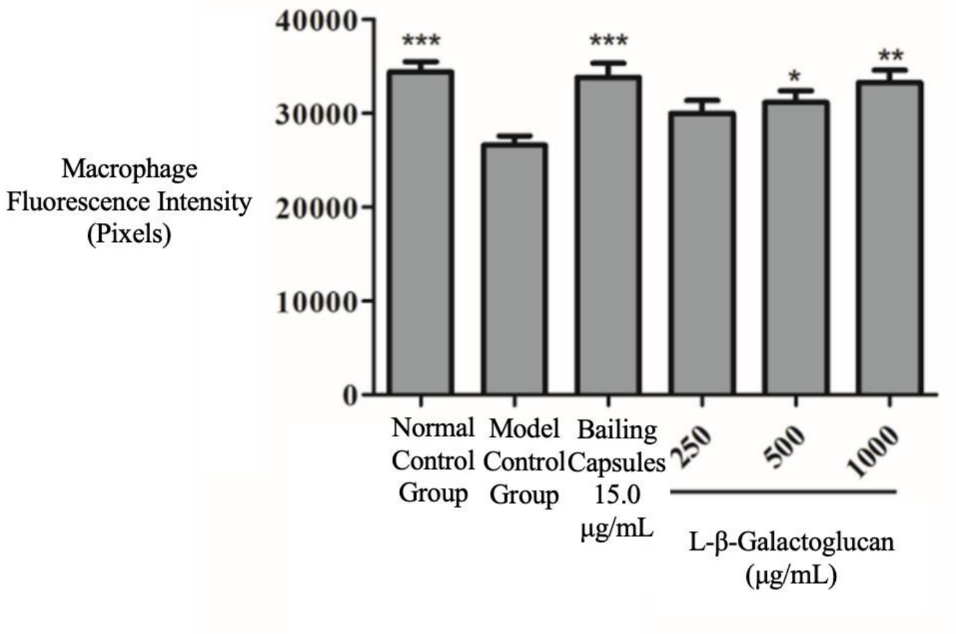


Figure 26

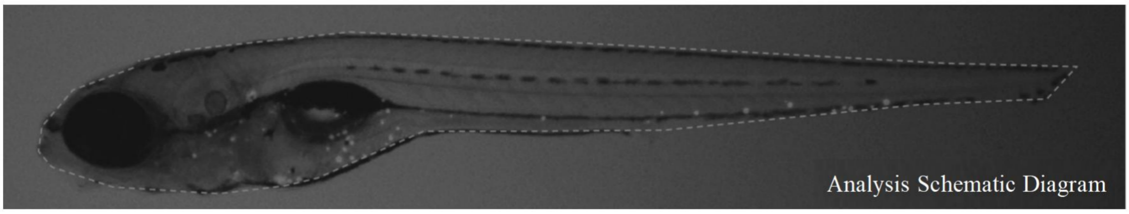


Figure 27

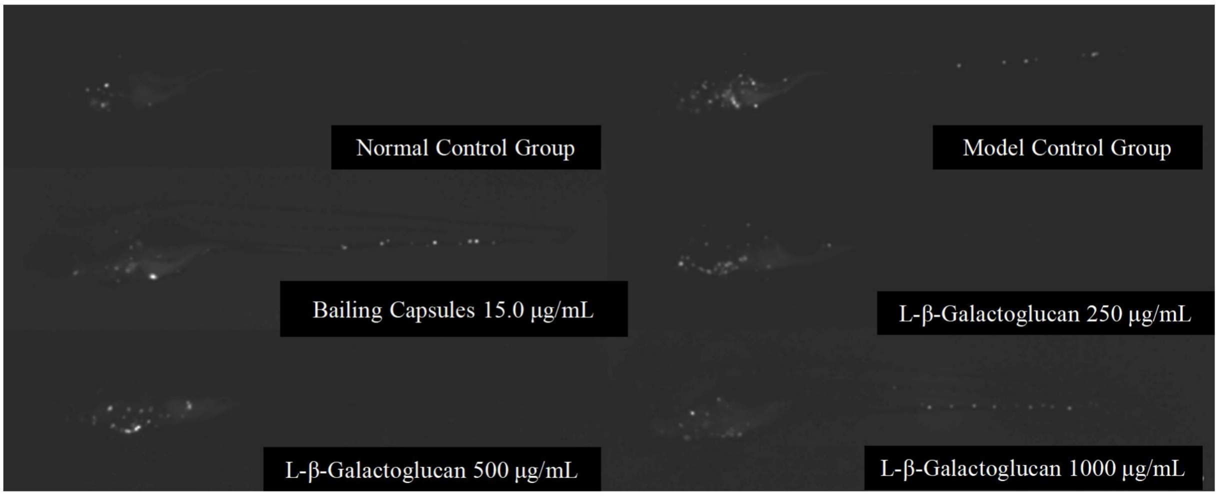


Figure 28

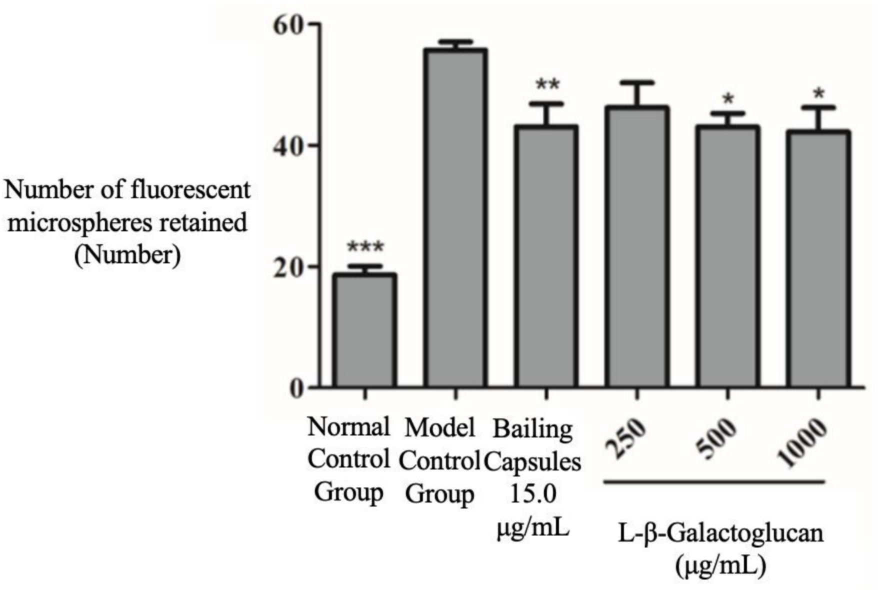


Figure 29

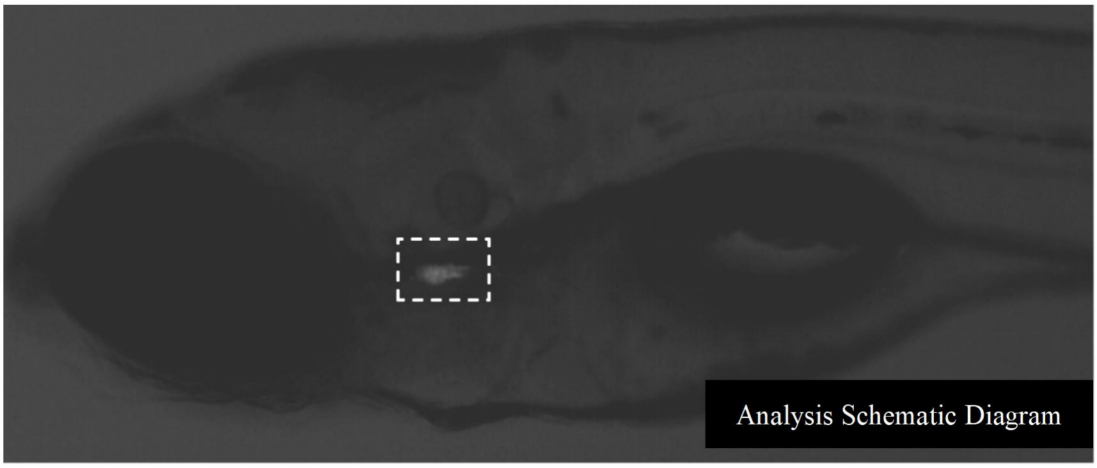


Figure 30

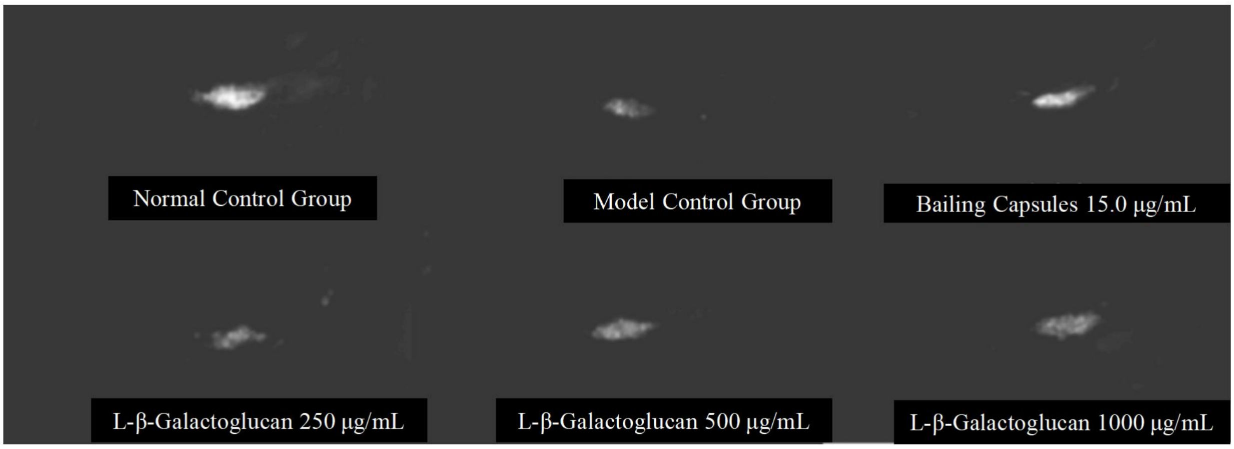


Figure 31

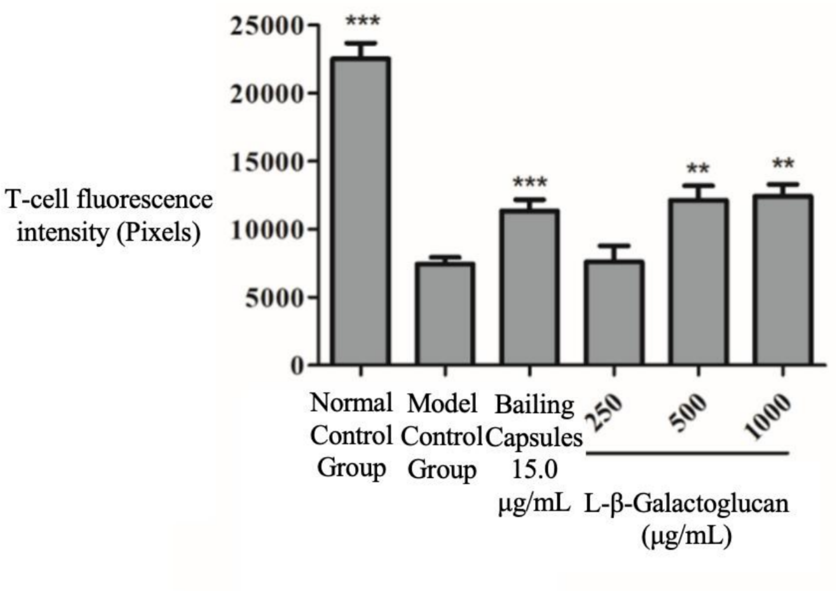


Figure 32

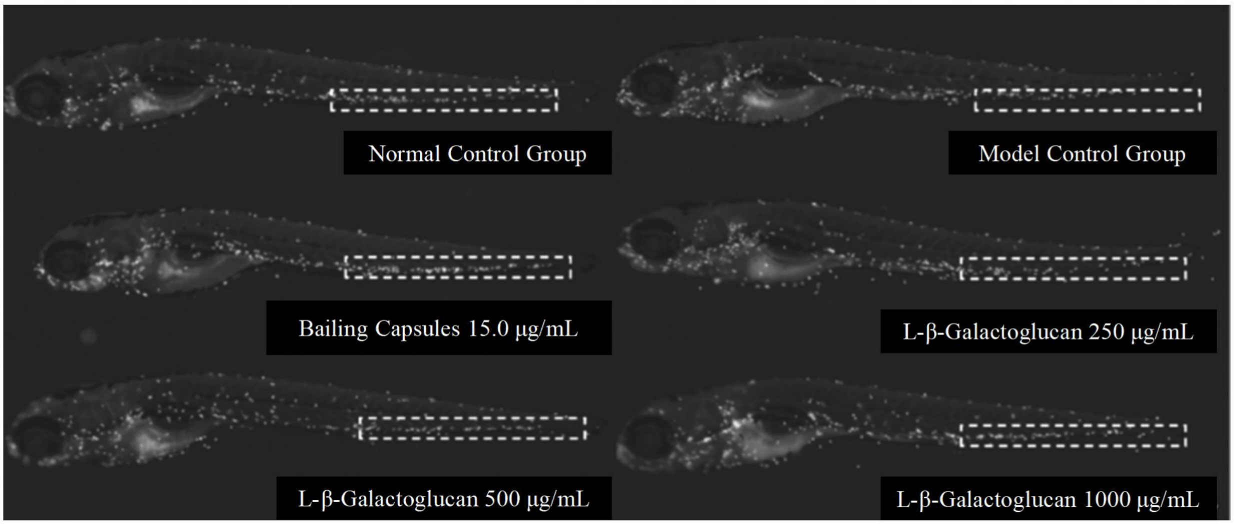


Figure 33

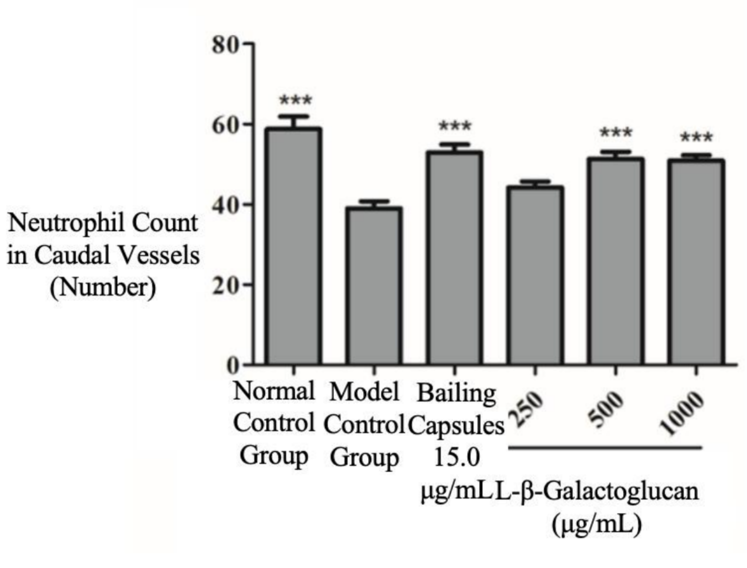


Figure 34

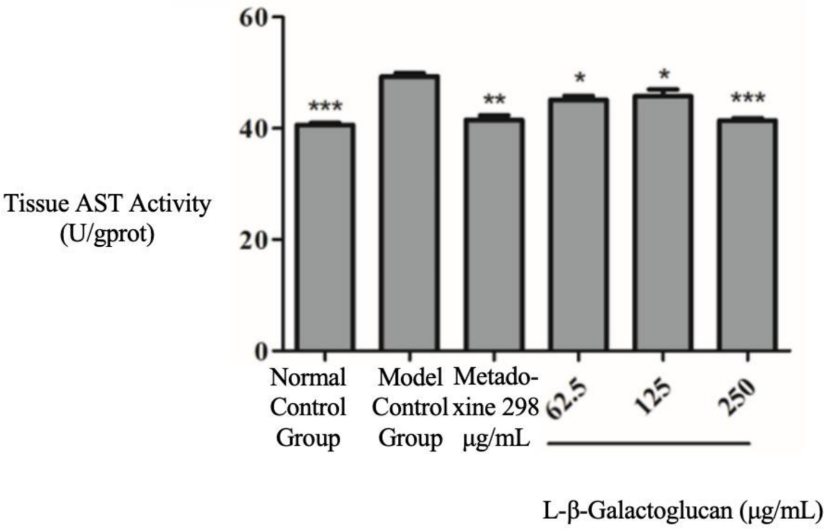


Figure 35

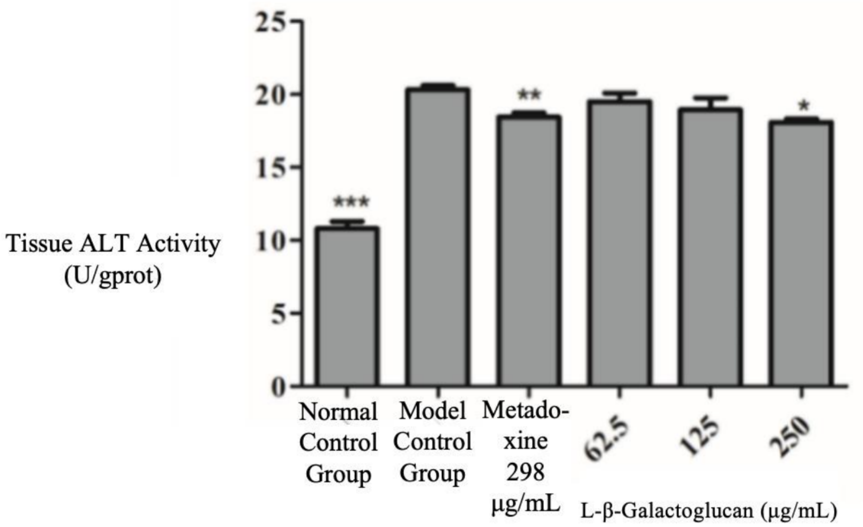


Figure 36

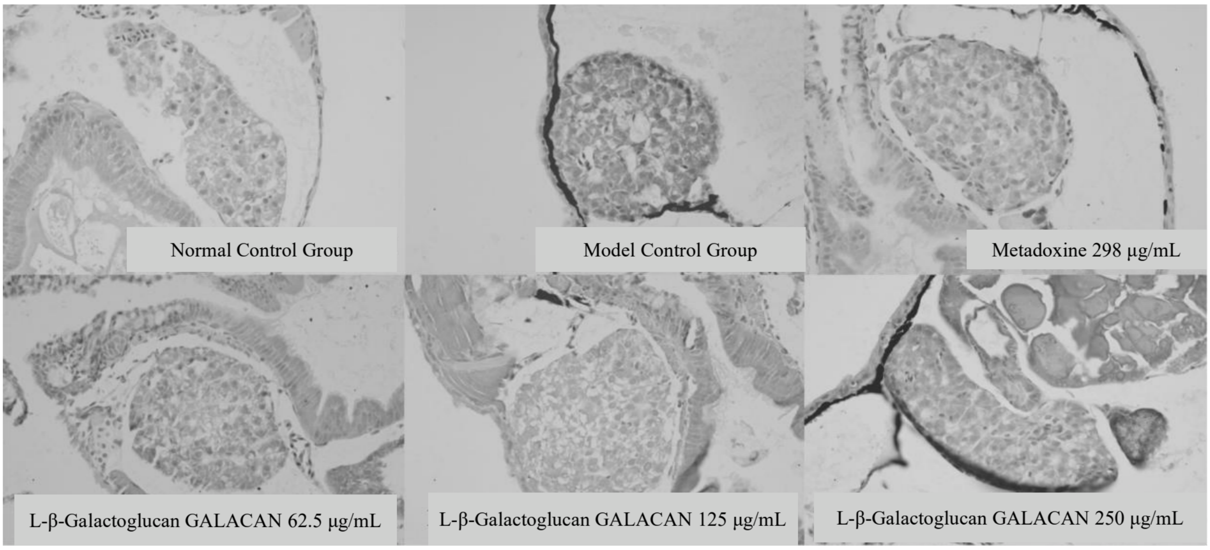


Figure 37

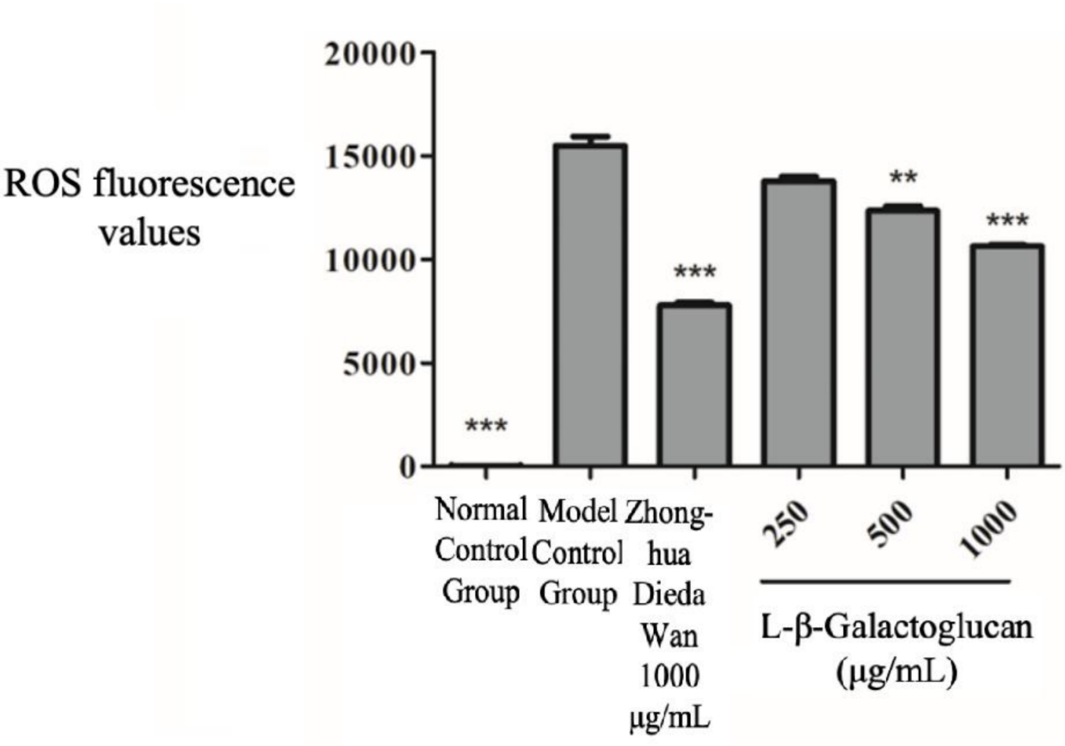


Figure 38

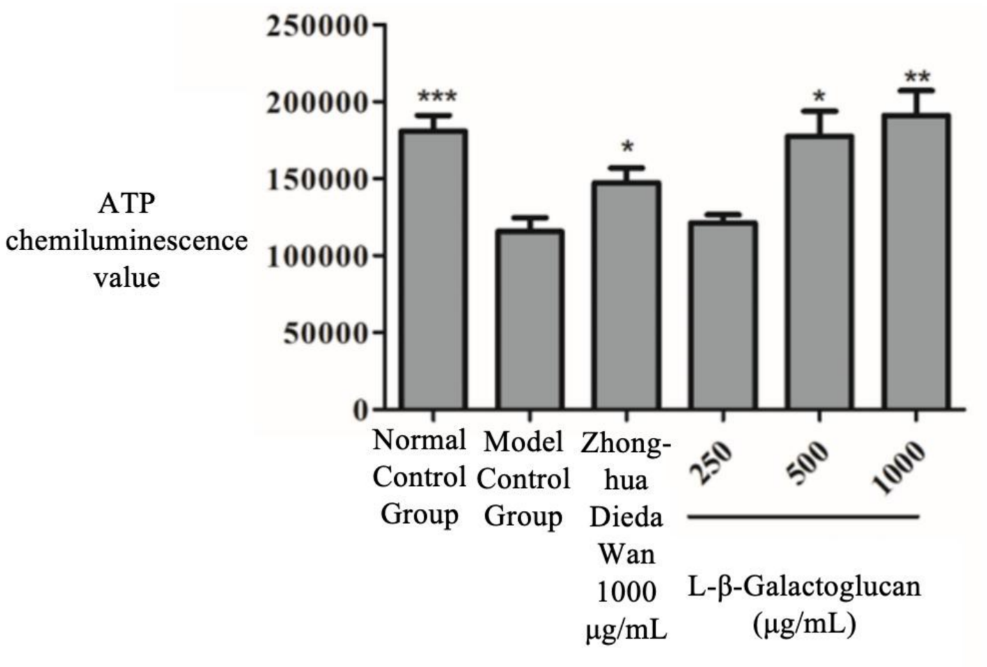


Figure 39

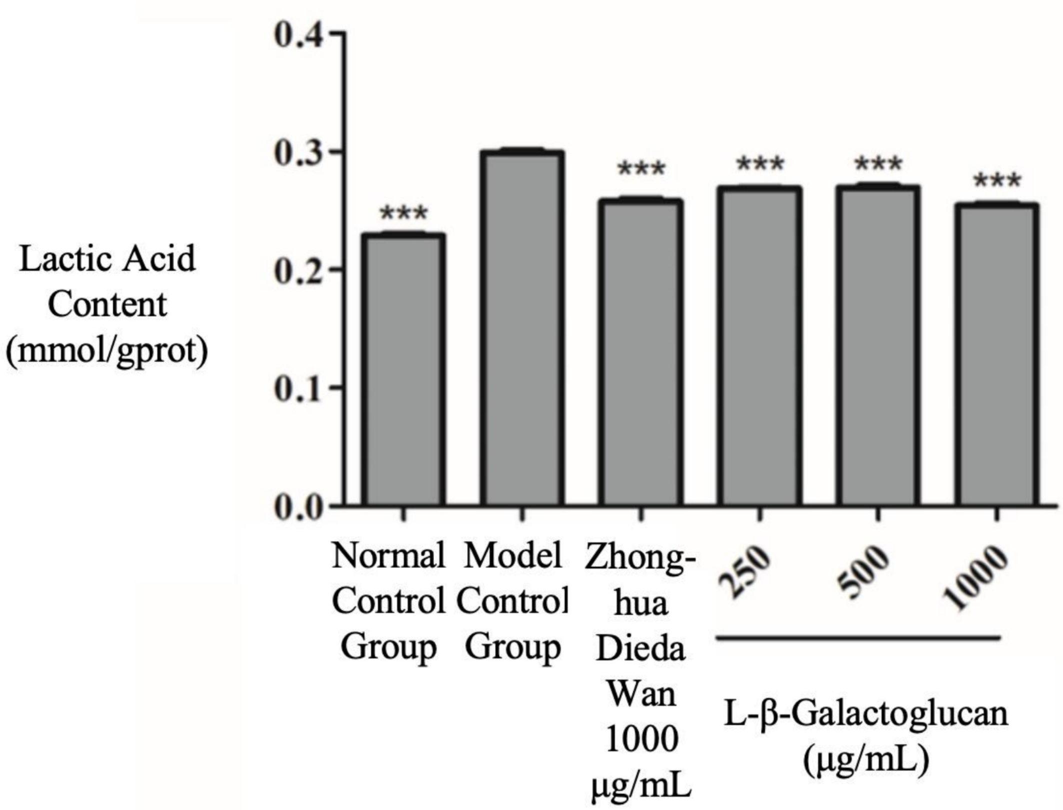


Figure 40

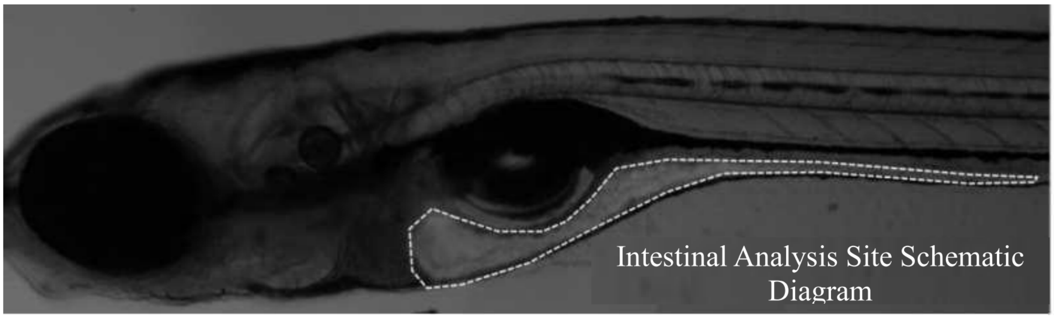


Figure 41

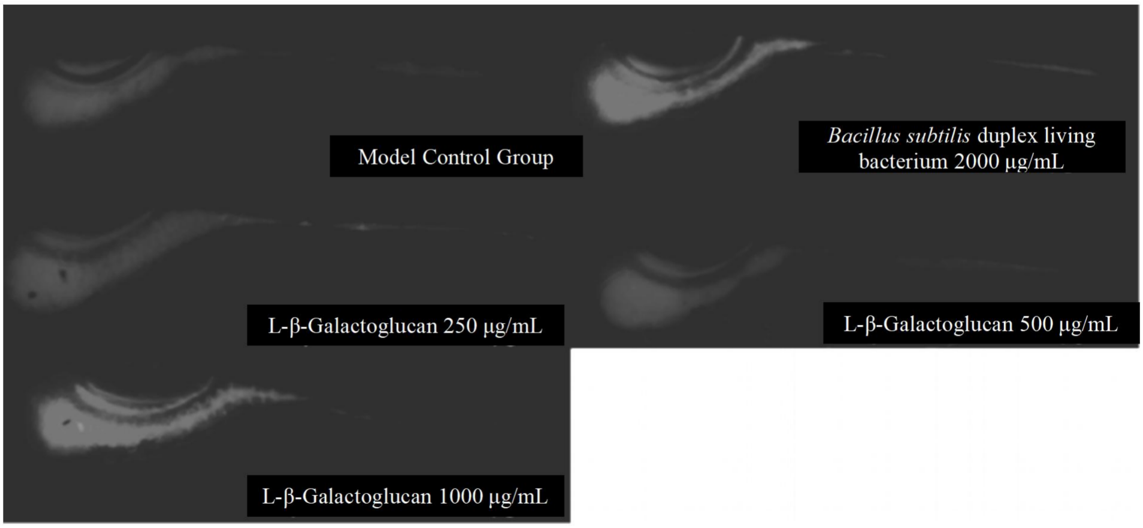


Figure 42

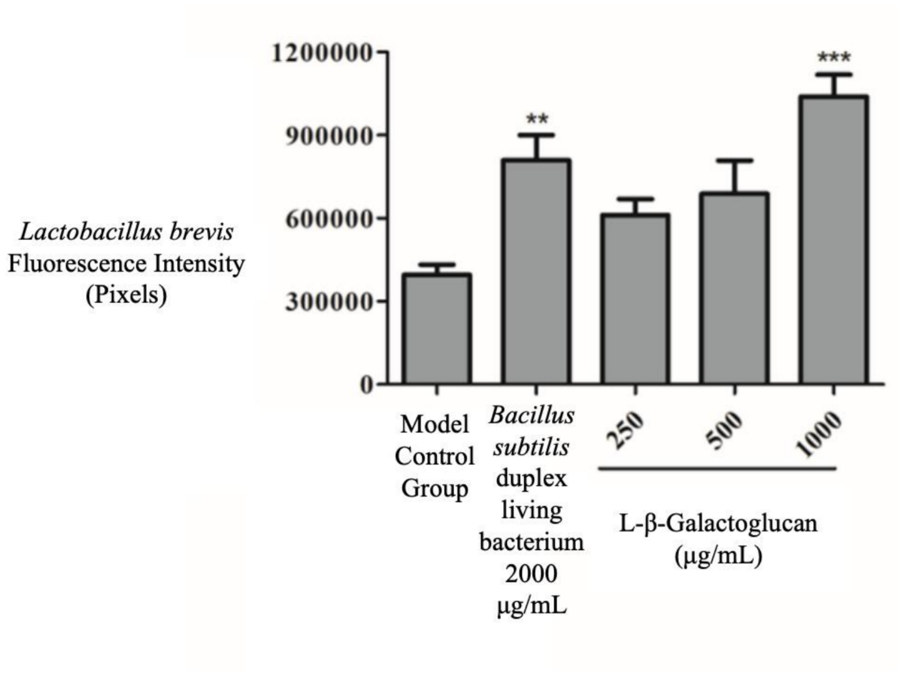


Figure 43

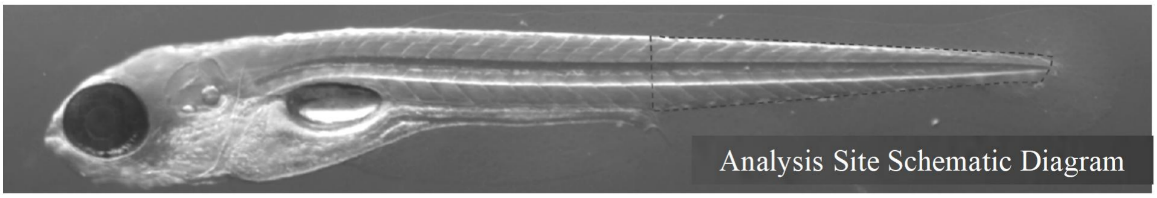


Figure 44

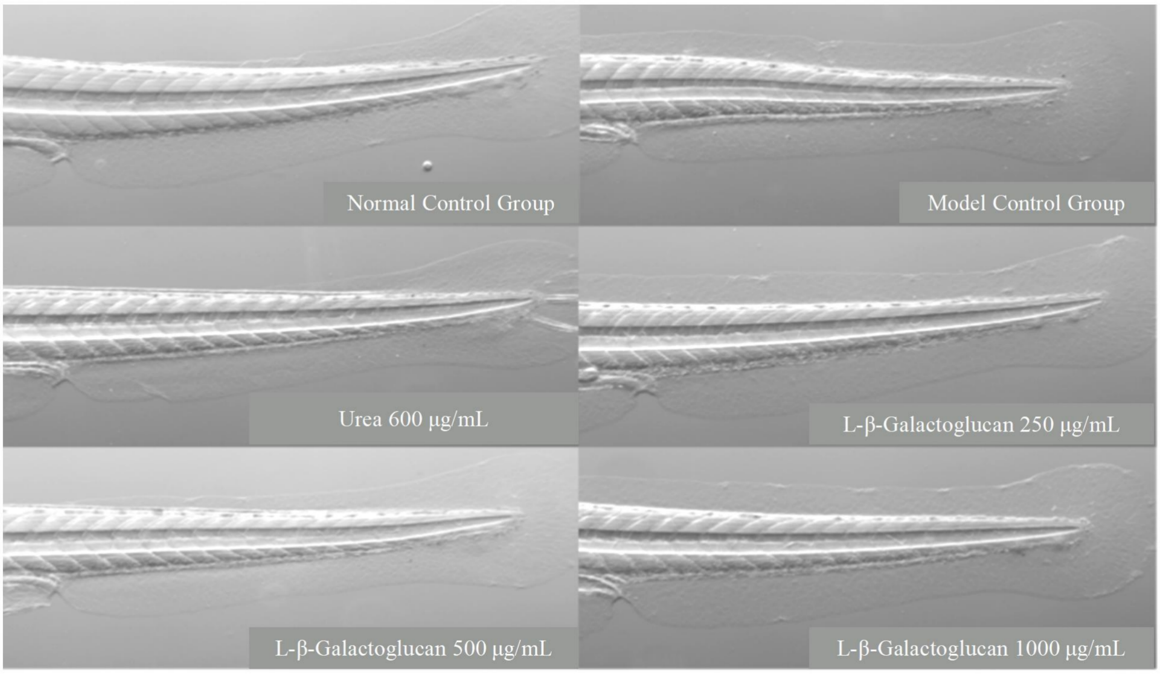


Figure 45

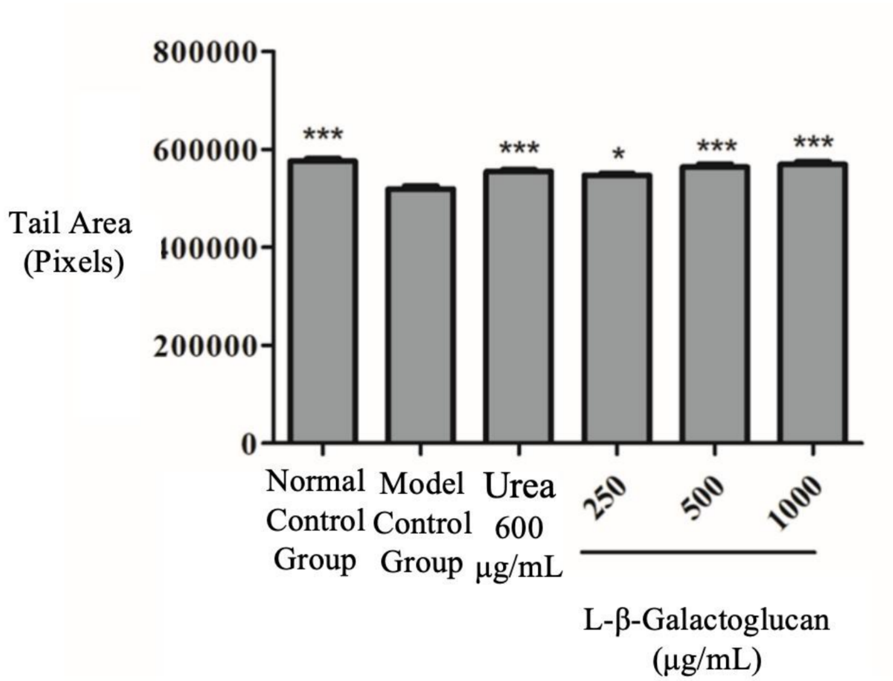


Figure 46

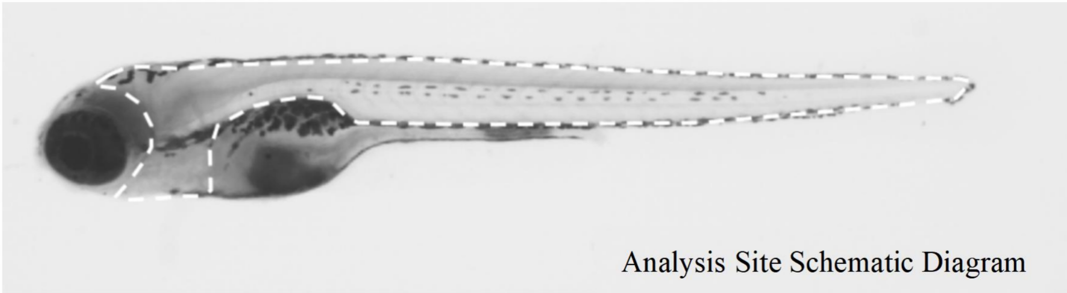


Figure 47

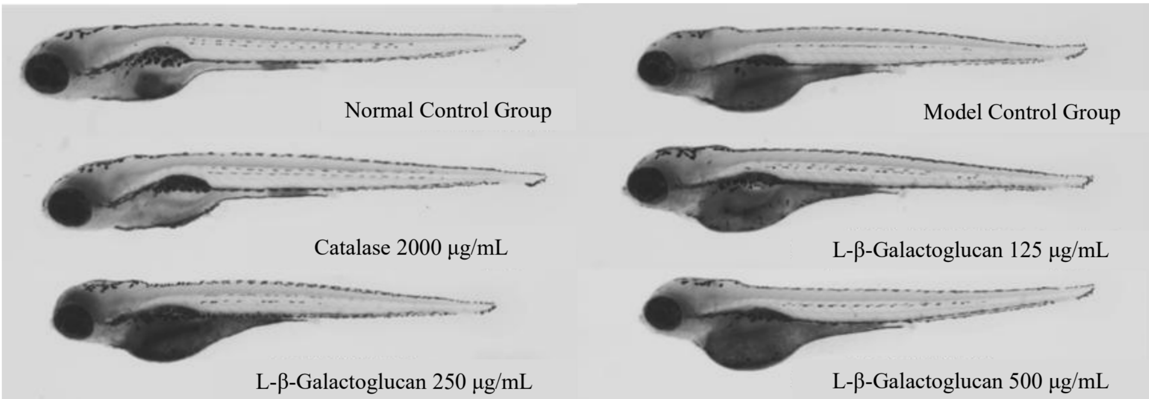


Figure 48

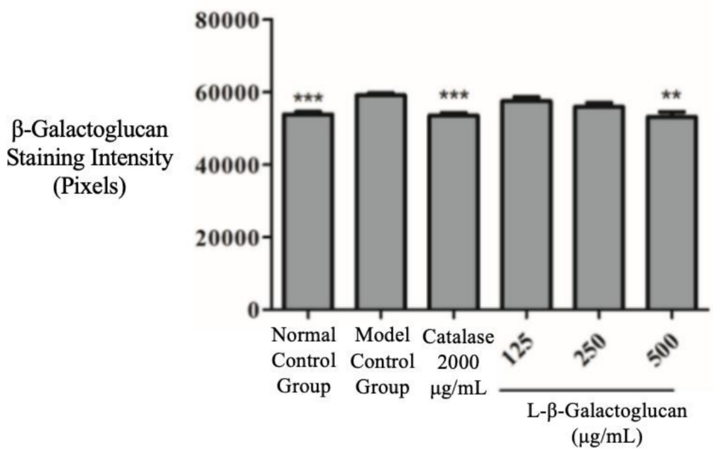


Figure 49

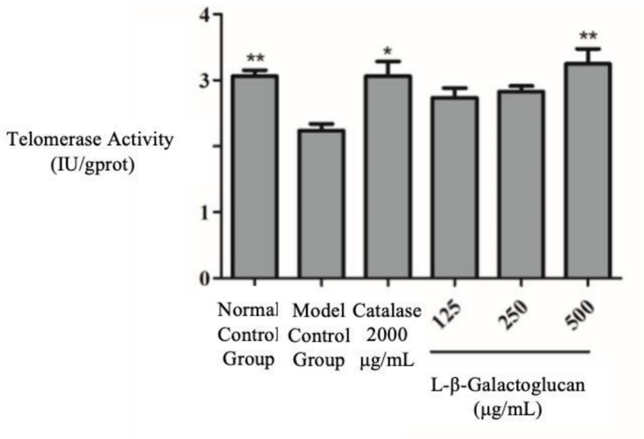


Figure 50

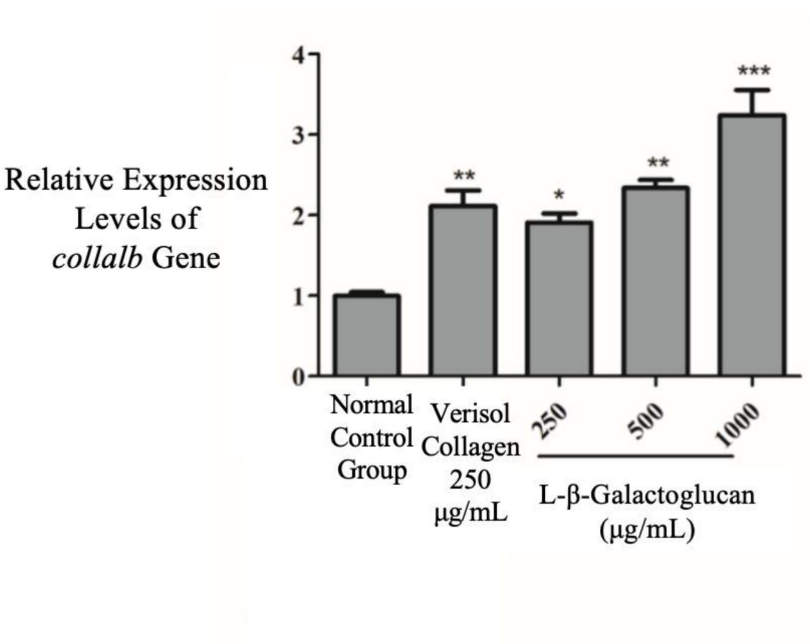


Figure 51

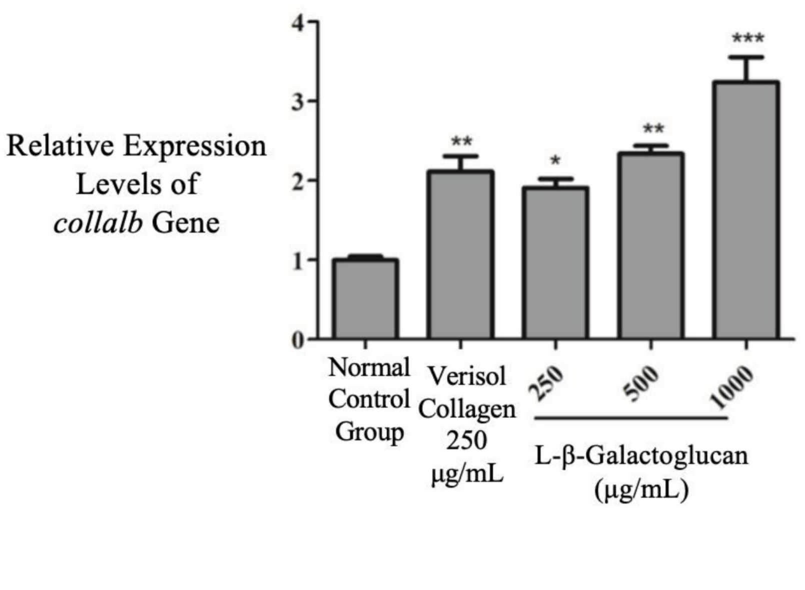


Figure 52

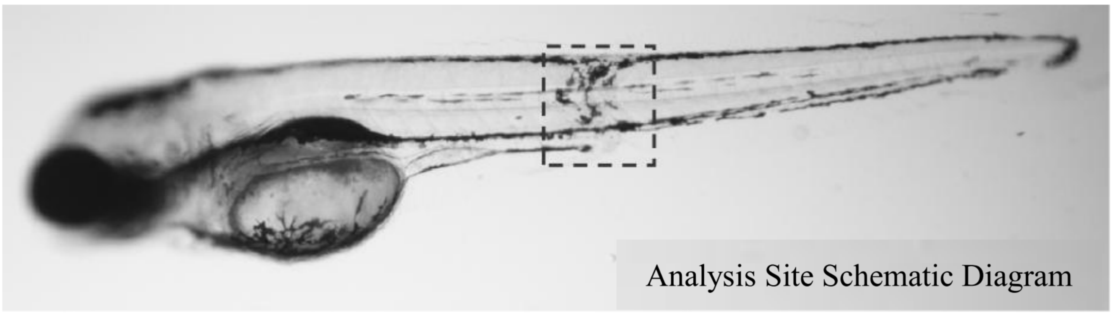


Figure 53

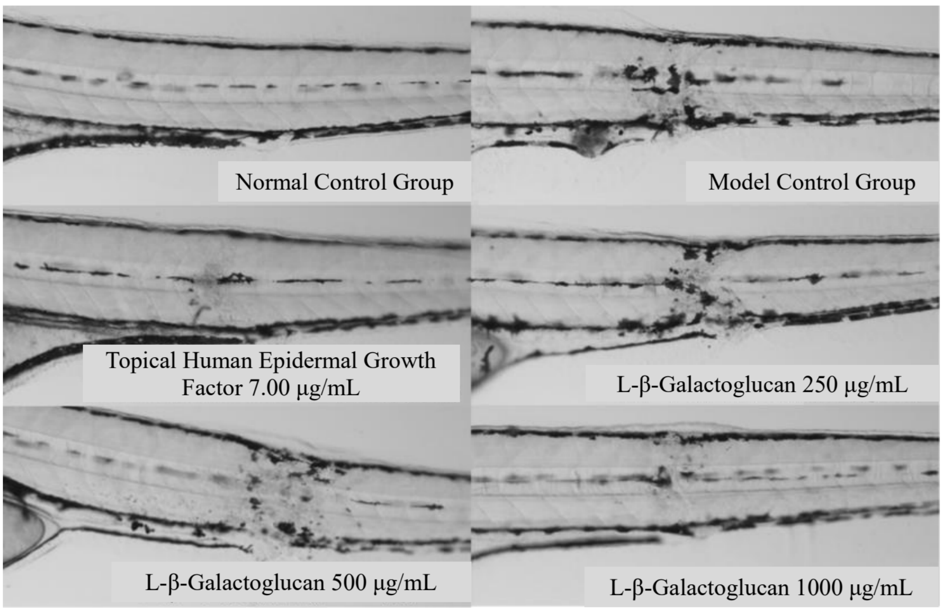


Figure 54

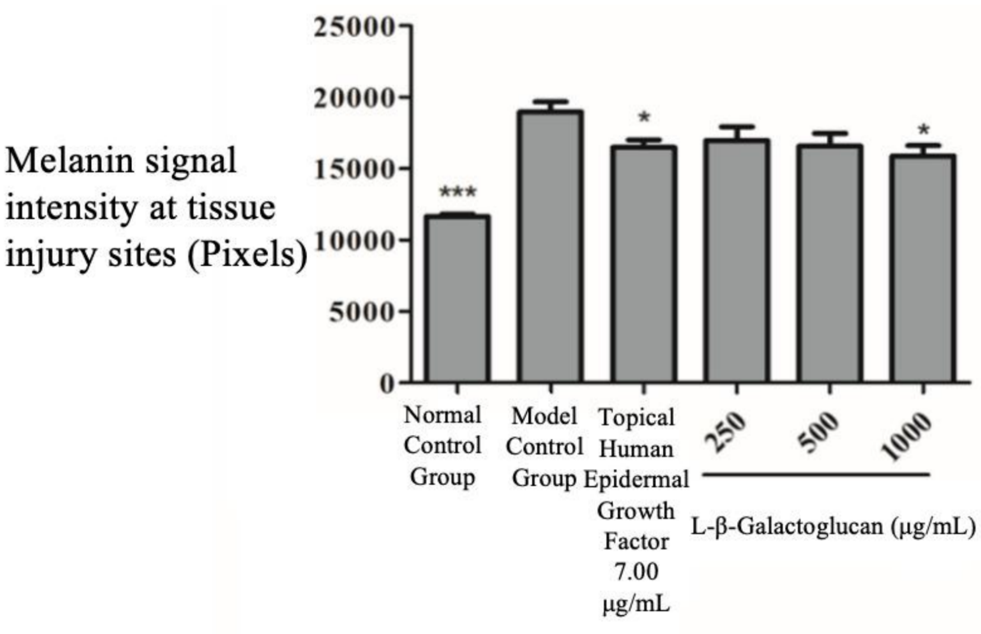


Figure 55

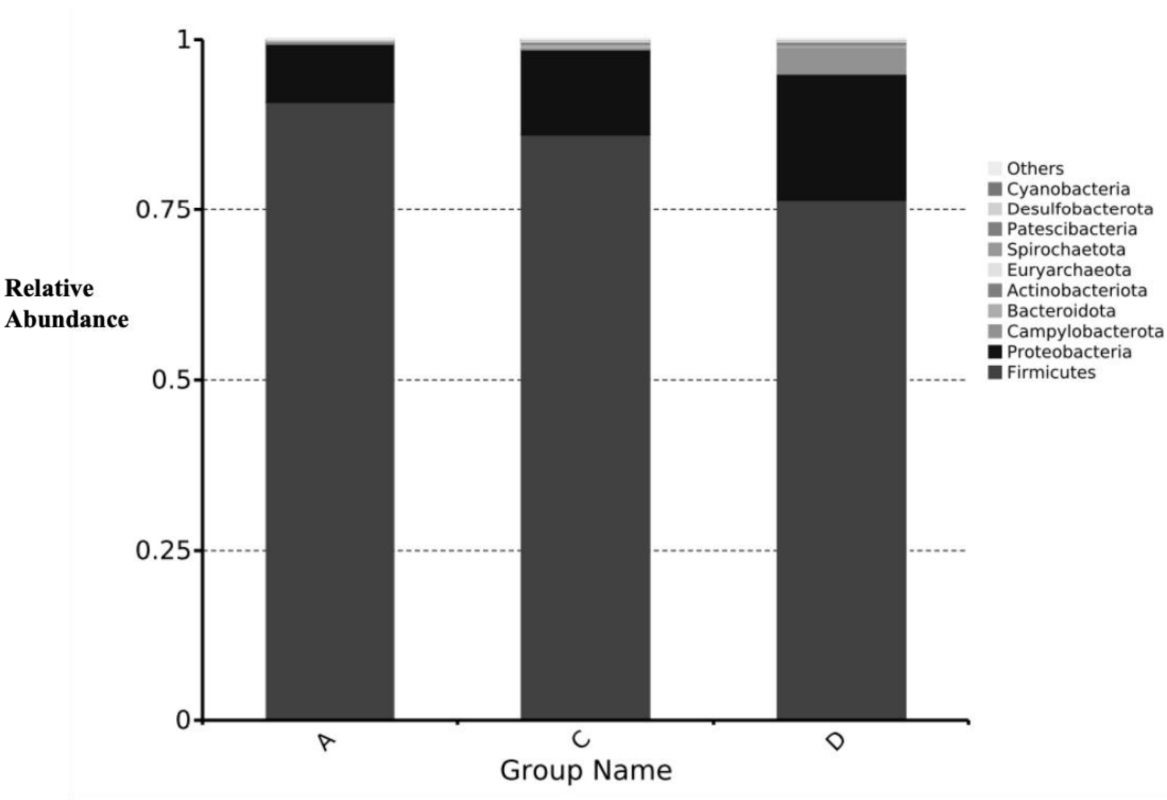


Figure 56

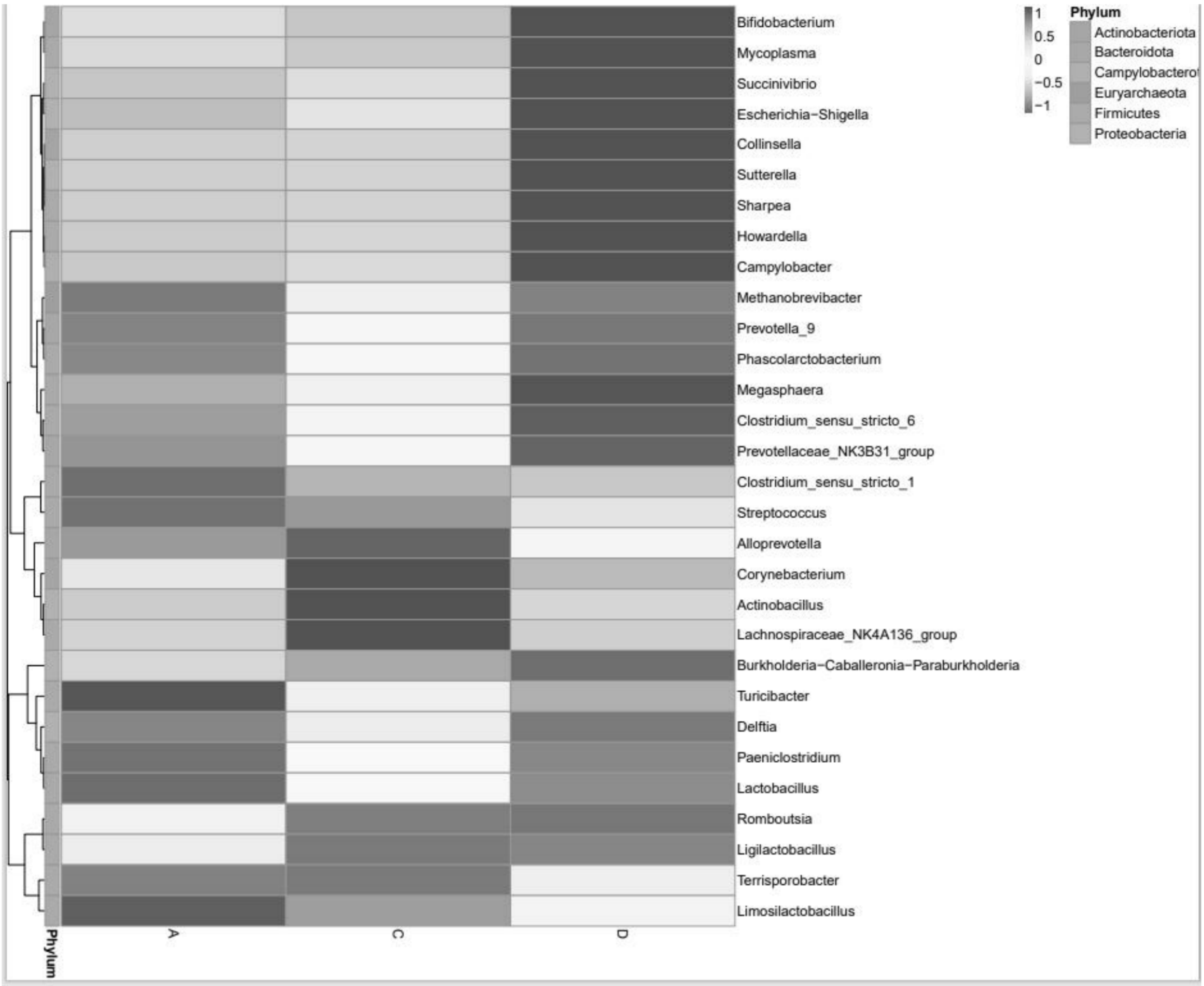
Bifidobacterium
Mycoplasma
Succinivibrio
Escherichia−Shigella
Collinsella
Sutterella
Sharpea
Howardella
Campylobacter
Methanobrevibacter
Prevotella_9
Phascolarctobacterium
Megasphaera
Clostridium_sensu_stricto_6
Prevotellaceae_NK3B31_group
Clostridium_sensu_stricto_1
Streptococcus
Alloprevotella
Corynebacterium
Actinobacillus
Lachnospiraceae_NK4A136_group
Burkholderia−Caballeronia−Paraburkholderia
Turicibacter
Delftia
Paeniclostridium
Lactobacillus
Romboutsia
Ligilactobacillus
Terrisporobacter
Limosilactobacillus
Phylum
A
C
D
1
0.5
0
−0.5
−1
Phylum
Actinobacteriota
Bacteroidota
Campylobacterot
Euryarchaeota
Firmicutes
Proteobacteria

Figure 57

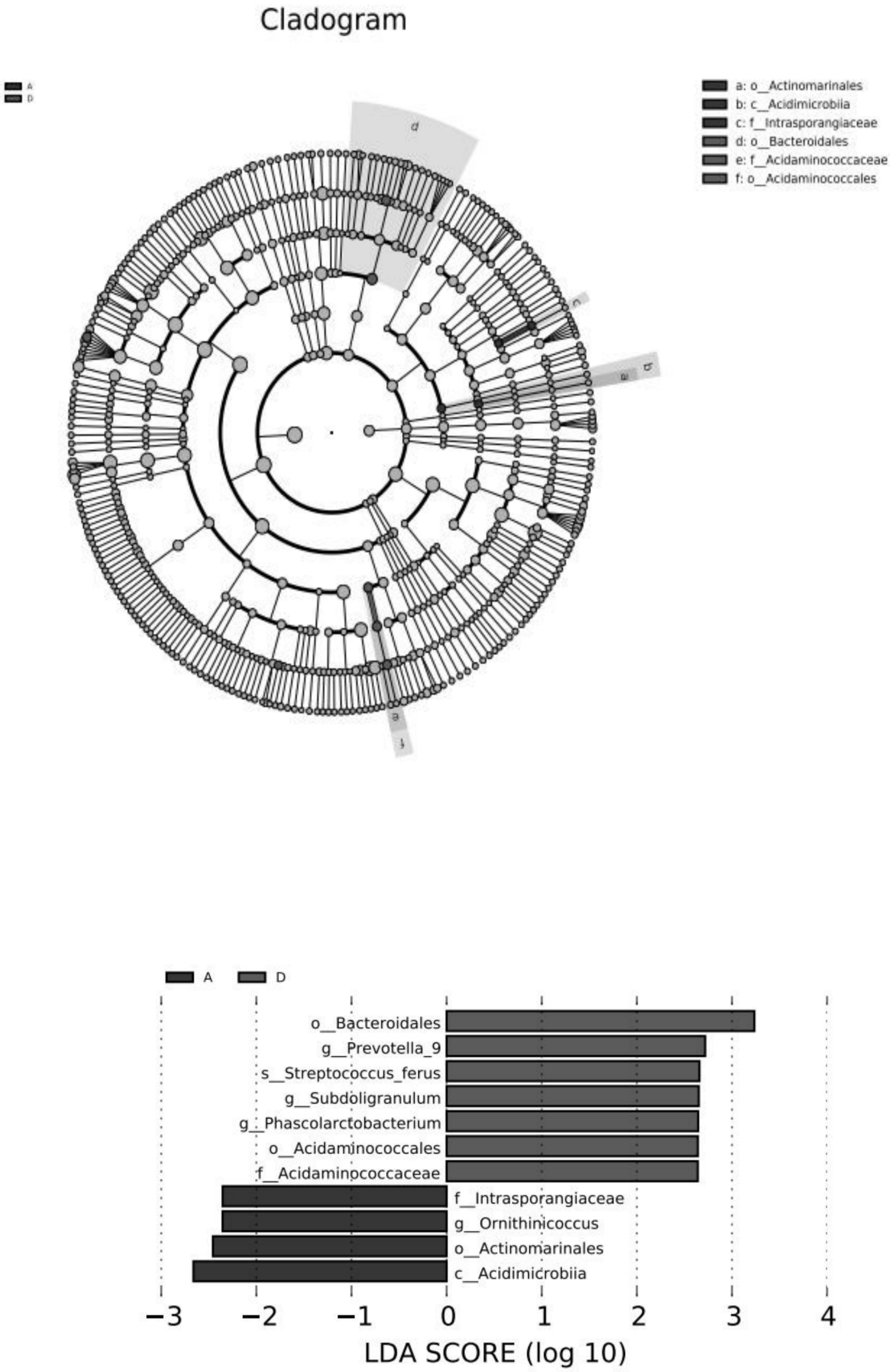
Cladogram
A
D
a: o__Actinomarinales
b: c__Acidimicrobiia
c: f__Intrasporangiaceae
d: o__Bacteroidales
e: f__Acidaminococcaceae
f: o__Acidaminococcales
d
c
b
a
e
f
A
D
o__Bacteroidales
g__Prevotella_9
s__Streptococcus_ferus
g__Subdoligranulum
g__Phascolarctobacterium
o__Acidaminococcales
f__Acidaminococcaceae
f__Intrasporangiaceae
g__Ornithinicoccus
o__Actinomarinales
c__Acidimicrobiia
−3
−2
−1
0
1
2
3
4
LDA SCORE (log 10)

Figure 58

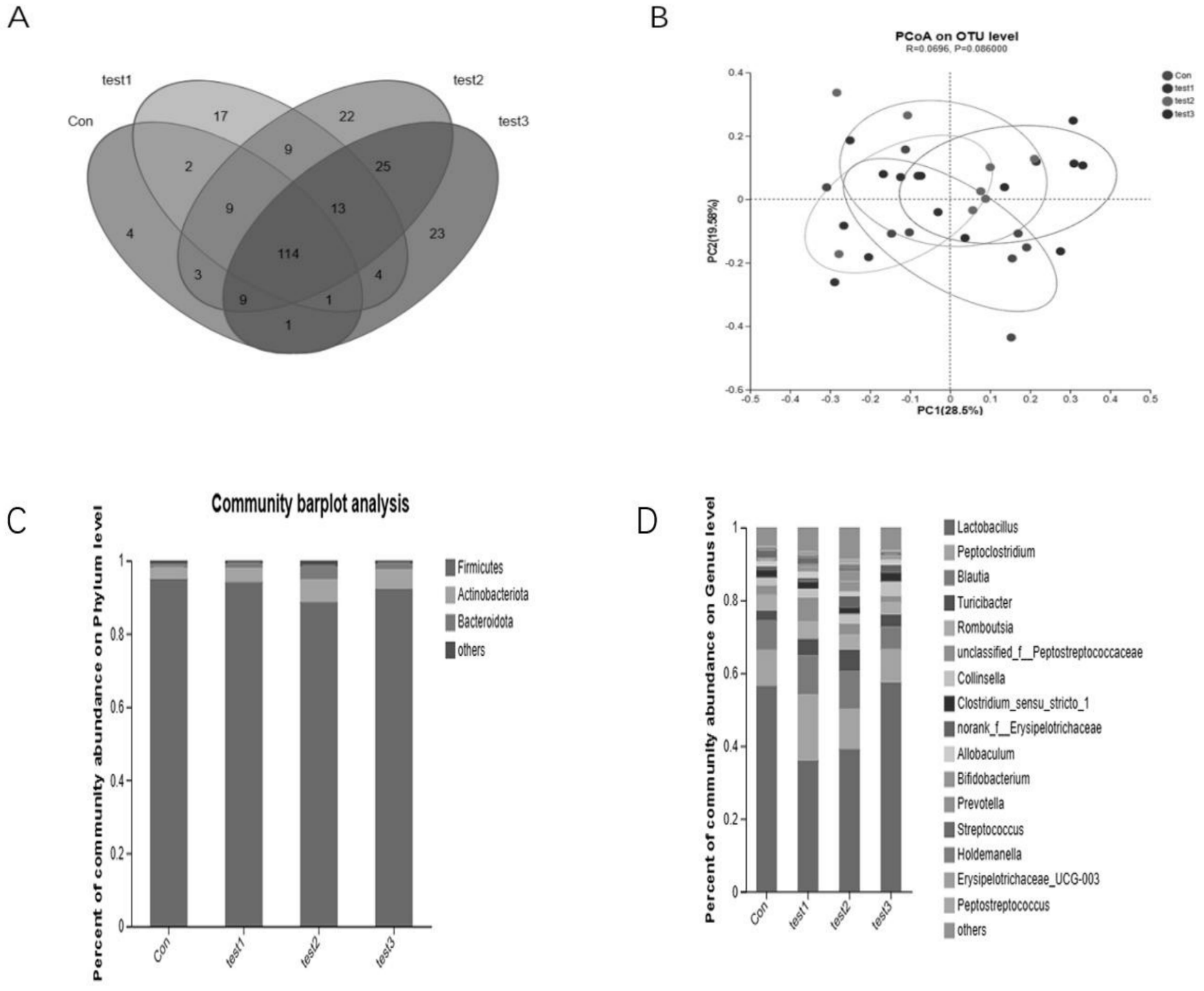
A
test1
test2
Con
test3
17
22
9
2
25
9
13
4
114
23
3
4
9
1
1
B
PCoA on OTU level
R=0.0696, P=0.086000
Con
test1
test2
test3
PC2(19.58%)
PC1(28.5%)
C
Community barplot analysis
Percent of community abundance on Phylum level
Firmicutes
Actinobacteriota
Bacteroidota
others
Con
test1
test2
test3
D
Percent of community abundance on Genus level
Lactobacillus
Peptoclostridium
Blautia
Turicibacter
Romboutsia
unclassified_f__Peptostreptococcaceae
Collinsella
Clostridium_sensu_stricto_1
norank_f__Erysipelotrichaceae
Allobaculum
Bifidobacterium
Prevotella
Streptococcus
Holdemanella
Erysipelotrichaceae_UCG-003
Peptostreptococcus
others
Con
test1
test2
test3

Figure 59

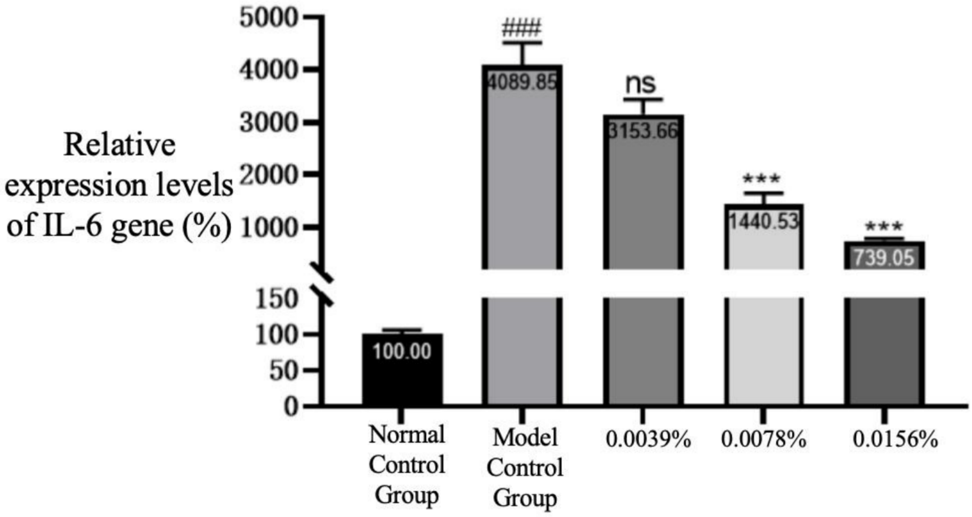
Relative expression levels of IL-6 gene (%)
5000
4000
3000
2000
1000
150
100
50
0
###
4089.85
ns
3153.66
***
1440.53
***
739.05
100.00
Normal Control Group
Model Control Group
0.0039%
0.0078%
0.0156%

Figure 60

**INTERNATIONAL SEARCH REPORT**

International application No. **PCT/CN2024/101362**

**A. CLASSIFICATION OF SUBJECT MATTER**

C08B37/00(2006.01)i; C12P19/04(2006.01)i; A61P1/16(2006.01)i; A61P3/10(2006.01)i; A61P17/00(2006.01)i; A61P37/04(2006.01)i; A61P39/06(2006.01)i; A61P31/10(2006.01)i

According to International Patent Classification (IPC) or to both national classification and IPC

**B. FIELDS SEARCHED**

Minimum documentation searched (classification system followed by classification symbols)

IPC: C08B,C12P,A61P

Documentation searched other than minimum documentation to the extent that such documents are included in the fields searched

Electronic data base consulted during the international search (name of data base and, where practicable, search terms used)

VCN, CNTXT, ENTXTC, DWPI, CNKI, ISI Web of Science: 土壤杆菌, 农杆菌, 土, 菌, 多糖, 聚糖, 半乳葡聚糖, 葡半乳聚糖, EPS, 葡萄糖, 半乳糖, glc, glu, gal, 发酵, agrobacterium, agrobacteria, soil, bacterium, bacteria, polysaccharide, polysaccharose, polyose, galactoglucan, glucogalactan, glucose, galactose, ferment

**C. DOCUMENTS CONSIDERED TO BE RELEVANT**

| Category* | Citation of document, with indication, where appropriate, of the relevant passages | Relevant to claim No. |
|---|---|---|
| A | CN 101935623 A (NANJING UNIVERSITY OF SCIENCE AND TECHNOLOGY) 05 January 2011 (2011-01-05)<br>entire document | 1-23 |
| A | CN 102816724 A (BRIGHT DAIRY & FOOD CO., LTD.) 12 December 2012 (2012-12-12)<br>entire document | 1-23 |
| A | CN 108752499 A (NANJING AGRICULTURAL UNIVERSITY et al.) 06 November 2018 (2018-11-06)<br>entire document | 1-23 |
| A | CN 111778300 A (SICHUAN SYNLIGHT BIO TECHNOLOGY CO., LTD.) 16 October 2020 (2020-10-16)<br>entire document | 1-23 |
| A | US 2020354481 A1 (KALEIDO BIOSCIENCES, INC.) 12 November 2020 (2020-11-12)<br>entire document | 1-23 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **12 October 2024** | **13 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/101362

C. DOCUMENTS CONSIDERED TO BE RELEVANT

| Category* | Citation of document, with indication, where appropriate, of the relevant passages | Relevant to claim No. |
|---|---|---|
| A | WO 9406927 A1 (PFIZER INC. et al.) 31 March 1994 (1994-03-31) entire document | 1-23 |

Form PCT/ISA/210 (second sheet) (July 2022)

**INTERNATIONAL SEARCH REPORT**
**Information on patent family members**

International application No.
**PCT/CN2024/101362**

| Patent document cited in search report | | | Publication date (day/month/year) | Patent family member(s) | | | Publication date (day/month/year) |
|---|---|---|---|---|---|---|---|
| CN | 101935623 | A | 05 January 2011 | CN | 101935623 | B | 23 May 2012 |
| CN | 102816724 | A | 12 December 2012 | CN | 102816724 | B | 28 May 2014 |
| CN | 108752499 | A | 06 November 2018 | | None | | |
| CN | 111778300 | A | 16 October 2020 | | None | | |
| US | 2020354481 | A1 | 12 November 2020 | US | 11697692 | B2 | 11 July 2023 |
| | | | | WO | 2019090181 | A1 | 09 May 2019 |
| | | | | EP | 3704161 | A1 | 09 September 2020 |
| WO | 9406927 | A1 | 31 March 1994 | CA | 2143868 | A1 | 31 March 1994 |
| | | | | DE | 69308320 | D1 | 03 April 1997 |
| | | | | DE | 69308320 | T2 | 21 August 1997 |
| | | | | JPH | 07506975 | A | 03 August 1995 |
| | | | | JP | 2761437 | B2 | 04 June 1998 |
| | | | | ATE | 149205 | T1 | 15 March 1997 |
| | | | | GR | 3023467 | T3 | 29 August 1997 |
| | | | | EP | 0672163 | A1 | 20 September 1995 |
| | | | | EP | 0672163 | B1 | 26 February 1997 |
| | | | | ES | 2102046 | T3 | 16 July 1997 |
| | | | | DK | 0672163 | T3 | 01 September 1997 |
| | | | | AU | 4687993 | A | 12 April 1994 |
| | | | | AU | 683016 | B2 | 30 October 1997 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023107516693